# EUROPEAN PATENT SPECIFICATION

(11) **EP 0 459 561 B1**
(45) Date of publication and mention of the grant of the patent: **20.09.1995**
(21) Application number: 91201216.8
(22) Date of filing: 22.05.1991
(51) Int. Cl.: C07D 409/06, A61K 31/47, C07D 409/04, C07D 401/04, C07D 215/22, C07D 405/04, C07D 401/12, C07D 409/12, C07D 413/04, C07D 404/04

(54) **Dioxo-tetrahydroquinoline derivatives**
Dioxo-tetrahydrochinolinderivate
Dérivés de dioxo-tétrahydroquinoline

(30) Priority: 31.05.1990 GB 9012165; 18.04.1991 GB 9108356
(43) Date of publication of application: 04.12.1991
(73) Proprietor: MERCK SHARP & DOHME LTD., Hoddesdon Hertfordshire EN11 9BU (GB)
(72) Inventor: Baker, Raymond, Much Hadhamm, Hertfordshire (GB); Stevenson, Graeme I., Sawbridgeworth, Hertfordshire (GB); Leeson, Paul D., Cambridge, Cambridgeshire (GB); Rowley, Michael, Harlow, Essex (GB)
(74) Representative: Thompson, John Dr.

(56) References cited:
- EP-A- 0 303 387

## Description

This invention relates to a class of 3-substituted 2,4-dioxo-1,2,3,4-tetrahydroquinolines which are selective non-competitive antagonists of N-methyl-D-aspartate (NMDA) receptors. More particularly, the class of compounds provided by the present invention are ligands for the strychnine-insensitive glycine modulatory site of the NMDA receptor and are therefore useful in the treatment and/or prevention of neurodegenerative disorders arising as a consequence of such pathological conditions as stroke, hypoglycaemia, cerebral palsy, transient cerebral ischaemic attack, cerebral ischaemia during cardiac pulmonary surgery or cardiac arrest, perinatal asphyxia, epilepsy, Huntington's chorea, Alzheimer's disease, Amyotrophic Lateral Sclerosis, Parkinson's disease, Olivo-ponto-cerebellar atrophy, anoxia such as from drowning, spinal cord and head injury, and poisoning by exogenous and endogenous NMDA receptor agonists and neurotoxins, including environmental neurotoxins.

By virtue of their NMDA receptor antagonist properties, the compounds according to the present invention are also useful as anticonvulsant agents, as well as being of value in the prevention or reduction of dependence on dependence-inducing agents such as narcotics.

NMDA receptor antagonists have recently been shown to possess analgesic (see, for example, Dickenson and Aydar, Neuroscience Lett., 1991, 121, 263; Murray et al., Pain, 1991, 44, 179; and Woolf and Thompson, Pain, 1991, 44, 293), antidepressant (see, for example, Trullas and Skolnick, Eur. J. Pharmacol., 1990, 185, 1) and anxiolytic (see, for example, Kehne et al., 1991, 193, 283) effects, and the compounds of the present invention may accordingly be useful in the management of pain, depression and anxiety.

The association of NMDA receptor antagonists with regulation of the nigrostriatal dopaminergic system has recently been reported (see, for example, Werling et al., J. Pharmacol. Exp. Ther., 1990, 255, 40; Graham et al., Life Sciences, 1990, 47, PL-41; and Turski et al., Nature (London), 1991, 349, 414). This suggests that the compounds of the present invention may thus be of assistance in the prevention and/or treatment of disorders of the dopaminergic system such as schizophrenia and Parkinson's disease.

Recent reports in the literature have also suggested a link between the neurotoxicity of certain viruses and the deleterious effects of these viruses on neurotransmission via excitatory amino acid receptors. By virtue of their activity as antagonists of NMDA receptors, therefore, the compounds of the present invention may be effective in controlling the manifestations of neuroviral diseases such as measles, rabies, tetanus (cf. Bagetta et al., Br. J. Pharmacol., 1990, 101, 776) and AIDS (cf. Lipton et al., Society for Neuroscience Abstracts, 1990, 16, 128.11).

NMDA antagonists have, moreover, been shown to have an effect on the neuroendocrine system (see, for example, van den Pol et al., Science, 1990, 250, 1276; and Urbanski, Endocrinology, 1990, 127, 2223), and the compounds of this invention may therefore also be effective in the control of seasonal breeding in mammals.

In addition, certain compounds of the invention are antagonists of 2-amino-3-hydroxy-5-methyl-4-isoxazolepropionic acid (AMPA) receptors, also known as quisqualate receptors. An excitatory amino acid projection from the prefrontal cortex to the nucleus accumbens (a particular region of the forebrain possessing dopamine-sensitive neurones) is well known to exist (see, for example, J. Neurochem., 1985, 45, 477). It is also well known that dopaminergic transmission in the striatum is modulated by glutamate (see, for example, Neurochem. Int., 1983, 5, 479), as also is the hyperactivity associated with presynaptic stimulation of the dopamine system by AMPA in the nucleus accumbens (cf. Life Sci., 1981, 28, 1597). Compounds which are antagonists of AMPA receptors are therefore of value as neuroleptic agents.

The use of 3-alkoxycarbonyl-substituted 2,4-dioxo-1,2,3,4-tetrahydroquinoline derivatives which are unsubstituted on the benzo moiety, as intermediates in the synthesis of various 2,4-dioxo-1,2,3,4-tetrahydroquinoline-3-carboxamide derivatives, is described in JP-A-43-23948. The latter compounds are stated to be antibacterial agents.

Various classes of 2,4-dioxo-1,2,3,4-tetrahydroquinolines, substituted at the 3-position by a cyano, carboxy or ester group, are described in BE-A-851866, DE-A-2806879, EP-A-0000153, US-4119720 and US-4221797. These compounds are stated to be antiallergic agents. The class of 3-cyano-substituted 2,4-dioxo-1,2,3,4-tetrahydroquinolines described in DE-A-3620856 are stated to be dyestuff intermediates. US-4362876 describes a process for preparing further 3-cyano-substituted 2,4-dioxo-1,2,3,4-tetrahydroquinoline derivatives. A still further class of 2,4-dioxo-1,2,3,4-tetrahydroquinolines, which are substituted on the benzo moiety by inter alia a cycloaliphatic group, is described in BE-A-814843. This class of compounds is alleged to have analgesic, antinociceptive, anti-inflammatory, antimicrobial, antibacterial, fungistatic, antiviral, coccidiostatic and antiallergic properties, with low toxicity.

Additional classes of 2,4-dioxo-1,2,3,4-tetrahydroquinoline derivatives are described in Monatsh. Chem., 1978, 109, 1075, Chem. Pharm. Bull., 1959, 7, 547, J. Org. Chem., 1964, 29, 2598, J. Org. Chem., 1969, 34, 2183, Eur. J. Med. Chem., 1981, 16, 251, Synthesis, 1979, 590, Yakugaku Zasshi, 1970, 90, 818, and J. Chem. Soc., 1950, 1678.

A class of 2,4-dioxo-1,2,3,4-tetrahydroquinoline-3-carboxamide derivatives is described in EP-A-0059698. These compounds are alleged to demonstrate an enhancing effect upon cell-mediated immunity, whilst also having a low toxicity, resulting in a favourable therapeutic index. EP-A-0059698 also discloses N-substituted 3-carboxy-2,4-dioxo-1,2,3,4-tetrahydroquinolines, and reactive derivatives thereof, as intermediates.

JP-A-59-16878 describes a process for producing 3-acetyl-2,4-dioxo-1,2,3,4-tetrahydroquinoline derivatives carrying a range of substituents on the benzo moiety. The compounds prepared by this process are stated to be useful intermediates in the production of therapeutic agents for allergic asthma.

A class of 2,4-dioxo-1,2,3,4-tetrahydroquinolines bearing an optionally substituted benzoyl moiety in the 3-position is described in EP-A-0101330. These compounds are stated to possess anti-inflammatory, antiallergic, antiasthmatic, antitussive and expectorant properties.

None of the aforementioned documents, however, discloses the particular class of 3-substituted 2,4-dioxo-1,2,3,4-tetrahydroquinolines provided by the present invention. Moreover, in none of those documents is there any suggestion that the compounds described therein would be of assistance in solving the problem of providing an effective agent for the treatment and/or prevention of conditions requiring the administration of an antagonist of NMDA and/or AMPA receptors.

EP-A-0303387 describes a class of 4-oxo-1,4-dihydroquinoline derivatives which are stated to be specific antagonists of NMDA receptors as well as being, in some cases, potent kainate/quisqualate antagonists. These compounds are stated therein to be useful in the treatment of a variety of neurodegenerative disorders.

The present invention accordingly provides the use of a compound of formula IA or a pharmaceutically acceptable salt thereof or a prodrug thereof: 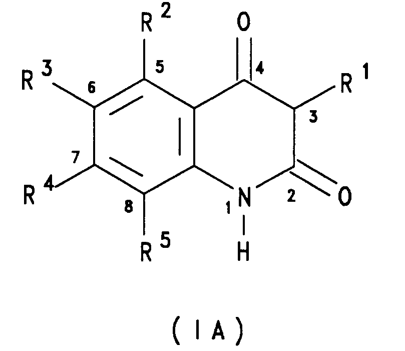
wherein
R¹ is a group of part formula (i) or (ii):

-(CH=CH)ₙ-T (i)

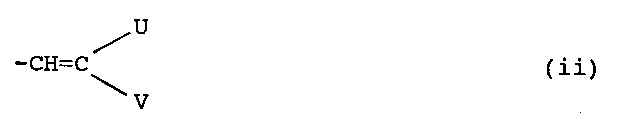

wherein
U and V independently represent cyano, carboxy, -COR⁶, -CO₂R⁶, -CO.SR⁶, -CONHOH or -CONHNH₂;
n is zero or 1, preferably zero;
T represents cyano, carboxy, -COR⁶, -CO₂R⁶, -CO.SR⁶, -CONHOH, -CONHNH₂ or a group of formula 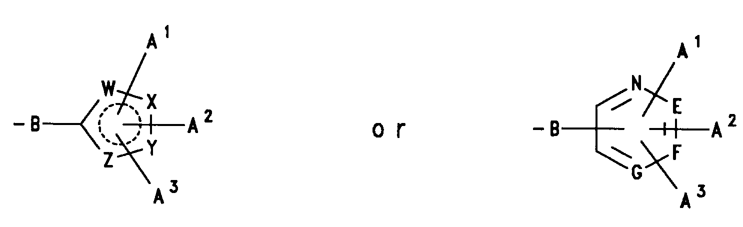
in which the broken circle represents two non-adjacent double bonds in any position in the five-membered ring;
B represents a bond or a carbonyl group (C=O);
W, X, Y and Z independently represent oxygen, sulphur, nitrogen or carbon, provided that no more than one of W, X, Y and Z represents oxygen or sulphur, at least one of W, X, Y and Z represents carbon and at least one of W, X, Y and Z is other than carbon;
one of E, F and G represents nitrogen or carbon and the remainder represent carbon;
A¹, A² and A³ represent one, two or three substituents not exceeding the maximum number permissible by the disposition of heteroatoms in the five- or six-membered ring, which substituents are independently selected from hydrogen, halogen, cyano, trifluoromethyl, nitro, -OR^{a}, -SR^{a}, -SOR^{a}, -SO₂R^{a}, -SO₂NR^{a}R^{b}, -NR^{a}R^{b}, -NR^{a}COR^{b}, -NR^{a}CO₂R^{b}, -COR^{a}, -CO₂R^{a}, -CONR^{a}R^{b}, or a hydrocarbon group comprising a straight-chained, branched or cyclic group containing up to 18 carbon atoms; or A¹ and A² or A² and A³ together represent the residue of an aromatic or heteroaromatic ring;
R², R³, R⁴ and R⁵ independently represent hydrogen, halogen, cyano, trifluoromethyl, nitro, -OR^{a}, -SR^{a}, -SOR^{a}, -SO₂R^{a}, -SO₂NR^{a}R^{b}, -NR^{a}R^{b}, -NR^{a}COR^{b}, -NR^{a}CO₂R^{b}, -COR^{a}, -CO₂R^{a}, -CONR^{a}R^{b}, or a hydrocarbon group comprising a straight-chained, branched or cyclic group containing up to 18 carbon atoms; or R² and R³, R³ and R⁴ or R⁴ and R⁵ together represent the residue of an aromatic or heteroaromatic ring;
R⁶ represents a hydrocarbon group comprising a straight-chained, branched or cyclic group containing up to 18 carbon atoms; and
R^{a} and R^{b} independently represent hydrogen or a hydrocarbon group comprising a straight-chained, branched or cyclic group containing up to 18 carbon atoms; for the manufacture of a medicament for the treatment and/or prevention of conditions, in particular neurodegenerative disorders, which require the administration of a selective non-competitive antagonist of NMDA receptors.

The present invention further provides the use of a compound of formula IA as defined above or a pharmaceutically acceptable salt thereof or a prodrug thereof for the manufacture of a medicament for the treatment and/or prevention of conditions, such as schizophrenia, which require the administration of an antagonist of AMPA receptors.

The compound of formula IA will in general exist in equilibrium with its other tautomeric forms, including those structures of formulae A to D: 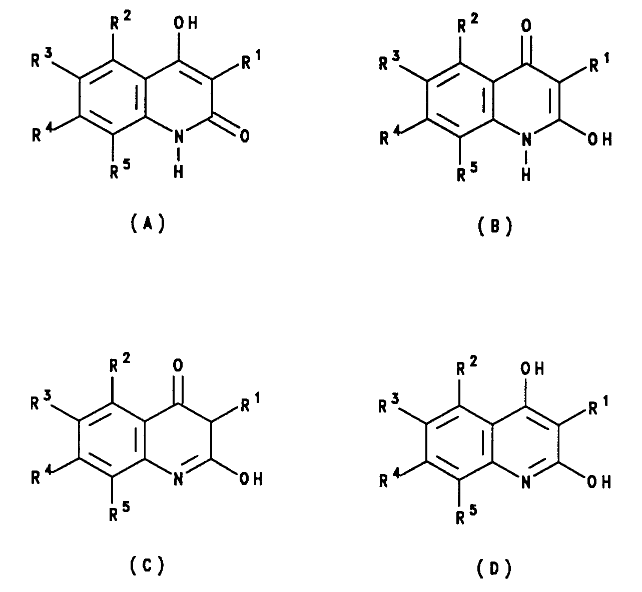
wherein R¹ to R⁵ are as defined with reference to formula IA above. Indeed, in the prior art references cited above, the compounds disclosed therein are variously designated by reference to one or other of these tautomeric forms. It is to be understood that all tautomeric forms of the compounds of formula IA, as well as all possible mixtures thereof, are included within the scope of the present invention.

The term "hydrocarbon" as used herein refers to straight-chained, branched and cyclic groups containing up to 18 carbon atoms. Within the scope of the expression "cyclic groups" are included heterocyclic groups. The hydrocarbon groups contain suitably up to 15 carbon atoms and conveniently up to 12 carbon atoms. Suitable hydrocarbon groups include C₁₋₆ alkyl, C₂₋₆ alkenyl, C₂₋₆ alkynyl, C₃₋₇ cycloalkyl, C₃₋₇ cycloalkyl(C₁₋₆)alkyl, aryl, aryl(C₁₋₆)alkyl, aryl(C₂₋₆)-alkenyl, aryl(C₂₋₆)alkynyl, C₃₋₇ heterocycloalkyl, C₃₋₇ heterocycloalkyl(C₁₋₆)alkyl, heteroaryl, heteroaryl-(C₁₋₆)alkyl and heteroaryl(C₂₋₆)alkenyl.

Suitable alkyl groups include straight-chained and branched alkyl groups containing from 1 to 6 carbon atoms. Typical examples include methyl and ethyl groups, and straight-chained or branched propyl and butyl groups. Particular alkyl groups are methyl, ethyl, n-propyl, isopropyl and t-butyl.

Suitable alkenyl groups include straight-chained and branched alkenyl groups containing from 2 to 6 carbon atoms. Typical examples include vinyl and allyl groups.

Suitable alkynyl groups include straight-chained and branched alkynyl groups containing from 2 to 6 carbon atoms. Typical examples include ethynyl and propargyl groups.

Suitable cycloalkyl groups include groups containing from 3 to 7 carbon atoms. Particular cycloalkyl groups are cyclopropyl and cyclohexyl.

Suitable aryl groups include phenyl and naphthyl groups.

Particular aryl(C₁₋₆)alkyl groups include benzyl, phenethyl, phenylpropyl and phenylbutyl.

A particular aryl(C₂₋₆)alkenyl group is phenylallyl.

A particular aryl(C₂₋₆)alkynyl group is phenylpropargyl.

Suitable heterocycloalkyl groups include piperidyl, piperazinyl and morpholinyl groups.

Suitable heteroaryl groups include pyridyl, quinolyl, isoquinolyl, pyridazinyl, pyrimidinyl, pyrazinyl, pyranyl, furyl, benzofuryl, thienyl, benzthienyl, pyrrolyl, indolyl, imidazolyl, oxadiazolyl and thiadiazolyl groups. Particular heteroaryl groups are pyridyl, furyl, benzofuryl, thienyl, benzthienyl, pyrrolyl, indolyl and oxadiazolyl.

Particular heteroaryl(C₁₋₆)alkyl groups include indolylethyl, indolylpropyl and thienylethyl.

A particular heteroaryl(C₂₋₆)alkenyl group is thienylvinyl.

The hydrocarbon group may in turn be optionally substituted by one or more groups selected from C₁₋₆ alkyl, adamantyl, phenyl, halogen, C₁₋₆ haloalkyl, trifluoromethyl, hydroxy, C₁₋₆ alkoxy, C₁₋₆ alkoxy-(C₁₋₆)alkoxy, aryloxy, keto, C₁₋₃ alkylenedioxy, nitro, cyano, carboxy, C₂₋₆ alkoxycarbonyl, C₂₋₆ alkoxycarbonyl-(C₁₋₆)alkyl, C₂₋₆ alkylcarbonyloxy, optionally substituted arylcarbonyloxy, C₂₋₆ alkylcarbonyl, optionally substituted arylcarbonyl, C₁₋₆ alkylthio, C₁₋₆ alkylsulphinyl, C₁₋₆ alkylsulphonyl, arylthio, amino, mono- or di(C₁₋₆)alkylamino, C₂₋₆ alkylcarbonylamino, C₂₋₆ alkoxycarbonylamino and C₂₋₆ alkoxycarbonylamino-(C₁₋₆)alkyl.

The term "halogen" as used herein includes fluorine, chlorine, bromine and iodine, especially chlorine.

The five-membered heteroaromatic ring containing the ring atoms W to Z may be, for example, a furan, thiophene, pyrrole, oxazole, thiazole, isoxazole, isothiazole, oxadiazole or thiadiazole ring, in particular a furan, thiophene, pyrrole, 1,2,4-oxadiazole, 1,3,4-oxadiazole, 1,2,4-thiadiazole or 1,3,4-thiadiazole ring. Preferably the ring is a furan, thiophene, pyrrole, 1,2,4-oxadiazole or 1,2,4-thiadiazole ring.

The six-membered heteroaromatic ring containing the ring atoms E, F and G is a pyridine, pyrazine, pyrimidine or pyridazine ring, preferably pyridine or pyrazine. In the case of a pyridine ring, E, F and G each represents carbon. In the case of a pyrazine ring, for example, G represents nitrogen, and E and F each represents carbon.

The number of substituents A¹, A² and/or A³ present on the five- or six-membered heteroaromatic ring containing the ring atoms W to Z or E to G respectively is one, two or three depending upon the disposition of heteroatoms in the heteroaromatic ring concerned. Thus where, for example, the five-membered heteroaromatic ring is an oxadiazole or thiadiazole ring, only one substituent will be permitted; where, for example, the five-membered heteroaromatic ring is an oxazole or thiazole ring, one or two substituents will be permitted; and where, for example, the five-membered heteroaromatic ring is a furan, thiophene or pyrrole ring, one, two or three substituents will be permitted. Where the heteroaromatic ring is a six-membered ring containing the ring atoms E, F and G it will be appreciated that one, two or three substituents A¹, A² and/or A³ will be permitted.

Suitable values for the groups A¹, A² and/or A³ include hydrogen, C₁₋₆ alkyl, C₂₋₆ alkenyl, C₂₋₆ alkynyl, C₃₋₇ cycloalkyl, C₃₋₇ cycloalkyl(C₁₋₆)alkyl, optionally substituted aryl, optionally substituted aryl(C₁₋₆)alkyl, halogen, cyano, trifluoromethyl, nitro, C₁₋₆ alkoxy, C₂₋₆ alkenyloxy, C₁₋₆ alkylthio, C₂₋₆ alkenylthio, -COR^{a} or -NR^{a}R^{b}, in which R^{a} and R^{b} are as defined above.

When T, U or V represents a group of formula -COR⁶, -CO₂R⁶ or -CO.SR⁶, the substituent R⁶ suitably represents C₁₋₆ alkyl, C₂₋₆ alkenyl, C₂₋₆ alkynyl, C₃₋₇ cycloalkyl, C₃₋₇ cycloalkyl(C₁₋₆)alkyl, aryl, aryl-(C₁₋₆)alkyl, aryl(C₂₋₆)alkenyl, aryl(C₂₋₆)alkynyl, C₃₋₇ heterocycloalkyl(C₁₋₆)alkyl, heteroaryl, heteroaryl-(C₁₋₆)alkyl or heteroaryl(C₂₋₆)alkenyl, any of which groups may be optionally substituted.

The benzo moiety of the tetrahydroquinoline ring system shown in formula IA above preferably contains at least one non-hydrogen substituent. Particular substituents include halogen, cyano, trifluoromethyl, nitro, hydroxy, amino, carboxy, C₁₋₆ alkyl, C₁₋₆ alkoxy, C₁₋₆ alkylthio and C₂₋₆ alkoxycarbonyl. Suitably, R⁵ represents hydrogen and R², R³ and R⁴ independently represent hydrogen, halogen, nitro, amino or C₁₋₆ alkyl, provided that at least one of R², R³ and R⁴ is other than hydrogen. Preferably, R³ and R⁵ each represents hydrogen and R² and R⁴ independently represent hydrogen, nitro, amino, methyl or halogen, especially chlorine, provided that at least one of R² and R⁴ is other than hydrogen. In a preferred embodiment, R⁴ represents chlorine.

Where R² and R³, R³ and R⁴, R⁴ and R⁵, A¹ and A² or A² and A³ represent the residue of an aromatic or heteroaromatic ring, this is suitably an optionally substituted benzene, pyridine, thiophene, thiazole or thiadiazole ring. As optional substituents on the aromatic or heteroaromatic ring may be mentioned nitro, and C₁₋₆ alkoxy such as methoxy.

Certain compounds falling within the definition of formula IA above are novel. Accordingly, in a further aspect the present invention provides a compound of formula IB or a salt or prodrug thereof: 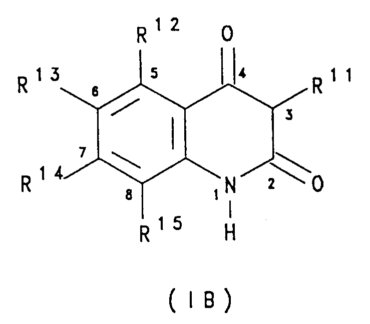
wherein
R¹¹ is a group of part formula (i) or (ii):

-(CH=CH)ₙ-T (i)

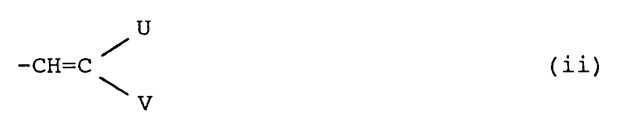

wherein
U and V independently represent cyano, carboxy, -COR⁶, -CO₂R⁶, -CO.SR⁶, -CONHOH or -CONHNH₂;
n is zero or 1, preferably zero;
T represents cyano, carboxy, -COR⁶, -CO₂R⁶, -CO.SR⁶, -CONHOH, -CONHNH₂ or a group of formula 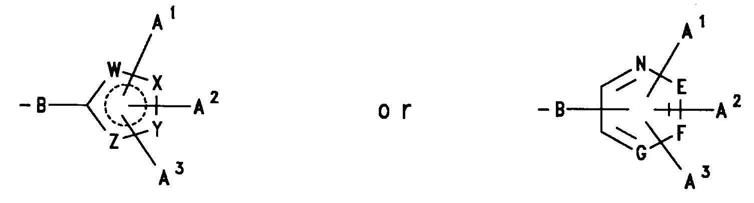
in which the broken circle represents two non-adjacent double bonds in any position in the five-membered ring;
B represents a bond or a carbonyl group (C=O);
W, X, Y and Z independently represent oxygen, sulphur, nitrogen or carbon, provided that no more than one of W, X, Y and Z represents oxygen or sulphur, at least one of W, X, Y and Z represents carbon and at least one of W, X, Y and Z is other than carbon;
one of E, F and G represents nitrogen or carbon and the remainder represent carbon;
A¹, A² and A³ represent one, two or three substituents not exceeding the maximum number permissible by the disposition of heteroatoms in the five- or six-membered ring, which substituents are independently selected from hydrogen, halogen, cyano, trifluoromethyl, nitro, -OR^{a}, -SR^{a}, -SOR^{a}, -SO₂R^{a}, -SO₂NR^{a}R^{b}, -NR^{a}R^{b}, -NR^{a}COR^{b}, -NR^{a}CO₂R^{b}, -COR^{a}, -CO₂R^{a}, -CONR^{a}R^{b}, or a hydrocarbon group comprising a straight-chained, branched or cyclic group containing up to 18 carbon atoms; or A¹ and A² or A² and A³ together represent the residue of an aromatic or heteroaromatic ring;
R¹², R¹³, R¹⁴ and R¹⁵ independently represent hydrogen, halogen, cyano, trifluoromethyl, nitro, -OR^{a}, -SR^{a}, -SOR^{a}, -SO₂R^{a}, -SO₂NR^{a}R^{b}, -NR^{a}R^{b}, -NR^{a}COR^{b}, -NR^{a}CO₂R^{b}, -COR^{a}, -CO₂R^{a}, -CONR^{a}R^{b}, or a hydrocarbon group comprising a straight-chained, branched or cyclic group containing up to 18 carbon atoms; or R¹² and R¹³, R¹³ and R¹⁴ or R¹⁴ and R¹⁵ together represent the residue of an aromatic or heteroaromatic ring;
R⁶ represents a hydrocarbon group comprising a straight-chained, branched or cyclic group containing up to 18 carbon atoms; and
R^{a} and R^{b} independently represent hydrogen or a hydrocarbon group comprising a straight-chained, branched or cyclic group containing up to 18 carbon atoms;
provided that:
(a) when two of the substituents R¹² to R¹⁵ represent hydrogen and the other two substituents R¹² to R¹⁵ independently represent hydrogen, C₁₋₈ alkyl, halogen, C₁₋₅ alkoxy, nitro, cyano, C₁₋₃ haloalkyl, carboxy or C₂₋₄ alkoxycarbonyl, then R¹¹ does not represent a benzoyl group optionally mono-, di- or trisubstituted by C₁₋₈ alkyl, halogen, C₁₋₅ alkoxy, nitro, cyano, C₁₋₃ haloalkyl, carboxy or C₂₋₄ alkoxycarbonyl;
(b) when R¹² to R¹⁵ independently represent hydrogen, C₁₋₅ alkyl, C₁₋₅ alkoxy, benzyloxy, C₂₋₆ alkoxycarbonyl, nitro or halogen, then R¹¹ does not represent acetyl;
(c) when one of R¹² to R¹⁵ represents hydrogen or methoxy and the remainder represent hydrogen, then R¹¹ does not represent -CONHNH₂ or -COR⁶, in which R⁶ is C₂₋₉ alkyl;
(d) when two of the substituents R¹² to R¹⁵ represent hydrogen and the other two substituents R¹² to R¹⁵ independently represent hydrogen, halogen, C₁₋₄ alkyl, C₁₋₄ alkoxy, nitro or trifluoromethyl or together represent methylenedioxy, then R¹¹ does not represent cyano, carboxy or -CO₂R⁶, in which R⁶ is C₁₋₉ alkyl, C₃₋₆ alkenyl or C₃₋₆ alkynyl;
(e) when two of the substituents R¹² to R¹⁵ represent hydrogen, a third substituent R¹² to R¹⁵ represents an optionally substituted C₅₋₈ cycloalkyl or cycloalkenyl group or adamantyl, and the remaining substituent R¹² to R¹⁵ represents hydrogen, halogen, lower alkyl or lower alkoxy, then R¹¹ does not represent carboxy or -COR¹⁶, in which R¹⁶ is an etherified hydroxyl group;
(f) when R¹² to R¹⁵ each represents hydrogen and R¹¹ is a group of part formula (ii) as defined above, then U and V are not simultaneously carboxy, and U is not carboxy or ethoxycarbonyl when V is cyano; and
(g) when R¹² to R¹⁵ each represents hydrogen, then R¹¹ does not represent unsubstituted 2-pyridyl, 3-pyridyl, 2-furyl or 2-benzothiazolyl.

Subject to the above provisos, the substituents R¹¹ to R¹⁵ in the compounds of formula IB correspond to the substituents R¹ to R⁵ respectively as defined with reference to the compounds of formula IA.

Representative values of R¹¹ include cyano, carboxy, cyclopropylcarbonyl, benzylcarbonyl, thienylvinylcarbonyl, methoxycarbonyl, ethoxycarbonyl, phenylthio-ethoxycarbonyl, propoxycarbonyl, allyloxycarbonyl, hydroxyphenyl-ethoxycarbonyl, bis(methoxymethoxy)phenyl-ethoxycarbonyl, (t-butoxycarbonylaminomethyl)phenyl-ethoxycarbonyl, hydroxyphenyl-propoxycarbonyl, hydroxyphenyl-butoxycarbonyl, methoxyphenyl-propenyloxycarbonyl, hydroxyphenyl-propynyloxycarbonyl, methoxyphenyl-propynyloxycarbonyl, thienyl-ethoxycarbonyl, indolyl-ethoxycarbonyl, methoxyindolyl-ethoxycarbonyl, indolyl-propoxycarbonyl, phenethylthio-carbonyl, hydroxyaminocarbonyl, hydrazinocarbonyl, furyl, methylfuryl, ethylfuryl, isopropylfuryl, phenylfuryl, benzofuryl, thienyl, methylthienyl, benzylthienyl, benzothienyl, N-methylpyrrolyl, benzoyl-(N-methyl)-pyrrolyl, methyloxadiazolyl, furoyl, methylfuroyl, dimethylfuroyl, benzofuroyl, nitro-benzofuroyl, thienoyl, methylthienoyl, bromothienoyl, dimethylthienoyl, benzothienoyl, N-methylpyrrolylcarbonyl, N-methylindolylcarbonyl, pyridyl and ethoxycarbonyl-ethenyl.

For use in medicine, the salts of the compounds of formula IB will be non-toxic pharmaceutically acceptable salts. Other salts may, however, be useful in the preparation of the compounds according to the invention or of their non-toxic pharmaceutically acceptable salts.

Suitable pharmaceutically acceptable salts of the compounds of formulae IA and IB above include alkali metal salts, e.g. sodium or potassium salts; alkaline earth metal salts, e.g. calcium or magnesium salts; and salts formed with suitable organic ligands, e.g. quaternary ammonium salts. Where appropriate, acid addition salts may, for example, be formed by mixing a solution of the compound according to the invention with a solution of a pharmaceutically acceptable non-toxic acid such as hydrochloric acid, fumaric acid, maleic acid, succinic acid, acetic acid, citric acid, tartaric acid, carbonic acid or phosphoric acid.

The present invention includes within its scope prodrugs of the compounds of formulae IA and IB above. In general, such prodrugs will be functional derivatives of the compounds of formulae IA and IB which are readily convertible in vivo into the required compound. Conventional procedures for the selection and preparation of suitable prodrug derivatives are described, for example, in "Design of Prodrugs", ed. H. Bundgaard, Elsevier, 1985.

Where the compounds according to the invention have at least one asymmetric centre, they may accordingly exist as enantiomers. Where the compounds according to the invention possess two or more asymmetric centres, they may additionally exist as diastereoisomers. It is to be understood that all such isomers and mixtures thereof are encompassed within the scope of the present invention.

One sub-class of compounds according to the invention is represented by the compounds of formula IIA, and salts and prodrugs thereof: 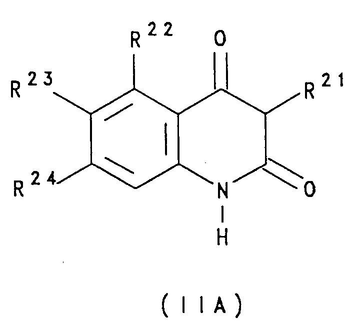
wherein
R²¹ represents -COR²⁶ or -CO₂R²⁶;
R²², R²³ and R²⁴ independently represent hydrogen, halogen, cyano, trifluoromethyl, nitro, hydroxy, amino, carboxy, C₁₋₆ alkyl, C₁₋₆ alkoxy, C₁₋₆ alkylthio or C₂₋₆ alkoxycarbonyl, provided that at least one of R²², R²³ and R²⁴ is other than hydrogen; and
R²⁶ represents C₃₋₇ cycloalkyl, aryl(C₁₋₆)-alkyl, aryl(C₂₋₆)alkenyl, aryl(C₂₋₆)alkynyl, heteroaryl(C₁₋₆)alkyl or heteroaryl(C₂₋₆)alkenyl, any of which groups may be optionally substituted.

Examples of optional subtituents on the group R²⁶ suitably include hydroxy, C₁₋₆ alkoxy, C₁₋₆ alkoxy(C₁₋₆)alkoxy and C₂₋₆ alkoxycarbonylamino(C₁₋₆)-alkyl, especially hydroxy, methoxy, methoxymethoxy and t-butoxycarbonylaminomethyl.

Particular values of R²⁶ with respect to formula IIA include cyclopropyl, benzyl, phenethyl, hydroxyphenethyl, bis(methoxymethoxy)phenethyl, (t-butoxycarbonylaminomethyl)phenethyl, phenylpropyl, hydroxyphenylpropyl, phenylbutyl, hydroxyphenylbutyl, phenylallyl, methoxyphenylallyl, phenylpropargyl, hydroxyphenylpropargyl, methoxyphenylpropargyl, indolylethyl, methoxyindolylethyl, indolylpropyl, thienylethyl and thienylvinyl. A preferred group R²⁶ is cyclopropyl.

Suitably, R²², R²³ and R²⁴ are independently selected from hydrogen, halogen, nitro, amino and C₁₋₆ alkyl, provided that at least one of R²², R²³ and R²⁴ is other than hydrogen. Preferably R²³ represents hydrogen, one of R²² and R²⁴ represents halogen or nitro, and the other of R²² and R²⁴ represents hydrogen, halogen or nitro. In a particular embodiment, R²² and R²³ each represents hydrogen and R²⁴ represents halogen, especially chlorine.

Another sub-class of compounds according to the invention is represented by the compounds of formula IIB, and salts and prodrugs thereof: 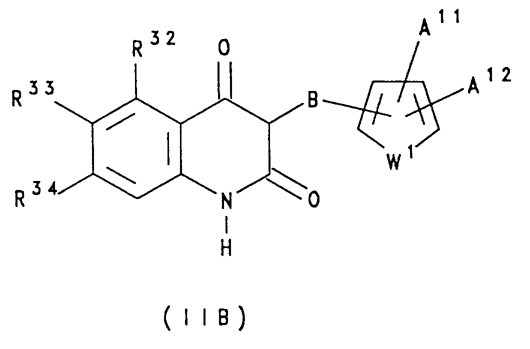
wherein
W¹ represents oxygen, sulphur or N-A¹³;
A¹¹ and A¹² independently represent hydrogen, halogen, cyano, trifluoromethyl, nitro, hydroxy, amino, carboxy, C₁₋₆ alkyl, C₂₋₆ alkenyl, C₂₋₆ alkynyl, C₃₋₇ cycloalkyl, C₃₋₇ cycloalkyl(C₁₋₆)alkyl, aryl, aryl(C₁₋₆)alkyl, C₁₋₆ alkoxy, C₂₋₆ alkenyloxy, C₁₋₆ alkylthio, C₂₋₆ alkenylthio, C₂₋₆ alkylcarbonyl, arylcarbonyl or C₂₋₆ alkoxycarbonyl; or A¹¹ and A¹² together represent the residue of an optionally substituted aromatic or heteroaromatic ring;
A¹³ represents hydrogen, C₁₋₆ alkyl or aryl(C₁₋₆)alkyl;
B represents a bond or a carbonyl group (C=O); and
R³², R³³ and R³⁴ independently represent hydrogen, halogen, cyano, trifluoromethyl, nitro, hydroxy, amino, carboxy, C₁₋₆ alkyl, C₁₋₆ alkoxy, C₁₋₆ alkylthio or C₂₋₆ alkoxycarbonyl, provided that at least one of R³², R³³ and R³⁴ is other than hydrogen.

Examples of suitable values for the groups A¹¹ and A¹² include hydrogen, halogen, C₁₋₆ alkyl, C₂₋₆ alkenyl, C₂₋₆ alkynyl, C₃₋₇ cycloalkyl, C₃₋₇ cycloalkyl(C₁₋₆)alkyl, aryl, aryl(C₁₋₆)alkyl, C₁₋₆ alkoxy, C₂₋₆ alkenyloxy, C₁₋₆ alkylthio, C₂₋₆ alkenylthio and arylcarbonyl. Particular values of A¹¹ and A¹² include hydrogen, bromo, methyl, ethyl, isopropyl, vinyl, allyl, cyclopropyl, cyclopropylmethyl, phenyl, benzyl, allyloxy, allylthio and benzoyl.

Where A¹¹ and A¹² together represent the residue of an optionally subtituted aromatic or heteroaromatic ring, this is preferably an optionally substituted benzene ring. Examples of optional substituents on the aromatic or heteroaromatic ring suitably include nitro, and C₁₋₆ alkoxy such as methoxy.

Suitably, A¹³ represents hydrogen or methyl, preferably methyl.

Suitably, R³², R³³ and R³⁴ are independently selected from hydrogen, halogen, nitro, amino and C₁₋₆ alkyl, provided that at least one of R³², R³³ and R³⁴ is other than hydrogen. Preferably R³³ represents hydrogen, one of R³² and R³⁴ represents halogen or nitro, and the other of R³² and R³⁴ represents hydrogen, halogen or nitro. In a particular embodiment, R³² and R³³ each represents hydrogen and R³⁴ represents halogen, especially chlorine.

A further sub-class of compounds according to the invention is represented by the compounds of formula IIC, and salts and prodrugs thereof: 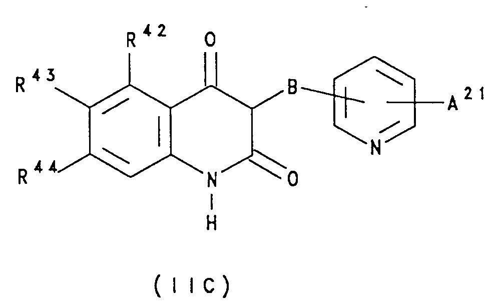
wherein
A²¹ represents hydrogen, halogen, cyano, trifluoromethyl, nitro, hydroxy, amino, carboxy, C₁₋₆ alkyl, C₁₋₆ alkoxy, C₁₋₆ alkylthio, C₂₋₆ alkylcarbonyl, arylcarbonyl or C₂₋₆ alkoxycarbonyl;
B represents a bond or a carbonyl group (C=O); and
R⁴², R⁴³ and R⁴⁴ independently represent hydrogen, halogen, cyano, trifluoromethyl, nitro, hydroxy, amino, carboxy, C₁₋₆ alkyl, C₁₋₆ alkoxy, C₁₋₆ alkylthio or C₂₋₆ alkoxycarbonyl, provided that at least one of R⁴², R⁴³ and R⁴⁴ is other than hydrogen.

Examples of suitable values for the group A²¹ include hydrogen, halogen, C₁₋₆ alkyl and arylcarbonyl. Preferably, A²¹ is hydrogen.

Suitably, R⁴², R⁴³ and R⁴⁴ are independently selected from hydrogen, halogen, nitro, amino and C₁₋₆ alkyl, provided that at least one of R⁴², R⁴³ and R⁴⁴ is other than hydrogen. Preferably R⁴³ represents hydrogen, one of R⁴² and R⁴⁴ represents halogen or nitro, and the other of R⁴² and R⁴⁴ represents hydrogen, halogen or nitro. In a particular embodiment, R⁴² and R⁴³ each represents hydrogen and R⁴⁴ represents halogen, especially chlorine.

Specific compounds within the scope of the present invention include:
3-benzyloxycarbonyl-7-chloro-2,4-dioxo-1,2,3,4-tetrahydroquinoline;
7-chloro-2,4-dioxo-3-(2-phenylethoxy)carbonyl-1,2,3,4-tetrahydroquinoline;
7-chloro-2,4-dioxo-3-(3-phenylpropoxy)carbonyl-1,2,3,4-tetrahydroquinoline;
7-chloro-2,4-dioxo-3-[2-(3-hydroxyphenyl)ethoxy]carbonyl-1,2,3,4-tetrahydroquinoline;
7-chloro-2,4-dioxo-3-[3-(4-hydroxyphenyl)propoxy]carbonyl-1,2,3,4-tetrahydroquinoline;
7-chloro-2,4-dioxo-3-[2-(2-hydroxyphenyl)ethoxy]carbonyl-1,2,3,4-tetrahydroquinoline;
7-chloro-3-cyclopropylmethoxycarbonyl-2,4-dioxo-1,2,3,4-tetrahydroquinoline;
7-chloro-2,4-dioxo-3-[2-(4-hydroxyphenyl)ethoxy]carbonyl-1,2,3,4-tetrahydroquinoline;
7-chloro-2,4-dioxo-3-(3-hydroxyphenylmethoxy)carbonyl-1,2,3,4-tetrahydroquinoline;
7-chloro-2,4-dioxo-3-(pyrid-2-ylmethoxy)carbonyl-1,2,3,4-tetrahydroquinoline;
2,4-dioxo-3-[3-(4-hydroxyphenyl)propoxy]carbonyl-7-nitro-1,2,3,4-tetrahydroquinoline;
7-chloro-2,4-dioxo-3-(2-hydroxyethoxy)carbonyl-1,2,3,4-tetrahydroquinoline;
7-chloro-2,4-dioxo-3-(3-thienyl)carbonyl-1,2,3,4-tetrahydroquinoline;
7-chloro-2,4-dioxo-3-(2-furyl)carbonyl-1,2,3,4-tetrahydroquinoline;
7-chloro-2,4-dioxo-3-(2-thienyl)carbonyl-1,2,3,4-tetrahydroquinoline;
7-chloro-3-(2,5-dimethyl-3-furyl)carbonyl-2,4-dioxo-1,2,3,4-tetrahydroquinoline;
7-chloro-2,4-dioxo-3-(5-methyl-2-furyl)carbonyl-1,2,3,4-tetrahydroquinoline;
7-chloro-2,4-dioxo-3-[2-(5-nitrobenzofuryl)carbonyl]-1,2,3,4-tetrahydroquinoline;
3-[2-(benzofuryl)carbonyl]-7-chloro-2,4-dioxo-1,2,3,4-tetrahydroquinoline;
3-[2-(benzo[b]thienyl)carbonyl]-7-chloro-2,4-dioxo-1,2,3,4-tetrahydroquinoline;
3-benzylcarbonyl-7-chloro-2,4-dioxo-1,2,3,4-tetrahydroquinoline;
7-chloro-2,4-dioxo-3-(5-methyl-2-thienyl)carbonyl-1,2,3,4-tetrahydroquinoline;
7-chloro-2,4-dioxo-3-(3-methyl-2-thienyl)carbonyl-1,2,3,4-tetrahydroquinoline;
7-chloro-3-(2,5-dimethyl-3-thienyl)carbonyl-2,4-dioxo-1,2,3,4-tetrahydroquinoline;
7-chloro-2,4-dioxo-3-[2-(3-thienyl)ethenyl]carbonyl-1,2,3,4-tetrahydroquinoline;
3-(5-bromo-2-thienyl)carbonyl-7-chloro-2,4-dioxo-1,2,3,4-tetrahydroquinoline;
7-chloro-2,4-dioxo-3-(1-methylpyrrol-2-yl)carbonyl-1,2,3,4-tetrahydroquinoline;
7-chloro-2,4-dioxo-3-(1-methylpyrrol-3-yl)carbonyl-1,2,3,4-tetrahydroquinoline;
7-chloro-2,4-dioxo-3-(1-methylindol-3-yl)carbonyl-1,2,3,4-tetrahydroquinoline;
7-chloro-3-cyclopropylcarbonyl-2,4-dioxo-1,2,3,4-tetrahydroquinoline;
7-chloro-2,4-dioxo-3-(5-methyl-2-furyl)-1,2,3,4-tetrahydroquinoline;
7-chloro-2,4-dioxo-3-(5-ethyl-2-furyl)-1,2,3,4-tetrahydroquinoline;
7-chloro-2,4-dioxo-3-(1-methylpyrrol-2-yl)-1,2,3,4-tetrahydroquinoline;
7-chloro-2,4-dioxo-3-(2-furyl)-1,2,3,4-tetrahydroquinoline;
3-(4-benzoyl-1-methylpyrrol-2-yl)-7-chloro-2,4-dioxo-1,2,3,4-tetrahydroquinoline;
3-(5-benzoyl-1-methylpyrrol-2-yl)-7-chloro-2,4-dioxo-1,2,3,4-tetrahydroquinoline;
7-chloro-2,4-dioxo-3-[2-(5-methoxyindol-3-yl)ethoxy]carbonyl-1,2,3,4-tetrahydroquinoline;
7-chloro-2,4-dioxo-3-[3-(3-methoxyphenyl)prop-2-ynyloxy]carbonyl-1,2,3,4-tetrahydroquinoline;
7-chloro-2,4-dioxo-3-(3-indol-3-ylpropoxy)carbonyl-1,2,3,4-tetrahydroquinoline;
3-[2-[3,4-bis(methoxymethoxy)phenyl]ethoxy]carbonyl-7-chloro-2,4-dioxo-1,2,3,4-tetrahydroquinoline;
7-chloro-2,4-dioxo-3-[4-(3-hydroxyphenyl)butoxy]carbonyl-1,2,3,4-tetrahydroquinoline;
7-chloro-2,4-dioxo-3-[3-(3-hydroxyphenyl)propoxy]carbonyl-1,2,3,4-tetrahydroquinoline;
7-chloro-2,4-dioxo-3-(2-indol-3-ylethoxy)carbonyl-1,2,3,4-tetrahydroquinoline;
7-chloro-2,4-dioxo-3-[3-(2-hydroxyphenyl)propoxy]carbonyl-1,2,3,4-tetrahydroquinoline;
3-[2-[4-(N-t-butoxycarbonylaminomethyl)phenyl]ethoxy]carbonyl-7-chloro-2,4-dioxo-1,2,3,4-tetrahydroquinoline;
7-chloro-2,4-dioxo-3-[2-(3-thienyl)ethoxy]carbonyl-1,2,3,4-tetrahydroquinoline;
7-chloro-2,4-dioxo-3-[2-(2-thienyl)ethoxy]carbonyl-1,2,3,4-tetrahydroquinoline;
7-chloro-2,4-dioxo-3-[3-(3-hydroxyphenyl)prop-2-ynyloxy]carbonyl-1,2,3,4-tetrahydroquinoline;
7-chloro-2,4-dioxo-3-[3-(3-methoxyphenyl)prop-2-enyloxy]carbonyl-1,2,3,4-tetrahydroquinoline;
7-chloro-2,4-dioxo-3-[2-(phenylthio)ethoxy]carbonyl-1,2,3,4-tetrahydroquinoline;
7-chloro-2,4-dioxo-3-[2-(phenyl)ethylthio]carbonyl-1,2,3,4-tetrahydroquinoline;
7-chloro-2,4-dioxo-3-(2-indol-3-ylethoxy)carbonyl-5-iodo-1,2,3,4-tetrahydroquinoline;
7-chloro-2,4-dioxo-3-(3-methyl-1,2,4-oxadiazol-5-yl)-1,2,3,4-tetrahydroquinoline;
7-chloro-2,4-dioxo-3-[2-(ethoxycarbonyl)ethenyl]-1,2,3,4-tetrahydroquinoline;
7-chloro-2,4-dioxo-1,2,3,4-tetrahydroquinoline-3-hydroxamic acid;
7-chloro-2,4-dioxo-5-iodo-1,2,3,4-tetrahydroquinoline-3-hydroxamic acid;
7-chloro-2,4-dioxo-1,2,3,4-tetrahydroquinoline-3-carboxylic acid hydrazide;
7-chloro-2,4-dioxo-3-(3-thienyl)-1,2,3,4-tetrahydroquinoline;
7-chloro-2,4-dioxo-3-(2-thienyl)-1,2,3,4-tetrahydroquinoline;
7-chloro-2,4-dioxo-3-(2-pyridyl)-1,2,3,4-tetrahydroquinoline;
7-chloro-2,4-dioxo-3-(3-pyridyl)-1,2,3,4-tetrahydroquinoline;
7-chloro-2,4-dioxo-3-(4-pyridyl)-1,2,3,4-tetrahydroquinoline;
3-(2-benzofuryl)-7-chloro-2,4-dioxo-1,2,3,4-tetrahydroquinoline;
3-(3-benzofuryl)-7-chloro-2,4-dioxo-1,2,3,4-tetrahydroquinoline;
7-chloro-2,4-dioxo-3-(3-methyl-2-furyl)-1,2,3,4-tetrahydroquinoline;
7-chloro-2,4-dioxo-3-(2-methyl-3-furyl)-1,2,3,4-tetrahydroquinoline;
7-chloro-2,4-dioxo-3-(4-isopropyl-2-furyl)-1,2,3,4-tetrahydroquinoline;
7-chloro-2,4-dioxo-3-(4-methyl-2-furyl)-1,2,3,4-tetrahydroquinoline;
7-chloro-2,4-dioxo-3-(5-phenyl-2-furyl)-1,2,3,4-tetrahydroquinoline;
7-chloro-2,4-dioxo-3-(5-methyl-2-thienyl)-1,2,3,4-tetrahydroquinoline;
3-(5-benzyl-2-thienyl)-7-chloro-2,4-dioxo-1,2,3,4-tetrahydroquinoline;
3-(3-benzo[b]thienyl)-7-chloro-2,4-dioxo-1,2,3,4-tetrahydroquinoline;
and salts and prodrugs thereof.

In addition, the following compounds are not specifically disclosed in the prior art, and are therefore novel compounds according to the present invention:
7-chloro-3-cyano-2,4-dioxo-1,2,3,4-tetrahydroquinoline;
2,4-dioxo-3-ethoxycarbonyl-6-nitro-1,2,3,4-tetrahydroquinoline;
7-chloro-2,4-dioxo-3-methoxycarbonyl-1,2,3,4-tetrahydroquinoline;
7-chloro-2,4-dioxo-3-n-propoxycarbonyl-1,2,3,4-tetrahydroquinoline;
6,7-dinitro-2,4-dioxo-3-ethoxycarbonyl-1,2,3,4-tetrahydroquinoline;
7-chloro-2,4-dioxo-3-ethoxycarbonyl-1,2,3,4-tetrahydroquinoline;
2,4-dioxo-3-ethoxycarbonyl-7-nitro-1,2,3,4-tetrahydroquinoline;
7-chloro-2,4-dioxo-3-n-pentyloxycarbonyl-1,2,3,4-tetrahydroquinoline;
7-chloro-2,4-dioxo-3-(2-propenyloxy)carbonyl-1,2,3,4-tetrahydroquinoline;
7-chloro-2,4-dioxo-3-ethoxycarbonyl-5-iodo-1,2,3,4-tetrahydroquinoline;
7-chloro-2,4-dioxo-5-ethyl-3-methoxycarbonyl-1,2,3,4-tetrahydroquinoline;
3-carboxy-7-chloro-2,4-dioxo-1,2,3,4-tetrahydroquinoline; and salts and prodrugs thereof.

The present invention also provides pharmaceutical compositions comprising at least one of the novel compounds according to the invention in association with a pharmaceutically acceptable carrier.

The pharmaceutical compositions of this invention are preferably in unit dosage forms such as tablets, pills, capsules, powders, granules, sterile parenteral solutions or suspensions, or suppositories, for oral, parenteral or rectal administration. For preparing solid compositions such as tablets, the principal active ingredient is mixed with a pharmaceutical carrier, e.g. conventional tableting ingredients such as corn starch, lactose, sucrose, sorbitol, talc, stearic acid, magnesium stearate, dicalcium phosphate or gums, and other pharmaceutical diluents, e.g. water, to form a solid preformulation composition containing a homogeneous mixture of a compound of the present invention, or a non-toxic pharmaceutically acceptable salt thereof. When referring to these preformulation compositions as homogeneous, it is meant that the active ingredient is dispersed evenly throughout the composition so that the composition may be readily subdivided into equally effective unit dosage forms such as tablets, pills and capsules. This solid preformulation composition is then subdivided into unit dosage forms of the type described above containing from 0.1 to about 500 mg of the active ingredient of the present invention. The tablets or pills of the novel composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of materials can be used for such enteric layers or coatings, such materials including a number of polymeric acids and mixtures of polymeric acids with such materials as shellac, cetyl alcohol and cellulose acetate.

The liquid forms in which the novel compositions of the present invention may be incorporated for administration orally or by injection include aqueous solutions, suitably flavoured syrups, aqueous or oil suspensions, and flavoured emulsions with edible oils such as cottonseed oil, sesame oil, coconut oil or peanut oil, as well as elixirs and similar pharmaceutical vehicles. Suitable dispersing or suspending agents for aqueous suspensions include synthetic and natural gums such as tragacanth, acacia, alginate, dextran, sodium carboxymethylcellulose, methylcellulose, polyvinyl-pyrrolidone or gelatin.

In the treatment of neurodegeneration, a suitable dosage level is about 0.01 to 250 mg/kg per day, preferably about 0.05 to 100 mg/kg per day, and especially about 0.05 to 5 mg/kg per day. The compounds may be administered on a regimen of 1 to 4 times per day.

The compounds of formula IA above, including the novel compounds according to the invention, may be prepared by a process which comprises the reductive cyclisation of a compound of formula III: 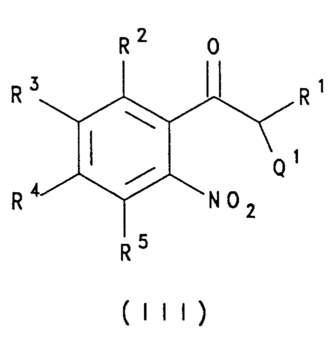
wherein R¹, R², R³, R⁴ and R⁵ are as defined above; and Q¹ represents a reactive carboxylate moiety.

The reaction is conveniently carried out using hydrogen as reducing agent in the presence of a suitable hydrogenation catalyst such as palladium on charcoal, in an inert solvent such as ether, at room temperature at a pressure in the region of 50 p.s.i.

Suitable values for the reactive carboxylate moiety Q¹ include esters, for example C₁₋₄ alkyl esters; acid anhydrides, for example mixed anhydrides with C₁₋₄ alkanoic acids; acid halides, for example acid chlorides; orthoesters; and primary, secondary and tertiary amides.

Preferably, the group Q¹ represents methoxycarbonyl or ethoxycarbonyl.

The intermediates of formula III above may conveniently be prepared by reacting a compound of formula Q¹-CH₂-R¹ with a compound of formula IV: 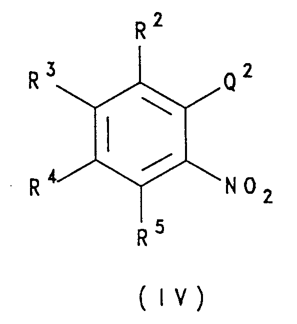
wherein R¹, R², R³, R⁴, R⁵ and Q¹ are as defined above, and Q² represents a reactive carboxylate moiety; in the presence of a strong base.

For example, when R¹ represents a C₂₋₇ alkoxycarbonyl group, e.g. ethoxycarbonyl, the reagent of formula Q¹-CH₂-R¹ is suitably diethyl malonate, and the strong base employed will advantageously be magnesium ethoxide.

Suitable values for the reactive carboxylate moiety Q² correspond to those defined above for Q¹. Preferably, the group Q² is an acid halide group, in particular an acid chloride group. A compound of formula IV wherein Q² represents an acid chloride group may conveniently be prepared from the corresponding compound of formula IV wherein Q² represents a carboxy group -CO₂H by treatment with thionyl chloride under standard conditions well known from the art.

In an alternative process, the compounds of formula IA above, including the novel compounds according to the invention, may be prepared by cyclisation of a compound of formula V: 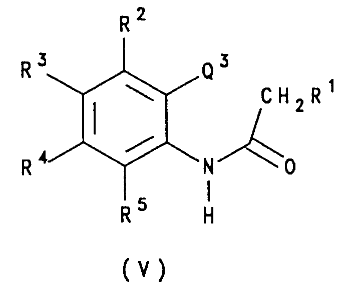
wherein R¹, R², R³, R⁴ and R⁵ are as defined above; and Q³ represents a reactive carboxylate moiety.

The cyclisation is conveniently effected by treatment of the compound of formula V with at least one equivalent of a strong base. The reaction conditions employed will vary depending upon the nature of the strong base utilised. For example, where potassium hexamethyldisilazide is utilised as the strong base, the reaction will suitably be effected at room temperature in tetrahydrofuran. Alternatively, where, for example, the strong base utilised is sodium methoxide or sodium ethoxide, the reaction will suitably be carried out at room temperature in an alcoholic solvent such as methanol or ethanol.

Suitable values for the reactive carboxylate moiety Q³ correspond to those defined above for Q¹. Preferably, the group Q³ is a C₁₋₄ alkyl ester such as methoxycarbonyl or ethoxycarbonyl.

The intermediates of formula V above may conveniently be prepared by reacting a compound of formula Q⁴-CH₂-R¹ with a compound of formula VI: 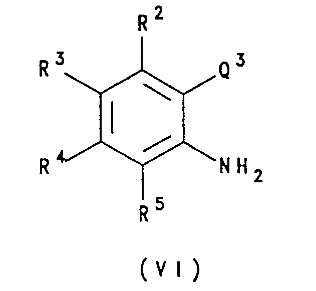
wherein R¹, R², R³, R⁴, R⁵ and Q³ are as defined above; and Q⁴ represents a reactive carboxylate moiety.

The reaction is conveniently effected in dichloromethane at room temperature or in 1,2-dichloroethane at reflux temperature, advantageously in the presence of a mild organic base such as triethylamine and/or 4-dimethylaminopyridine.

Suitable values for the reactive carboxylate moiety Q⁴ correspond to those defined above for Q¹. Preferably, the group Q⁴ is an acid halide group, in particular an acid chloride group. An alternative preferred species of formula Q⁴-CH₂-R¹ is the activated ester derivative formed upon reaction of the carboxy compound HO₂C-CH₂-R¹ with bis(2-oxo-3-oxazolidinyl)-phosphinic chloride (BOP-Cl).

The aromatic intermediates of formulae IV and VI above, including the precursors of formula IV wherein Q² represents -CO₂H, where they are not commercially available, may be prepared by the methods described in the accompanying Examples, or by methods analogous thereto which will be readily apparent to those skilled in the art.

In a further process, the compounds of formula IA above, including the novel compounds according to the invention, may be prepared by reacting a compound of formula Q¹-CH₂-R¹ with a compound of formula VII: 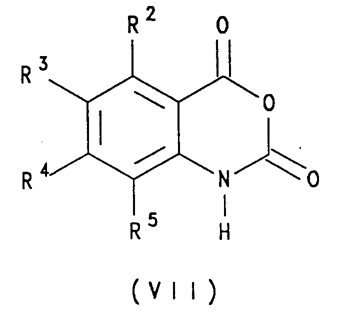
wherein R¹, R², R³, R⁴, R⁵ and Q¹ are as defined above; in the presence of a strong base such as sodium methoxide or sodium ethoxide.

The conditions for effecting this reaction are generally as described in J. Med. Chem., 1976, 41, 825.

Where they are not commercially available, the intermediates of formula Q¹-CH₂-R¹ and Q⁴-CH₂-R¹ may be prepared by methods analogous to those described in the accompanying Examples, or by standard procedures well known from the art.

It will be appreciated that any compound of formula IA or IB initially obtained from any of the above processes may, where appropriate, subsequently be elaborated into a further desired compound of formula IA or IB respectively.

For example, a compound of formula IA or IB initially obtained wherein R¹ represents a group of formula -CO₂R⁶ may subsequently be converted into the desired compound of formula IA or IB wherein R¹ represents a different group -CO₂R⁶ by conventional transesterification procedures known from the art. In particular, it has been found in many cases that this transesterification can be brought about simply by heating the ester of formula IA or IB initially obtained, at a temperature in excess of 130°C, in an alcohol of formula R⁶OH in which R⁶ represents the residue of the ester group -CO₂R⁶ in the desired final product of formula IA or IB, the alcohol R⁶OH itself simultaneously serving as the solvent.

Alternatively, a compound of formula IA or IB wherein R¹ represents a five-membered heteroaromatic ring containing the ring atoms W to Z as defined above may suitably be prepared from a corresponding compound of formula IA or IB wherein R¹ represents -CO₂R⁶ by standard methods well known from the art.

Where the above-described processes for the preparation of the compounds according to the invention give rise to mixtures of stereoisomers, these isomers may be separated by conventional techniques such as preparative chromatography. The compounds may be prepared in racemic form, or individual enantiomers may be prepared either by enantiospecific synthesis or by resolution. The compounds may, for example, be resolved into their component enantiomers by standard techniques, such as the formation of diastereomeric pairs by salt formation with an optically active acid, such as (-)-di-p-toluoyl-d-tartaric acid and/or (+)-di-p-toluoyl-l-tartaric acid followed by fractional crystallization and regeneration of the free base. The compounds may also be resolved by formation of diastereomeric esters or amides, followed by chromatographic separation and removal of the chiral auxiliary.

During any of the above synthetic sequences it may be necessary and/or desirable to protect sensitive or reactive groups on any of the molecules concerned. This may be achieved by means of conventional protecting groups, such as those described in Protective Groups in Organic Chemistry, ed. J.F.W. McOmie, Plenum Press, 1973; and T.W. Greene, Protective Groups in Organic Synthesis, John Wiley & Sons, 1981. The protecting groups may be removed at a convenient subsequent stage using methods known from the art.

The compounds useful in this invention potently and selectively block responses to NMDA and/or AMPA in a brain slice from rat cortex, and inhibit glycine binding to the strychnine-insensitive site present on the NMDA receptor and/or AMPA binding to rat forebrain membranes.

### Cortical Slice Studies

The effects of compounds of the invention on responses to NMDA and AMPA were assessed using the rat cortical slice as described by Wong et al., Proc. Natl. Acad. Sci. USA, 1986, 83, 7104. The apparent equilibrium constant (K_{b}) was calculated from the righthand shift in the NMDA or AMPA concentration-response curves produced by the compound under test. Of those compounds of the accompanying Examples which were tested, all were found to possess a K_{b} value in response to NMDA of below 150 µM. The compounds of Examples 49 and 50 were tested and were found to possess a K_{b} value in response to AMPA of below 150 µM in each case.

### Binding Studies

The ability of test compounds to displace either ³H-glycine binding or ³H-L-689,560 (trans-2-carboxy-5,7-dichloro-4-phenylaminocarbonylamino-1,2,3,4-tetrahydroquinoline) binding to the strychnine-insensitive site present on the NMDA receptor of rat forebrain membranes was determined. ³H-Glycine binding was measured by the method of Donald et al., Proceedings of The British Pharmacological Society, University of Nottingham, September 1988, Abstract P122. For ³H-L-689,560 binding, 100 µg of membrane protein was incubated at 4°C for 45 or 120 min with 50 mM Tris-acetate buffer (pH 7.0) and 1 nM ³H-L-689,560 in a final volume of 0.5 ml. Non-specific binding was determined with 1 mM glycine and bound radioactivity was separated by filtration through Whatman GF/B filters. The concentration of the compounds of the accompanying Examples required to displace 50% of the specific binding of either tritiated ligand (IC₅₀) could thereby be derived. Of those compounds which were tested, all were found to possess an IC₅₀ value of below 50 µM.

The ability of test compounds to displace ³H-AMPA binding to rat forebrain membranes was determined by the method of Honore et al., Neurosci. Lett., 1985, 54, 27. The compound of accompanying Example 50 was tested and was found to possess an IC₅₀ value of below 50 µM.

### Whole-cell Patch-clamp Studies

The ability of test compounds to block the potentiation of NMDA responses by glycine in cultured cortical neurones was measured using patch-clamp techniques as described by Foster and Kemp, J. Neurosci., 1989, 9, 2191. The compound of accompanying Example 26 was tested and was active at concentrations below 20 µM.

### EXAMPLE 1

### Step 1: 2-Ethoxycarbonyl-5-chloro-phenylisocyanate

A solution of phosgene in toluene (36.3ml x 20%) was added dropwise over 30 min to a stirred solution of ethyl-4-chloroanthranilate (10.0g) and triethylamine (15ml) in dry tetrahydrofuran at -5°C. The temperature was maintained at -5°C for 1h and then allowed to warm to room temperature. After 1h ether (200ml) was added and the solution filtered. Removal of solvent under reduced pressure afforded the product as a white solid (11.2g). δ (CDCl₃) 1.39 (3H, t, *J* = 7.5Hz, CH₂CH₃), 4.34 (2H, q, *J* = 7.5Hz, CH₂CH₃), 7.22 (1H, d, *J* =CH₂CH₃, 1.0Hz, H-6), 7.28 (1H, dd, *J* = 6.5 and 1.0Hz, H-4), 8.31 (1H, d, *J* = 6.5Hz, H-3); m/z (EI⁺) 225.

### Step 2: 3-(3-Thienoyl)-7-chloro-2,4-dioxo-1,2,3,4-tetrahydroquinoline

3-Acetylthiophene (1.1g) in dry tetrahydrofuran (10ml) was added dropwise to a solution of lithium hexamethyldisilazide (9.0ml x 10mol) in dry tetrahydrofuran (20ml) at -78°C. The resulting solution was allowed to warm to 0°C over 30 min and then re-cooled to -78°C. A solution of 2-ethoxycarbonyl-5-chlorophenylisocyanate (1.0 g) in dry tetrahydrofuran (10ml) was added dropwise over 15 min. The temperature was maintained at -78°C for 30 min and then allowed to warm to room temperature. After 2h the reaction mixture was poured into IN HCL (50ml), extracted with ether (4 x 50ml), washed with water (50ml) and brine (50ml), dried over MgSO₄, filtered and the solvent removed under reduced pressure to afford a brown solid. Recrystallisation from methanol gave the product as pale yellow needles (740mg) mp 260-263°C; δ (CDCl₃) 1.4 (3H, t, *J* = 7.5Hz, CH₂CH₃), 4.02 (2H, s, CH₂CO), 4.4 (2H, q, *J* = 7.5Hz, CH₂CH₃), 7.05 (1H, dd, *J* = 8.5 and 1.0Hz, H-6), 7.1 (1H, m, 5-CH-CH), 7.4 (1H, m, 5-CH-CH), 7.45 (1H, d, *J* = 8.5Hz, H-5), 8.01 (1H, m, 5-CH-C), 8.8 (1H, d, *J* = 1.0Hz, H-8). Sodium methoxide (61.4g) was added to a suspension of this compound (200mg) in dry methanol (20ml). The resulting mixture was warmed to reflux for 18h. The reaction mixture was cooled to room temperature and poured into IN HCl (50ml). The resulting precipitate was filtered off and recrystallised from dimethylformamide to afford the product as pale yellow needles. mp 313-320°C (dec); (Found: C, 54.67; H, 2.63; N, 4.66 C₁₄H₈NO₃SCl+0.1H₂O requires C, 54.67; H, 2.68; N, 4.55%); δ (d₆-DMSO) 7.23 (1H, dd, *J* = 9.5, 1.0Hz, H-6), 7.33 (1H, d, *J* = 1.0Hz, H-8), 7.45 (1H, dd, *J* = 5.5 and 1.0Hz, 5-CH-CH), 7.56 (1H, dd, *J* = 5.5 and 2.0Hz, 5-CH-CH), 7.95 (1H, d, *J* = 9.5Hz, H-5), 8.26 (1H, dd, *J* = 2.0 and 1.0Hz, 5-CH-C); m/s (EI⁺) 305.
The following compounds were made by the same method (Method A), typified by Example 1, Step 2 above.

### EXAMPLE 2

### 7-Chloro-3-(2-furanoyl)-2,4-dioxo-1,2,3,4-tetrahydroquinoline

Yellow needles mp 310°C; (Found: C, 57.90; H, 2.70; N, 4.65; C₁₄H₈ClNO₄ requires C, 58.05; H, 2.78; N, 4.84%); δ (d₆ DMSO) 6.69 (1H, dd, *J* = 3.6 and 1.5Hz -OCHCH-), 7.24 (1H, dd, *J* = 8.5 and 2.0Hz, H-6), 7.33 (2H, -OCHCHCH-, H-8), 7.94 (1H, d, *J* = 8.5Hz, H-5), 7.99 (1H, d, *J* = 1.5Hz, -OCH), 11.57 (1H, br s, NH).

### EXAMPLE 3

### 7-Chloro-2,4-dioxo-3-(2-thienoyl)-1,2,3,4-tetrahydroquinoline

Yellow needles mp 314-315°C; (Found: C, 54.68; H, 2.52; N, 4.54; C₁₄H₈CLNO₃S. 0.05 H₂O requires C, 54.84; H, 2.66; N, 4.57%); δ (d₆ DMSO) 7.18 (1H, dd, *J* = 5.0Hz and 4.0Hz, -5CHCH-), 7.31 (1H, dd, *J* = 8.5Hz and 2.0Hz, H-6), 7.34 (1H, d, *J =* 2.0Hz, H-8), 7.71 (1H, dd, *J* = 4.0Hz and 1.0Hz, -SCHCHCH-), 7.52 (1H, d, *J* = 8.5Hz, H-5), 8.02 (1H, dd, *J* = 5.0Hz and 1.0Hz, -SCH), 11.64 (1H, br s, NH).

### EXAMPLE 4

### 7-Chloro-3-[3-(2,5-dimethylfuranoyl)]-2,4-dioxo-1,2,3,4-tetrahydroquinoline

Yellow needles mp 254-256°C; (Found: C, 60.22; H, 3.80; N, 4.43. C₁₆H₁₂ClNO₄ requires C, 60.48; H, 3.81; N, 4.41%); δ (DMSO d₆) 2.21 (3H, s, -CH₃), 2.40 (3H, s, CH₃), 6.20 (1H, s, OCCH), 7.24 (1H, dd, *J* = 8.5Hz and 2.0Hz, H-6), 7.32 (1H, d, *J* = 2.0Hz, H-8), 7.94 (1H, d, *J* = 8.5Hz, H-5), 11.58 (1, br s, NH). m/z (EI⁺) 317 (M⁺).

### EXAMPLE 5

### 7-Chloro-3-[2-(5-methylfuranoyl)]-2,4-dioxo-1,2,3,4-tetrahydroquinoline

Yellow needles mp 272°C (dec); (Found: C, 59.33; H, 3.09; H, 4.57. C₁₅H₁₄ClNO₄ requires C, 59.32; H, 3.32; N, 4.61%) δ (DMSO d₆) 2.38 (3H, s, -CH₃), 6.35 (1H, d, *J* = 3.5Hz, MeCCH), 7.24 (1H, dd, *J* = 8.5Hz and 2.0Hz, H-6), 7.26 (1H, d, *J* = 3.5Hz, MeCCHCH), 7.32 (1H, d, *J* = 2.0Hz, H-8), 7.92 (1H, d, *J* = 8.5Hz, H-5), 11.58 (1H, br s, -NH); m/z (EI⁺) 303 (M⁺).

### EXAMPLE 6

### 7-Chloro-3-[2-(5-nitrobenzofuranoyl)]-2,4-dioxo-1,2,3,4-tetrahydroquinoline

Yellow needles mp 320°C (dec); (Found: C, 55.96; H, 2.14; N, 7.36. C₁₈H₉Cl N₂O₆ requires C, 56.20; H, 2.36; N, 7.28%), 7.27 (1H, dd, *J* = 8.5Hz and 2.0Hz, H-6), 7.35 (1H, d, *J* = 2.0Hz, H-8), 7.92 (1H, s, benzofuran H-3), 7.97 (1H, d, *J* = 8.5Hz, H-5), 7.99 (1H, d, *J* = 9.0Hz, benzofuran H-7), 8.38 (1H, dd, *J* = 9.0Hz and 2.5Hz, benzofuran H-6), 8.76 (1H, d, *J* = 2.5Hz, benzofuran H-4), 11.66 (1H, br s, NH); m/z (CI⁺, NH₃), 385 (M⁺+H).

### EXAMPLE 7

### 3-(2-Benzofuranoyl)-7-chloro-2,4-dioxo-1,2,3,4-tetrahydroquinoline

Yellow needles mp 295°C; (Found: C, 65.58; H, 2.84; N, 4.08. C₁₈H₁₀NO₄ requires C, 65.64; H, 2.97; N, 4.12%); δ (DMSO d₆) 7.26 (1H, dd, *J* = 8.5Hz and 2.0Hz, H-6), 7.35 (2H, m, *J* = 7.5Hz, *J* = 2.0Hz, Ar-H, H-8), 7.56 (1H, td, *J* = 8.5Hz and 1.0Hz, Ar-H), 7.72 (1H, d, *J* = 8.5Hz, Ar-H), 7.75 (1H, s, OCCH), 7.79 (1H, d, *J* = 7.5Hz, Ar-H), 7.96 (1H, d, *J* = 8.5Hz, H-5), 11.65 (1H, br s, NH); m/z (EI⁺) 339 (M⁺).

### EXAMPLE 8

### 3-(3-Benzo[b]thienoyl)-7-chloro-2,4-dioxo-1,2,3,4-tetrahydroquinoline

A solution of 3-acetylbenzo[b]thiophene (1.54g) in dry tetrahydrofuran (10ml) was added dropwise to a solution of potassium hexamethyldisilazide (17.5ml x 0.5M) in dry tetrahydrofuran at -78°C. The resulting solution was allowed to warm to 0°C over 30 min and then re-cooled to -78°C. A solution of 2-ethoxycarbonyl-5-chlorophenyl isocyanate (1.0g) in dry tetrahydrofuran (10ml) was added dropwise over 15 min. The temperature was maintained at -78°C for 30 min, and then warmed to room temperature. After 2h the reaction mixture was poured into IN HCl (100ml) and the precipitate filtered off. Recrystallisation from dimethylformamide afforded the product as yellow needles. mp 306-307°C; (Found: C, 59.85; H, 2.69; N, 3.99. C₁₈H₁₀NO₃SCl 0.3H₂O requires C, 59.85; H, 2.95; N, 3.88%); δ (d₆-DMSO) 7.20 (1H, dd, *J* = 6.0 and 1.0Hz, H-6), 7.36 (1H, d, *J* = 1.0Hz, H-8), 7.48-7.52 (2H, m, H-5_{'} and H-6_{'}), 7.96 (1H, d, *J* = 6.0Hz, H-5), 8.01 (1H, d, *J* = 5.0Hz, H-4_{'}), 8.45 (1H, s, H-2_{'}), 8.47 (1H, d, *J* = 5.0Hz, H-7_{'}); m/z (EI⁺) 355.
The following compounds were made by the same general method(Method B), typified by Example 8 above.

### EXAMPLE 9

### 3-(2-Phenylacetyl)-7-chloro-2,4-dioxo-1,2,3,4-tetrahydroquinoline

White needles m.p. 174-176°C; (Found: C, 62.28; H, 4.01; N, 4.87. C₁₇H₁₇NO₃Cl+0.75H₂O requires: C, 62.40; H, 4.15; N, 4.30%); δ (CDCl₃), 3.80 (2H, s, Ar-CH₂), 7.05 (1H, dd, *J* = 6.0 and 1.0Hz, H-6), 7.0 (5H, m, 5 x Ar-H), 7.99 (1H, d, *J* = 6.0Hz, H-5), 8.82 (1H, d, *J* = 1.0Hz, H-8), 10.6 (1H, br s, NH).

### EXAMPLE 10

### 3-(2-[5-Methylthienoyl])-7-chloro-2,4-dioxo-1,2,3,4-tetrahydroquinoline

White needles m.p. 302-303°C; (Found: C, 55.74; H, 2.80; N, 4.49. C₁₅H₁₀NO₃SCl+0.1H₂O requires: C, 56.03; H, 3.19; N, 4.36%); δ (d₆-DMSO) 2.51 (3H, s, CH₃), 6.90 (1H, d, *J* = 1.5Hz, CH₃C-CH), 7.24 (1H, dd, *J* = 5.5 and 1.0Hz, H-6), 7.31 (1H, d, *J* = 1.0Hz, H-8), 7.54 (1H, d, *J* = 1.5Hz, SC-CH), 7.93 (1H, d, *J* = 5.5Hz, H-5); m/z (EI⁺) 318.

### EXAMPLE 11

### 3-(2-[3-Methylthienoyl])-7-chloro-2,4-dioxo-1,2,3,4-tetrahydroquinoline

White needles m.p. 260-261°C; (Found: C, 55.59; H, 2.97; N, 4.57: C₁₅H₁₀NO₃SCl+0.2H₂O requires: C, 55.71; H, 3.24; N, 4.33%); δ (d₆-DMSO) 2.4 (3H, s, CH₃), 7.05 (1H, d, *J* = 4.5Hz, CH₃C-CH), 7.25 (1H, dd, *J* = 6.5 and 1.0Hz, H-6), 7.3 (1H, d, *J* = 1.0Hz, H-8), 7.85 (1H, d, *J* = 4.5Hz, 5_{'}-CH), 7.95 (1H, d, *J* = 6.5Hz, H-5), 11.85 (1H, s, NH); m/z (EI⁺) 318.

### EXAMPLE 12

### 3-(3-[2,5-Dimethylthienoyl])-7-chloro-2,4-dioxo-1,2,3,4-tetrahydroquinoline

White needles m.p. 224-225°C; (Found: C, 57.60; H, 3.25; N, 4.05. C₁₆H₁₂NO₃SCl requires: C, 57.57; HG, 3.62; N, 4.20%); δ (d₆-DMSO) 2.25 (3H, s, CH₃), 2.5 (3H, s, CH₃), 6.8 (1H, s, 5-CCH), 7.2 (1H, dd, *J* = 7.0 and 1.0Hz, H-6), 7.25 (1H, d, *J* = 1.0Hz, H-8), 7.95 (1H, d, *J* = 7.0Hz, H-5), 11.6 (1H, s, NH); m/z (EI⁺) 333.

### EXAMPLE 13

### (E)-3-(3-[3-Thienyl]-2-propen-1-oyl)-7-chloro-2,4-dioxo-1,2,3,4-tetrahydroquinoline

White needles m.p. 320-322°C; (Found: C, 57.72; H, 2.84; N, 4.21. C₁₆H₁₀NO₃SCl requires: C, 57.92; H, 3.04; N, 4.22%); δ (d₆-DMSO), 7.24 (1H, dd, *J* = 7.0 and 1.0Hz, H-6), 7.31 (1H, s, CH-CHCH), 7.46 (1H, d, *J* = 1.0Hz, H-8), 7.66 (1H, d, *J* = 4.5Hz, 5-CH-CH), 7.93 (1H, d, *J* = 9.5Hz, C-CHCH-CO), 7.96 (1H, d, *J* = 7.0Hz, H-5), 8.01 (1H, d, *J* = 4.5Hz, 5-CH-CH), 8.34 (1H, d, *J* = 9.5Hz, C-CH-CH-CO); m/z (EI⁺) 331.

### EXAMPLE 14

### 3-(2-[5-Bromothienoyl])-7-chloro-2,4-dioxo-1,2,3,4-tetrahydroquinoline

White needles m.p. 309-310°C; (Found: C, 43.75; H, 1.64; N, 3.72. C₁₄H₇NO₃Cl Br requires: C, 43.71; H, 1.83; N, 3.64%); δ (d₆-DMSO) 7.25 (1H, dd, *J* = 6.0 and 1.0Hz, H-6), 7.32 (1H, d, *J* = 3.0Hz, Br-CH), 7.34 (1H, d, *J* = 1.0Hz, H-8), 7.59 (1H, d, *J* = 3.0Hz, SC-CH), 7.94 (1H, d, *J* = 6.0Hz, H-5); m/z (EI⁺) 384.

### EXAMPLE 15

### 7-Chloro-3-[2-(1-methylpyrroloyl)]-2,4-dioxo-1,2,3,4-tetrahydroquinoline

Yellow needles m.p. > 300°C; (Found: C, 59.13; H, 3.72; N, 9.03; C₁₅H₁₁ClN₂O₃+0.1 H₂O requires C, 59.16; H, 3.71; N, 9.20%); δ (d₆ DMSO) 3.94 (3H, s, -CH₃), 6.05 (1H, dd, *J* = 4.0Hz and 2.5Hz, -NHCHCH), 6.63 (1H, dd, *J* = 4.0Hz and 1.5Hz, -NC HCHCH), 7.17 (1H, dd, *J =* 2.5Hz and 1.5Hz, -NCH), 7.21 (1H, dd, *J* = 8.5Hz and 2.0Hz, H-6), 7.30 (1H, d, *J* = 2.0Hz, H-8), 7.90 (1H, d, *J* = 8.5Hz, H-5), 11.48 (1H, br s, NH); m/z (EI⁺) 302 (M⁺).

### EXAMPLE 16

### 7-Chloro-3-[3-(1-methylpyrroloyl)]-2,4-dioxo-1,2,3,4-tetrahydroquinoline

Yellow needles m.p. 272°C dec; (Found: C, 59.33; H, 3.38; N, 9.01; C₁₅H₁₁ClN₂O₃ requires C, 59.52; H, 3.66; N, 9.25%); δ (d₆ DMSO) 3.65 (3H, s, -CH₃), 6.58 (1H, m, NCHCH), 6.78 (1H, m, NCHCH), 7.23 (1H, dd, *J* = 8.5Hz and 2.0Hz, H-6), 7.31 (1H, d, *J* = 2.0Hz, C-8), 7.70 (1H, br s, NCH), 7.94 (1H, br s, NCH), 7.94 (1H, d, *J* = 8.5Hz, H-5), 11.42 (1H, br s, NH); m/z (EI⁺) 302 (M⁺).

### EXAMPLE 17

### 7-Chloro-[3-(1-methylindoloyl)]-2,4-dioxo-1,2,3,4-tetrahydroquinoline

Yellow needles, m.p. 282°C (dec); (Found: C, 64.55; H, 3.35; H, 7.85 C₁₉H₁₃ClN₂O₄ requires C, 64.69; H, 3.71; N, 7.94%); δ (DMSO d₆), 3.84 (3H, s, -CH₃), 7.22-7.32 (3H, m, 3 x Ar-H), 7.35 (1H, d, *J* = 2.0Hz, H-8), 7.55 (1H, dd, *J* = 6.0Hz and 2.0Hz, Ar-H), 7.96 (1H, d, *J* = 8.5z, H-5), 8.23 (2H, m, Ar-H, NCH), 11.52 (1H, br s, NH); m/z (EI⁺) 352 (M⁺).

### EXAMPLE 18

### 3-(Cyclopropanecarbonyl)-7-chloro-2,4-dioxo-1,2,3,4-tetrahydroquinoline

White needles m.p. 227-230°C; (Found: C, 58.98; H, 3.81; N, 5.19. C₁₃H₁₀NO₃Cl requires C, 59.22; H, 3.82; N, 5.31%); δ (d₆-DMSO), 1.2-1.28 (4H, m, CH₂CH₂), 3.96-4.01 (1H, m, COCHCH₂), 7.26 (1H, dd, *J* = 9.0 and 1.5Hz, H-6), 7.31 (1H, d, *J* = 1.5Hz, H-8), 7.97 (1H, d, *J* = 9.0Hz, H-5), 11.63 (1H, br s, NH); m/z (EI⁺) 264.

### EXAMPLE 19

### 3-(5'-[2'-Methylfuranyl])-7-chloro-2,4-dioxo-1,2,3,4-tetrahydroquinoline

Triethylamine (1.04ml) was added to a stirred solution of bis(2-oxo-3-oxazolidinyl)phosphinic chloride (1.03g), methyl-4-chloroanthranilate (0.68g) and 5-methylfuranyl-2-acetic acid (0.52g) in 1,2-dichloroethane (50ml). The solution was warmed to reflux overnight. The reaction mixture was cooled to room temperature and washed with water. The organic layer was separated, dried over MgSO₄, filtered and the solvent removed under reduced pressure. Chromatography on silica gel using 10% ethyl acetate in n-hexane as eluant afforded the product as a white solid (0.66g) δ (CDCl₃) 2.28 (3H, s, Ar-CH₃), 3.86 (2H, s, CH₂CONAr), 3.95 (3H, s, CO₂CH₂), 5.95 (1H, d, *J* = 2.0Hz, CH₃C-CHCH), 6.05 (1H, d, *J* = 2.0Hz, CH₃C-CHCH), 7.01 (1H, dd, *J* = 7.0 and 1.0Hz, ArH), 7.95 (1H, d, *J* = 7.0Hz, ArH), 8.80 (1H, d, *J* = 1.0Hz, ArH). Potassium hexamethyldisilazane (11.1ml of a 0.5mol solution in toluene) was added to a stirred solution of this compound (0.66g) in dry tetrahydrofuran (20ml) at room temperature. The resulting solution was stirred at room temperature for 1.5 hours. At the end of this time methanol (20ml) was added and the solution evaporated to dryness under reduced pressure. The residual solid was redissolved in methanol (20ml) and 1N HCl added. The resulting precipitate was filtered off and recrystallised from dimethylformamide to afford the title compound as white needles (0.26g) m.p. 281-282°C (Found: C, 60.44; H, 3.55; N, 4.84 C₁₄H₁₀MO₃Cl+0.1H₂O requires C, 60.60; H, 3.70; N, 5.04%), δ (d₆-DMSO), 2.55 (3H, s, ArCH₃), 6.19 (1H, d, *J* = 1.0Hz, CH₃C-CHCH), 6.86 (1H, d, *J* = 1.0Hz, CH₃-C-CHCH), 7.21 (1H, dd, *J* = 6.0 and 1.0Hz, H-6), 7.24 (1H, d, *J* = 1.0Hz, H-8), 7.91 (1H, d, *J* = 6.0Hz, H-5).
The following compounds were made by the same method (Method C), typified by Example 19 above.

### EXAMPLE 20

### 3-(2'[5'-Ethylfuryl])-7-chloro 2,4 dioxo-1,2,3,4-Tetrahydroquinoline

White needles m.p. 263-265°C (Found: C, 62.48; H, 3.81; N, 4.58. C₁₅H₁₂NO₃Cl requires C, 62.19; H, 4.18; N, 4.83%); δ (d₆-DMSO) 1.30 (3H, t, *J* = 7.0Hz, CH₂ CH₃), 2.77 (2H, q, *J* = 7.0Hz, CH₂CH₃), 6.18 (1H, d, *J* = 1.5Hz, CH₃-CCH), 7.14 (1H, dd, *J* = 7.0 and 1.0Hz, H-6), 7.31 (1H, d, *J* = 1.5Hz, CHCHC), 7.34 (1H, d, *J* = 1.0Hz, H-8), 7.91 (1H, d, *J* = 7.0Hz, H-5); m/z (EI⁺) 289.

### EXAMPLE 21

### 3-(2'-[1'-Methylpyrrolyl])-7-chloro-2,4-dioxo-1,2,3,4-tetrahydroquinoline

White needles m.p. 340°C; (Found: C, 59.60; H, 4.11; N, 10.02 C₁₄H₁₁N₃O₂Cl requires C, 59.65; H, 4.22; N, 9.94%); δ (d₆-DMSO) 3.41 (3H, s, NCH₃), 5.97-6.02 (1H, m, H-5'), 6.05-6.10 (1H, m, H-4'), 6.84-6.91 (1H, m, H-3'), 7.20 (1H, dd, *J* = 7.2 and 2.0Hz, H-6), 7.32 (1H, d, *J* = 2.0Hz, H-8), 7.88 (1H, d, *J* = 7.2Hz, H-5), 10.18 (1H, br s, NH).

### EXAMPLE 22

### 3-(2'-Furanyl)-7-chloro-2,4-dioxo-1,2,3,4-tetrahydroquinoline

White needles m.p. 246-250°C; δ (d₆-DMSO) 6.60 (1H, m, H-4'), 6.98 (1H, dd, *J* = 4.0 and 1.0Hz, H-3'), 7.24 (1H, dd, *J* = 9.0 and 1.5Hz, H-6), 7.34 (1H, d, *J* = 1.5Hz, H-8), 7.74 (1H, d, *J* = 1.0Hz, H-5'), 7.96 (1H, d, *J* = 9.0Hz, H-5), 11.66 (1H, s NH).

### EXAMPLE 23

### 3-(2'-[4'-Benzoyl-1'-methylpyrrolyl])-7-chloro-2,4-dioxo 1,2,3,4 Tetrahydroquinoline

White needles m.p. 282-284°C (Found: C, 65.49; H, 3.80; N, 7.09. C₂₁H₁₅N₂O₃Cl requires C, 65.64; H, 4.09; N, 7.29%); δ (d₆-DMSO) 3.50 (3H, s. NCH₃). 6.48 (1H, d, *J* = 1.0Hz, COCCHC), 7.08 (1H, dd, *J* = 6.0 and 1.0Hz, H-6), 7.17 (1H, d, *J* = 1.0Hz, CH₃N-CH), 7.44-7.62 (3H, m, 3 x Ar-H), 7.75-7.81 (2H, m, 2 x Ar-H), 7.96 (1H, d, *J* = 6.0Hz, H-6); m/z (EI⁺) 378.

### EXAMPLE 24

### 3-(2'-[5'-Benzoyl-1'-methylpyrroloyl])-7-chlor-2,4-dioxo 1,2,3,4 Tetrahydroquinoline

White needles m.p. 295-296°C (Found: C, 65.30; H, 3.89; N, 7.13. C₂₁H₁₅N₂O₃Cl + 0.4H₂O requires C, 65.34; H, 4.12; N, 7.25%); δ (d₆-DMSO) 3.78 (3H, s, NCH₃), 6.08 (1H, d, *J* = 1.0Hz, C-CHCH), 6.70 (1H, d, *J* = 1.0Hz, C-CHCH), 7.24 (1H, dd, *J* = 7.5 and 1.0Hz, H-6), 7.4-7.48 (3H, m, 3 x Ar-H), 7.6-7.7 (2H, m, 2 x ArH), 7.95 (1H, d, *J* = 7.5Hz, H-5); m/z (EI⁺) 378.

### EXAMPLE 25

### Ethyl 7-Chloro-2,4-dioxo-3-(1,2,3,4-tetrahydroquinoline)carboxylate

Ethyl malonyl chloride (26ml) in dry dichloromethane (100ml) was added dropwise over 1h to a stirred solution of ethyl 4-chloroanthranilate (20.4g), triethylamine (43ml) and 4-dimethylaminopyridine (0.5g) in dry dichloromethane (300ml) at 0°C. After 1h triethylamine (43ml) was added, then ethyl malonyl chloride (26ml) in dry dichloromethane (100ml) added over 1h. The suspension was evaporated in vacuo, diluted with ethyl acetate, washed twice with 1M citric acid, then saturated sodium bicarbonate solution, water and brine, dried (MgSO₄) and evaporated in vacuo to give an oil (53g). 47g of this oil was dissolved in ethanol (500ml) then sodium methoxide (16.2g) was added. After 30 min the mixture was diluted with water, washed with ethyl acetate, and the aqueous layer acidified with concentrated hydrochloric acid. The resulting precipitate was collected and dried to give 19g of white solid. 17g of this solid was recrystallised from dimethyl formamide (300ml), keeping the temperature below 100°C, then washed with water, ethanol and ether, and dried to give the ester (8.81g) as white needles, m.p. >340°C; (Found: C, 53.68; H, 3.78; N, 5.28. C₁₂H₁₀NO₄Cl requires C, 53.85; H, 3.77; N, 5.23%), δ (d₆-DMSO) 13.3 (1H, br s), 11.58 (1H, s), 7.92 (1H, d, *J* = 8.6 Hz, H-5), 7.30 (1H, d, *J* = 1.9 Hz, H-8), 7.24 (1H, dd, *J* = 1.9 and 8.6 Hz, H-6), 4.33 (2H, q, *J* = 7.1 Hz, CH₂), 1.30 (3H, t, *J* 7.1 Hz, CH₃); m/z (EI⁺) 267 (M⁺).

### EXAMPLE 26

### Methyl 7-Chloro-2,4-dioxo-3-(1,2,3,4-tetrahydroquinoline)carboxylate

Ethyl 7-chloro-2,4-dioxo-3-(1,2,3,4-tetrahydroquinoline)carboxylate (0.42g) and dry methanol (25ml) were heated in a sealed apparatus with an internal temperature of 130°C for 20 min. The mixture was cooled, the solid collected and recrystallised from dimethylformamide, keeping the temperature below 110°C, to give the ester (107mg) as white needles, sublimes 240°C; (Found: C, 52.04; H, 3.27; N, 5.63. C₁₁H₈NO₄Cl requires C, 52.09; H, 3.18; N, 5.52%); δ (d₆-DMSO) 11.60 (1H, s), 7.93 (1H, d, *J* = 8.6 Hz, H-5), 7.29 (1H, d, *J* = 2.0 Hz, H-8), 7.24 (1H, dd, *J* = 2.0 and 8.6 Hz, H-6), 3.85 (3H, s, CH₃); m/z (EI⁺) 253 (M⁺).

### EXAMPLE 27

### Propyl 7-Chloro-2,4-dioxo-3-(1,2,3,4-tetrahydroquinoline)carboxylate

Ethyl 7-chloro-2,4-dioxo-3-(1,2,3,4-tetrahydroquinoline)carboxylate (0.42g) and 1-propanol (4ml) were heated in a sealed apparatus with an internal temperature of 160°C for 20 min. The mixture was cooled and the ester collected as fine white needles, m.p. >320°C (Found: C, 55.39; H, 4.32; N, 4.96. C₁₃H₁₂NO₄Cl requires C, 55.43; H, 4.29; N, 4.97%); δ (d₆-DMSO) 11.48 (1H, s), 7.92 (1H, d, *J* = 8.6 Hz, H-5), 7.29 (1H, d, *J* = 0.6 Hz, H-8), 7.23 (1H, dd, *J* = 0.6 and 8.6 Hz, H-6), 4.23 (2H, t, *J* = 7 Hz, OCH₂), 1.69 (2H, sextet, *J* = 7 Hz, OCH₂CH₂), 0.97 (3H, t, *J* = 7 Hz, CH₃); m/z (CI⁺, NH₃) 282 (M⁺+H).

### EXAMPLE 28

### 7-Chloro-3-cyano-2,4-dioxo-1,2,3,4-tetrahydroquinoline

n-Butyl chloroformate (1.5ml) was added to cyanoacetic acid (1g) and triethylamine (1.65ml) in dry dichloromethane (20ml) at -23°C. Alter 15 min ethyl 4-chloroathranilate (901mg), triethylamine (1.65ml) and 4-dimethylaminopyridine (100mg) in dry dichloromethane (7ml) were added. After stirring at -23°C for 15 min the mixture was brought to 0°C for 1h, then diluted with ethyl acetate, washed with 1M citric acid, saturated sodium bicarbonate solution, water, and brine, dried (MgSO₄), evaporated in vacuo, and purified by flash chromatography to give ethyl 2-cyanoacetamido-4-chlorobenzoate as a white solid (214mg). 190mg of this solid was suspended in methanol (10ml) and sodium methoxide (50mg) added. After stirring at room temperature for 30 min the mixture was acidified with 1M hydrochloric acid, extracted with ethyl acetate (x3), the combined organic layers washed with water and brine, dried (MgSO₄) and evaporated in vacuo to give the tetrahydroquinoline (81mg) as long needles m.p. 314-316°C (from dimethylformamide/ethyl acetate/hexanes); (Found: C, 54.27; H, 2.41; N, 12.70. C₁₀H₅N₂O₂Cl requires C, 54.44; H, 2.28; N, 12.70%); δ (d₆-DMSO) 11.63 (1H, s), 7.97 (1H, d, *J* = 8.6 Hz, H-5), 7.29 (1H, d, *J* = 1.8 Hz, H-8), 7.24 (1H, dd, *J* = 1.8 and 8.6 Hz, H-6); m/z (CI⁺, NH₃) 221 (M⁺+H).
The following examples were made by the same method (Method D): Typically, ethyl 7-chloro-2,4-dioxo-3-(1,2,3,4-tetrahydroquinoline)carboxylate (0.4g), and the relevant alcohol (1g) were heated together at 140°C for 20 min, cooled, ethyl acetate added, and the solid collected, dried and recrystallised.

### EXAMPLE 29

### 3-(4-Hydroxyphenyl)-1-propyl 7-Chloro-2,4-dioxo-3-(1,2,3,4-tetrahydroquinoline)carboxylate

White solid, m.p. 210-211°C (from dimethylformamide/acetone/water); (Found: C, 60.92; H, 4.42; N, 3.78. C₁₉H₁₆NO₅Cl requires C, 61.05; H, 4.32; N, 3.75); δ (d₆-DMSO) 13.3 (1H, br s), 11.55 (1H, s), 9.11 (1H, s), 7.93 (1H, d, *J* = 8.7 Hz, H-5), 7.31 (1H, d, *J* = 1.7 Hz, H-8), 7.24 (1H, dd, *J* = 1.7 and 8.7 Hz, H-6), 7.02 (2H, d, *J* = 8.4 Hz, CHCHCOH), 6.67 (2H, d, *J* = 8.4 Hz, CHCOH), 4.24 (2H, t, *J* = 6.5 Hz, CH₂OCO), 2.63 (2H, t, *J* = 6.5 Hz, CH₂Ar), 1.92 (2H, quintet, *J* = 6.5 Hz, CH₂CH₂CH₂); m/z (CI⁺, NH₃) 239 (M⁺-C₉H₁₁O+H)

### EXAMPLE 30

### 2-(3-Hydroxyphenyl)-1-ethyl 7-Chloro-2,4-dioxo-3-(1,2,3,4-tetrahydroquinoline)carboxylate

White needles, m.p. 257-259°C (from dimethylformamide/acetone/water) (Found: C, 59.92; H, 3.99; N, 3.95. C₁₈H₁₄NO₅Cl requires C, 60.09; H, 3.92; N, 3.89%); δ (d₆-DMSO) 13.3 (1H, br s), 11.58 (1H, s), 9.27 (1H, s), 7.93 (1H, d, *J* = 8.7 Hz, H-5), 7.29 (1H, d, *J* = 1.8 Hz, H-8), 7.24 (1H, dd, *J* = 1.8 and 8.7 Hz, H-6), 7.08 (1H, t, *J* = 7.8 Hz, CHCHCOH), 6.76 (1H, d, *J* = 7.8 CHCHCHCOH), 6.71 (1H, d, *J* = 1 Hz, CCHCOH), 6.62 (1H, dd, *J* = 1 and 7.8 Hz, CHCHCOH), 4.44 (2H, t, *J* = 7.2 Hz, CH₂OCO), 2.92 (2H, t, *J* = 7.2 Hz, CH₂Ar); m/z (EI⁺) 359 (M⁺).

### EXAMPLE 31

### 2-(5-Methoxy-3-indole)ethyl 7-Chloro-2,4-dioxo-3-(1,2,3,4-tetrahydroquinoline)carboxylate

White plates m.p. 229-231° C (from dimethylformamide/acetone); (Found: C 60.27; H, 4.28; N, 7.22. C₂₁H₁₇N₂O₅Cl + 0.3 dimethylformamide requires C, 60.50; H, 4.42; N, 7.41%); δ (d₆ - DMSO) 13.3 (1H, br s), 11.62 (1H, s), 10.74 (1H, s), 7.93 (1H, d, *J* = 8.6Hz, H-5), 7.35-7.30 (2H, m, H-8 and indole H-2), 7.25 (1H, dd, *J* = 2.0 and 8.6Hz, H-6), 7.22 (1H, d, *J* = 8.7Hz, indole H-7), 7.08 (1H, d, *J* = 2.2Hz, indole H-4), 6.70 (1H, dd, *J* = 2.0 and 8.7Hz, indole H-6), 4.49 (2H, t, *J* = 7Hz, OCH₂), 3.74 (3H, s, Me), 3.10 (2H, t, *J* = 7Hz, CH₂Ar); m/z (EI⁺) 368 (M-CO₂).

### EXAMPLE 32

### 3-(3-Methoxyphenyl)prop-2-ynyl 7-Chloro-3-(1,2,3,4-tetrahydroquinoline)carboxylate

White needles m.p. >310°C (from dimethylformamide/water); (Found: C, 61.99; H, 3.56; N, 3.93. C₂₀H₁₄ClNO₅ + 0.1 H₂O requires C, 62.30; H, 3.71; N, 3.63%); δ (d₆ DMSO) 13.0 (1H, br s), 11.64 (1H, s), 7.95 (1H, d, *J* = 8.6Hz, H-5), 7.33-7.29 (2H, m, H-8 and OCCHCH), 7.25 (1H, dd, *J* = 2.0 and 8.6Hz, H-6), 7.06 (1H, d, *J* = 7.6Hz, CHCHCH), 7.02 (1H, d, *J* = 2.2Hz, OCCHC), 7.01 (1H, dd, *J* 8 ad 2.2Hz, CHCHCH), 5.20 (2H, s, CH₂), 3.77 (3H, s, Me); m/z (CI⁺, NH₃) 239 (M⁺-C₁₀H₉O+H).

### EXAMPLE 33

### 3-(3-Indole)-1-propyl 7-Chloro-2,4-dioxo-3-(1,2,3,4-tetrahydroquinoline)carboxylate

Pale yellow solid m.p. 219-220°C (from dimethylformamide/acetone); (Found: C, 63.24; H, 4.07, N, 6.91. C₂₁H₁₇N₂O₄Cl requires C, 63.56; 4.32; N, 7.06%); δ (d₆-DMSO) 13.3 (1H, br s), 11.57 (1H, s), 10.76 (1H, s), 7.94 (1H, d, *J* = 8.6Hz, H-5), 7.56 (1H, d, *J* = 7.9Hz, indole H-4), 7.35-7.30 (2H, m, H-8 and indole H-7), 7.25 (1H, dd, *J* 1.8 and 8.6Hz, H-6), 7.16 (1H, s, indole H-2), 7.05 (1H, t, *J* = 7Hz, indole H-5 or H-6), 6.95 (1H, t, *J* = 7Hz, indole H-6 or H-5), 4.32 (2H, t, *J* = 7Hz, OCH₂), 2.86 (2H, t, *J* = 7Hz CH₂Ar), 2.06 (2H, quin, *J* = 7Hz, CH₂CH₂CH₂); m/z (CI⁺, NH₃) 239 (M⁺-C₁₁H₁₂N + H).

### EXAMPLE 34

### 2-[3,4-Bis(methoxymethoxy)phenyl]ethyl 7-Chloro-2,4-dioxo-3-(1,2,3,4-tetrahydroquinoline)carboxylate

White cubes, m.p. 202-204°C (from dimethylformamide/acetone/water); (Found: C, 57.04; H, 4.81; N, 3.08. C₂₂H₂₂NO₈Cl requires C, 56.96; H, 4.78; N, 3.02%); δ (d₆-DMSO) 13.3 (1H, br s), 11.58 (1H, s), 7.93 (1H, d, *J* = 8.6Hz, H-5), 7.30 (1H, d, *J* = 1.9Hz, H-8), 7.24 (1H, dd, *J* = 1.9 and 8.6Hz, H-6), 7.13 (1H, d, *J* = 1.9Hz, OCCHC), 7.00 (1H, d, *J* = 7.5Hz, OCCHCH), 6.93 (1H, dd, *J* = 1.9 and 7.5Hz, OCCHCH), 5.17 (2H, s, OCH₂O), 5.13 (2H, s, OCH₂O), 4.43 (2H, t, *J* = 7Hz, OCOCH₂), 3.38 (3H, s, Me), 3.37 (3H, s, Me), 2.93 (2H, t, *J* = 7Hz, CH₂Ar); m/z (CI⁺, NH₃) 464 (M⁺+H).

### EXAMPLE 35

### 4-(3-Hydroxyphenyl)-1-butyl 7-Chloro-3-)1,2,3,4-tetrahydroquinoline)carboxylate

White lozenges, mp. 187-188°C (from acetone); (Found: C, 62.15; H, 4.45; N, 3.59. C₂₀H₁₈ClNO₅ requires C, 61.94; H, 4.86; N, 3.61%); δ (d₆-DMSO) 13.3 (1H, br s), 11.54 (1H, s), 9.19 (1H, s), 7.93 (1H, d, *J* = 8.6Hz, H-5), 7.30 (1H, d, *J* = 1.8Hz, H-8), 7.24 (1H, dd, *J* = 1.8 and 8.6Hz, H-6), 7.05 (1H, t, *J* = 7.7Hz, HOCCHCH), 6.62 (1H, d, *J* = 7.7Hz, CHCHCH), 6.61 (1H, d, *J* = 2.5Hz, HOCCHC), 6.56 (1H, dd, *J* = 7.7 and 2.5Hz, CHCHCH), 4.30 (2H, br s, OCH₂), 2.55 (2H, br s, CH₂Ar), 1.69 (4H, br s, CH₂CH₂CH₂CH₂); m/z (EI⁺) 387 (M⁺).

### EXAMPLE 36

### 3-(3-Hydroxyphenyl)-1-propyl 7-Chloro-2,4-dioxo-3-(1,2,3,4-tetrahydroquinoline)carboxylate

White solid, m.p. 224-226°C (from dimethylformamide/acetone/water); (Found: C, 60.87; H, 4.25; N, 3.72. C₁₉H₁₆NO₅Cl requires C, 61.05; N, 4.31; 3.74%); δ (d₆-DMSO) 13.3 (1H, br s), 11.54 (1H, s), 9.2 (1H, br s), 7.93 (1H, d, *J* = 8.6Hz, H-5), 7.31 (1H, d, *J* = 1.9Hz, H-8), 7.24 (1H, dd, *J* = 1.9 and 8.6, H-6), 7.07 (1H, t, *J* = 8Hz, HOCCHCH), [6.85-6.8 (2H, m) and 6.57 (1H, dd, *J* = 1.7 and 8Hz), other ArH], 4.28 (2H, t, *J* = 7Hz, OCH₂), 2.66 (2H, t, *J* = 7Hz, CH₂Ar), 1.96 (2H, quin, *J* = 7Hz, CH₂CH₂CH₂); m/z (EI⁺) 373 (M⁺).

### EXAMPLE 37

### 2-(3-Indole)-ethyl 7-Chloro-2,4-dioxo-3-(1,2,3,4-tetrahydroquinoline)carboxylate

White amorphous solid m.p. 233-235°C (from dimethylformamide); (Found: C, 62.62; H, 3.70; N, 7.17. C₂₀H₁₅N₂O₄Cl requires C, 62.76; H, 3.95; N, 7.32%); δ (d₆-DMSO) 13.4 (1H, br s), 11.58 (1H, s), 10.87 (1H, s), 7.93 (1H, d, *J* = 8.6Hz, H-5), 7.60 (1H, d, *J* = 7.8Hz, indole H-4), 7.4-7.3 (3H, m, H-8, indole H-2 and H-7), 7.24 (1H, dd, *J* = 2.0 and 8.6Hz, H-6), 7.07 (1H, t, *J* = 7Hz, indole H-5 or H-6), 6.98 (1H, t, *J* = 7Hz, indole H-6 or H-5), 4.51 (2H, t, *J* = 7Hz, OCH₂), 3.14 (2, t, *J* 7Hz, CH₂Ar); m/z (CI⁺, NH₃) 239 (M⁺-C₁₀H₁₀N+H).

### EXAMPLE 38

### 3-(2-Hydroxyphenyl)-1-propyl 7-Chloro-2,4-dioxo-3-(1,2,3,4-tetrahydroquinoline)carboxylate

White cubes m.p. 225-227°C (from dimethylformamide/acetone/water); (Found: C, 60.96; H, 4.28; N, 3.75. C₁₉H₁₆NO₅Cl requires C, 61.05; H, 4.31; N, 3.75%); δ (d₆-DMSO) 13.3 (1H, br s), 11.55 (1H, s), 9.26 (1H, br s), 7.93 (1H, d, *J* = 8.7Hz, H-5), 7.30 (1H, d, *J* = 1.96 H-8), 7.24 (1H, dd, *J* = 1.9 and 8.7Hz, H-6), 7.09 (1H, dd, *J* = 1.6 and 7Hz, CH₂CCH), 7.00 (1H, dt, *J* = 1.6 and 7Hz, HOCCHCH), 6.78 (1H, dd, *J* = 1 and 7Hz, HOCCH), 6.70 (1H, dt, *J* = 1 and 7Hz, CH₂CCHCH), 4.26 (2H, t, *J* = 7Hz, OCH₂), 2.67 (2H, t, *J* = 7Hz, CH₂Ar), 1.96 (2H, quin, *J* = 7Hz, CH₂CH₂CH₂); m/z (CI⁺, NH₃) 374 (M⁺+H).

### EXAMPLE 39

### 2-[4-(Tert-butyloxycarbonylaminomethyl)phenyl]ethyl 7-Chloro-2,4-dioxo-3-(-1,2,3,4-tetrahydroquinoline)carboxylate

White needles m.p. 239-241°C (from dimethylformamide); (Found: C, 60.72; H, 5.33; N, 5.95. C₂₄H₂₅N₂O₆Cl requires C, 60.95; H, 5.33; N, 5.92%); δ (d₆-DMSO) 13.3 (1H, br s), 11.56 (1H, s), 7.92 (1H, d, *J* = 8.6Hz, H-5), 7.3-7.25 (4H, m, H-8, NH, ArH), 7.24 (1H, dd, *J* = 2 and 8.6Hz, H-6), 7.15 (2H, d, *J* = 8Hz, ArH), 4.44 (2H, t, *J* = 7Hz, OCH₂), 4.08 (2H, d, *J* = 5.7Hz, NCH₂), 2.98 (2H, t, *J* = 7Hz, CH₂Ar), 1.38 (9H, s, ^{t}Bu); m/z (CI⁺, NH₃) 473 (M⁺+H).

### EXAMPLE 40

### 1-(2-Propenyl) 7-Chloro-2,4-dioxo-3-(1,2,3,4-tetrahydroquinoline)carboxylate

White needles m.p. >320°C (from dimethylformamide); (Found: C, 55.79; H, 3.63; N, 5.00. C₁₃H₁₀ClNO₄ requires C, 55.83; H, 3.60; N, 5.01%); δ (d₆-DMSO) 13.3 (1H, br s), 11.58 (1H, s), 7.94 (1H, dd, *J* = 8.6Hz, H-5), 7.30 (1H, d, *J* 1.9Hz, H-8), 7.25 (1H, dd, *J* = 1.9 and 8.6Hz, H-6), 6.06-5.96 (1H, m, CHCH₂), 5.57 (1H, d with other fine coupling, *J* = 17Hz, C = CH_{A}H_{B}), 5.27 (1H, d with other fine coupling, *J* = 9Hz, C = CH_{A}H_{B}), 4.83-4.81 (2H, m, OCH₂); m/z (EI⁺) 279 (M⁺).

### EXAMPLE 41

### 2-(3-Thiophene)ethyl 7-Chloro-2,4-dioxo-3-(1,2,3,4-tetrahydroquinoline)carboxylate

White needles m.p. 293°C (sublimes) (from dimethylformamide/water); (Found: C, 54.79; H, 3.37; N, 4.08. C₁₆H₁₂ClNO₄S requires C, 54.94; H, 3.46; N, 4.00%); δ (d₆-DMSO) 13.3 (1H, br s), 11.59 (1H, s), 7.94 (1, d, *J* = 8.7Hz, H-5), 7.45 (1H, dd, *J* = 4.8 and 3.0Hz, SCHCH), 7.41 (1H, d, *J* = 1.7Hz, SCHC), 7.30 (1H, d, *J* = 1.9Hz, H-8), 7.25 (1H, dd, *J* = 8.7 and 1.9Hz, H-6), 7.19 (1H, dd, *J* = 4.8 and 1.1Hz, SCHCH), 4.46 (2H, t, *J* = 6.7Hz, OCH₂), 3.04 (2H, t, *J* = 6.7Hz, CH₂Ar); m/z (CI⁺, NH₃) 350 (M⁺+H).

### EXAMPLE 42

### 2-(2-Thiophene)ethyl 7-Chloro-2,4-dioxo-3-(1,2,3,4-tetrahydroquinoline)carboxylate

White needles m.p. 315°C (dec) (from dimethylformamide/water); (Found: C, 55.02; H, 3.20; N, 4.07. C₁₆H₁₂NO₄SCl requires C, 54.94; H, 3.46; N, 4.00%); δ (d₆-DMSO) 13.2 (1H, br s), 11.57 (1H, br s), 7.94 (1H, d, *J* = 8.6Hz, H-5), 7.35 (1H, dd, *J* = 5.1 and 1Hz, SCH), 7.30 (1H, d, *J* = 1.9Hz, H-8), 7.25 (1H, dd, *J* = 1.9 and 8.6Hz, H-6), 7.07 (1H, dd, *J* = 3.4 and 1Hz, SCHCHCH), 6.96 (1H, dd, *J* = 5.1 and 3.4Hz, SCHCH), 4.47 (2H, t, *J* = 6.8Hz, OCH₂), 3.23 (2H, t, *J* = 6.8Hz, CH₂Ar); m/z (CI⁺, NH₃) 350 (M⁺+H).

### EXAMPLE 43

### 2-(4-Hydroxyphenyl)-1-ethyl 7-Chloro-2,4-dioxo-3-(1,2,3,4-tetrahydroquinoline)carboxylate

White needles m.p. 258-260°C (from dimethylformamide/acetone); (Found: C, 59.72; H, 4.02; N, 4.02. C₁₈H₁₄NO₅Cl requires C, 60.09; H, 3.92; N, 3.89%); δ (d₆-DMSO) 13.3 (1H, br s), 11.59 (1H, s), 9.19 (1H, s), 7.93 (1H, d, *J* = 8.7Hz, H-5), 7.29 (1H, d, *J* = 1.9Hz, H-8), 7.24 (1H, dd, *J* = 1.9 ad 8.7Hz, H-6), 7.14 (2H, d, *J* = 8.4Hz, CH₂CCH), 6.67 (2H, d, *J* = 8.4Hz, HOCCH), 4.39 (2H, t, *J* = 7.1Hz, OCH₂), 2.89 (1H, t, *J* = 7.1Hz, CH₂Ar); m/z (FAB⁻) 358 (M⁺-H).

### EXAMPLE 44

### 2-(2-Hydroxyphenyl)-1-ethyl 7-Chloro-2,4-dioxo-3-(1,2,3,4-tetrahydroquinoline)carboxylate

White needles m.p. >320°C (from dimethylformamide/acetone/water); (Found: C, 59.91; H, 3.95; N, 3.91. C₁₈H₁₄NO₅Cl requires C, 60.09; H, 3.92; N, 3.89%); δ (d₆-DMSO) 13.3 (1H, br s), 11.58 (1H, s), 9.45 (1H, br s), 7.93 (1H, d, *J* = 8.6Hz, H-5), 7.29 (1H, d, *J* = 1.9Hz, H-8), 7.24 (1H, dd, *J* = 1.9 and 8.6Hz, H-6), 7.18 (1H, dd, *J* = 1.5 and 8Hz, CH₂CH), 7.04 (1H, dt, *J* = 1.5 and 8Hz, HOCCHCH), 6.80 (1H, d, *J* = 8Hz, HOCCH) 6.71 (1H, t, *J* = 8Hz, CH₂CCHCH), 4.43 (2H, t, *J* = 7.4Hz, OCH₂), 2.96 (2H, t, *J* = 7.4Hz, CH₂Ar); m/z (EI⁺) 359 (M⁺).

### EXAMPLE 45

### 3-(3-Hydroxyphenyl)prop-2-ynyl 7-Chloro-2,4-dioxo-3-(1,2,3,4-tetrahydroquinoline)carboxylate

Off-white crystalline solid, m.p. 234-236°C (from dimethylformamide/acetone/water); (Found: C, 61.34; H, 3.07; N, 3.61. C₁₉H₁₅ClNO₅. 0.1 H₂O requires C, 61.42; H, 3.31; N, 3.77%); δ (d₆-DMSO) 13.0 (1H, v. br s), 11.65 (1H, br s), 9.69 (1H, br s), 7.95 (1H, d, *J* = 8.6Hz, H-5), 7.3 (1H, d, *J* = 7.8Hz, H-8), 7.26 (1H, dd, *J* = 8.6 and 1.9Hz, H-6), 7.19 (1H, t, *J* =7.8Hz, CHCHCH), 6.90 (1H, d, *J* = 7. 8Hz, CHCHCH), 6.83 (1H, s, C(OH) CHC), 6.82 (1H, d, *J* = 7.8Hz, CHCHCH), 5.18 (2H, s, CH₂); m/z (CI⁺, NH₃) 239 (M-CH₂CCAr).

### EXAMPLE 46

### (E)-(3-Methoxyphenyl)prop-2-enyl 7-Chloro-2,4-dioxo-3-(1,2,3,4-tetrahydroquinoline)carboxylate

Fine white needles, m.p. = 181-183°C (from dimethyl formamide/acetone); (Found: C, 62.07; H, 4.07; N, 3.64. C₂₀H₁₆ClNO₅ requires C, 62.26; H, 4.18; N, 3.63%); δ (d₆-DMSO) 13.3 (1H, v, br s), 11.61 (1H, br s), 7.95 (1H, d, *J* = 8.6Hz, H-5), 7.30 (1H, d, *J* = 1.8Hz, H-8), 7.30-7.27 (1H, Ar-H), 7.25 (1H, dd, *J* = 8.6 and 1.8Hz, H-6), 7.06-7.03 (2H, m, 2 x Ar-H), 6.88-6.85 (1H, m, Ar-H), 6.82 (1H, d, *J* = 16.1Hz, Ar CH), 6.48 (1H, dt, *J* = 16.1 and 6.0Hz, CH₂CH), 4.98 (2H, d, *J* = 6.0Hz, CH₂), 3.77 (3H, s, OCH₃); m/z (EI⁺), 341 (M-CO₂).

### EXAMPLE 47

### 2-Phenylthioethyl 7-Chloro-2,4-dioxo-(1,2,3,4-tetrahydroquinoline)carboxylate

Fine white needles, m.p. 206-208°C (from dimethylformamide); (Found: C, 57.34; H, 3.51; N, 3.73. C₁₈H₁₄NSO₄Cl requires C, 57.53; H, 3.75; N, 3.73%); δ (d₆-DMSO) 13.0 (1H, br s), 11.57 (1H, s), 7.93 (1H, d, *J* = 8.6Hz, H-5), 7.43 (2H, dd, *J* = 7.5 and 1.4Hz, SCCH), 7.29 (2H, t, *J* = 7.5Hz, SCCHCH), 7.26 (1H, d, *J* = 1.6Hz, H-8), 7.21 (1H, dd, *J* = 8.6 and 1.6Hz, H-6), 7.19 (1H, dt, *J* = 1.4 and 7.5Hz, SCCHCHCH), 4.42 (2H, t, *J* = 7.2Hz, OCH₂), 3.3 (2H, t, *J* = 7.2Hz, SCH₂); m/z (CI⁻, NH₃), 374 (M⁺-H).

### EXAMPLE 48

### S-2-Phenylethyl 7-Chloro-2,4-dioxo-3-(1,2,3,4-tetrahydroquinoline)thiocarboxylate

Using phenyethyl mercaptan to give pale yellow needles, m.p. > 320°C (from dimethyl formamide/acetone/water), change of crystalline form at 237-241°C; (Found: C, 60.25; H, 3.73; N, 3.97. C₁₈H₁₄ClNO₃S requires C, 60.08; H, 3.92; N, 3.89%); δ (d₆-DMSO) 15.0 (1H, v br s), 11.82 (1H, br s), 8.00 (1H, d, *J* = 8.6Hz, H-5), 7.34-7.22 (7H, m, H-6, H-8, 5 x Ar), 3.19 (2H, t, *J* = 7.6Hz, SCH₂), 2.91 (2H, t, *J* = 7.6Hz, CH₂ Ph); m/z (EI⁺) 255 (M-C₆H₅CH₂CH₂+H).

### EXAMPLE 49

### Ethyl 7-Nitro-2,4-dioxo-3-(1,2,3,4-tetrahydroquinoline)carboxylate

Ethyl malonyl chloride (3.15g) in dry dichloromethane (10ml) was added dropwise to a stirred solution of ethyl-4-nitroanthranilate (2.01g), triethylamine (4.7ml) and 4-dimethylaminopyridine (0.080g) in dry dichloromethane (10ml) at 0°C under N₂. After complete addition the reaction was maintained at 0°C for 45 min before further triethylamine (4.7ml) was introduced, followed by dropwise addition of ethyl malonyl chloride (3.15g) in dry dichloromethane (10ml). The reaction was stirred at 0°C for 45 min. The suspension was concentrated in vacuo, diluted with ethyl acetate, washed twice with 10% citric acid followed by saturated sodium hydrogen carbonate solution, water and brine. The organics were dried (MgSO₄) and concentrated in vacuo to give a deep orange oil (2.51g). The oil was dissolved in ethanol (15ml) and a solution of sodium methoxide (0.84g) in ethanol (15ml) added with vigorous stirring. After 1 hour the mixture was diluted with ethyl acetate and extracted three times with 1N sodium hydroxide solution. The combined aqueous fractions were acidified to pH0 with 2N hydrochloric acid and the resulting precipitate filtered off, washed with water and dried in vacuo over phosphorus pentoxide to give 1.3g of a deep yellow solid. This solid was washed with boiling ethanol and dried to yield the ester (0.637g) as fine light yellow crystals, m.p. >325°C (Found: C, 51.56; H, 3.59; N, 9.98. C₁₂H₁₀N₂O₆ requires C, 51.81; H, 3.62; N, 10.07%); δ (D₆-DMSO) 11.89 (1H, br s), 8.16 (1H, d, *J* 8.8 Hz, H-5), 8.10 (1H, d, *J* = 2.2 Hz, H-8), 7.96 (1H, dd, *J* = 2.2 and 8.8 Hz, H-6), 4.33 (2H, q, *J* = 7.1 Hz, CH₂), 1.30 (3H, t, *J* = 7.1 Hz, CH₃), m/z (EI⁺) 278 (M⁺).

### EXAMPLE 50

### Ethyl 6,7-Dinitro-2,4-dioxo-3-(1,2,3,4-tetrahydroquinoline)carboxylate

A 1:1 mixture of concentrated sulphuric and concentrated nitric acids (0.5ml:0.5ml) was added dropwise to a cooled (ice bath) stirred suspension of ethyl 7-nitro-2,4-dioxo-3-(1,2,3,4-tetrahydroquinoline)carboxylate (0.139g) in concentrated sulphuric acid (2ml) under N₂. The reaction was warmed to 60°C for 45 min, then cooled to room temperature and diluted with cold distilled water. The resulting precipitate was filtered off, washed with water and dried in vacuo to give the ester (0.055g) as fine cream coloured crystals, m.p. 229-230°C (from ethanol); (Found: C, 44.44; H, 2.78; N, 12.86. C₁₂H₉N₃O₈ requires C, 44.59; H, 2.81; N, 13.00%); δ (d₆-DMSO) 12.37 (1H, br s), 8.68 (1H, s, H-5), 7.80 (1H, s, H-8), 4.31 (2H, q, *J* = 7.1 Hz, CH₂), 1.29 (3H, t, *J* = 7.1 Hz, CH₃); m/z (EI⁺) 323 (M⁺).

### EXAMPLE 51

### Ethyl 7-Chloro-2,4-dioxo-5-iodo-3-(1,2,3,4-tetrahydroquinoline carboxylate

A mixture of 3-chloro-5-iodoaniline (63.41g) in water (150ml), concentrated hydrochloric acid (22.1ml) and 1,4-dioxan (∼60ml) was heated to gain solution before being added to a mixture of chloral hydrate (90.24g) and sodium sulphate (650g) in water (600ml) which had been warmed to 50°C, Hydroxylamine hydrochloride (110.56g) in water (250ml) was then added and the reaction heated at reflux for 45 mins before being allowed to cool to room temperature and the resultant yellow precipitate of 3-chloro-5-iodophenylisonitrosoacetanilide filtered off, washed with water and dried in vacuo over silica gel.

A sample of the isonitrosoacetanilide (45g) was added portionwise to pre-warmed concentrated sulphuric acid (175ml, 50°C) keeping the internal temperature between 50°C and 70°C, using an ice bath. After complete addition, the now dark solution was heated at 80°C for 10 minutes before being allowed to cool to room temperature and poured on to ten times the reaction volume of ice. The resultant slurry was swirled vigorously and left to stand for one hour before filtering off the resultant rust coloured precipitate, washing with water and drying in vacuo over phosphorus pentoxide. This yielded a mixture of 6-chloro-4-iodo and 4-chloro-6-iodo isatins. δ (d₆-DMSO), 11.26 (1H, s, N'H), 11.18 (1H, s, NH), 7.55 (1H, d, *J* = 1.6Hz, H-5 or H-7), 7.50 (1H, d, *J* = 1.0Hz, H-5' or H-7'), 7.25 (1H, d, *J* = 1.0Hz, H-5' or H-7'), 6.98 (1H, d, *J* = 1.6Hz, H-5 or H-7).

30% hydrogen peroxide (35.7ml) was added portionwise to a solution of the mixture above isatins (53.68g) at room temperature in 1N sodium hydroxide solution (525ml). Once effervescence had stopped, the reaction was cautiously neutralised with 2N hydrochloric acid and filtered to remove insolubles before acidifying to pH 2-3. The resultant sandy yellow precipitate was filtered off and washed with water before drying in vacuo over phosphorus pentoxide to yield a mixture of the 2-amino-4-chloro-6-iodo and 2-amino6-chloro-4-iodo benzoic acids (10.56g). Dissolving the mixture of isomers (8g) in boiling acetone and reducing the volume until a solid started to crystallise out, resulted in the formation of the Schiff's base (enriched (10:1) in the more prevalent 4-chloro-6-iodo isomer). Hydrolysis of this imine with 2N hydrochloric acid yielded the amino benzoic acid. Repetition of this process gave the 2-amino-4-chloro-6-iodo benzoic acid (3.75g) in > 95% purity. δ (d₆-DMSO) 7.05 (1H, d, *J* = 1.9Hz, H-3 or H-5), 6.79 (1H, d, *J* = 1.9Hz, H-3 or H-5).

Treatment of an ethereal solution of the acid (2.68g) with diazomethane and concentration in vacuo, yielded the desired methyl 2-amino-4-chloro-6-iodobenzoate (2.81g) as a yellow oil which crystallised on standing. δ (d₆-DMSO) 7.04 (1H, d, *J* = 1.9Hz, H-3 or H-5), 6.78 (1H, d, *J* = 1.9Hz, H-3 or H-5), 5.89 (2H, s, NH₂), 3.61 (3H, s, CH₃).

Addition under nitrogen at room temperature of a solution of ethyl malonyl chloride (2.03g) in dry dichloromethane (15ml) to a solution of the benzoate (2.81g) in dry dichloromethane (15ml), resulted in the rapid formation of a gelatinous white precipitate. The solvent was removed in vacuo and the precipitate triturated with diethyl ether to leave the methyl 2-malonamido-4-chloro-6-iodo benzoate as an off white solid (2.33g).

Cyclisation of the methyl 2-malonamido-4-chloro-6-iodo benzoate was achieved by addition of a solution of sodium methoxide (0.25g) in ethanol (10ml) under nitrogen, to a vigorously stirred solution of the benzoate (1.83g) in ethanol (15ml) at room temperature. After 1¹/₄ hours the reaction was diluted with ethyl acetate and extracted three times with 1N sodium hydroxide. The combined aqueous fractions were acidified to pH 1 with 2N hydrochloric acid and the resultant white precipitate filtered off and washed with water, before being crystallised from hot DMF (keeping the solvent temperature ≦ 80°C). This gave the title compound as very fine needle crystals (0.571g), melting point 212-216°C; (Found: C, 36.48; H, 2.09; N, 3.49. C₁₂H₉ClINO₄ requires C, 36.62; H, 2.31; N, 3.56%); δ (d₆-DMSO) 11.65 (1H, s), 7.86 (1H, d, *J* = 2.0Hz, H-6 or H-8), 7.33 (1H, d, *J* = 2.0Hz, H-6 or H-8), 4.35 (2H, q, *J* = 7.1Hz, CH₂CH₃), 1.31 (3H, t, *J* = 7.1Hz, CH₂CH₃); m/z (EI⁺) 393 (M⁺).

### EXAMPLE 52

### 2-(3-Indole)-1-ethyl 7-Chloro-2,4-dioxo-5-iodo-3-(1,2,3,4-tetrahydroquinoline) carboxylate

Tryptophol (0.68g) and ethyl 7-chloro-2,4-dioxo-5-iodo-3-(1,2,3,4-tetrahydroquinoline) carboxylate (0.160g) were mixed together as a slurry in diethyl ether and the solvent removed in vacuo to leave the reactants as a finely blended residue. This was then heated under nitrogen at 150°C for 40 mins before introducing a further 0.65g of tryptophol and heating at 150°C for 25 mins. The reaction was allowed to cool to room temperature before washing with diethyl ether. The resultant white solid was crystallised from DMF to give the title compound as fine white crystals (0.109g), melting point 240-244°C-decomposes; (Found: C, 47.05; H, 2.63; N, 5.40. C₂₀H₁₄ClIN₂O₄ requires C, 47.22; H, 2.77; N, 5.51%); δ (d₆-DMSO) 11.67 (1H, s), 10.87 (1H, s), 7.87 (1H, d, *J* = 2.1Hz, H-6 or H-8), 7.60 (1H, d, *J* = 7.1Hz, indole H-4 or indole H-7), 7.33 (3H, m, H-6 or H-8, and indole H-2, and indole H-4 or indole H-7), 7.06 (1H, t, *J* = 7.1Hz, indole H-5 or indole H-6), 6.98 (1H, t, *J* = 7.0Hz, indole H-5 or indole H-6), 4.52 (2H, t, *J* = 7.2Hz, CH₂OCOR), 3.14 (2H, t, *J* = 7.2Hz, CH₂-indole); m/z (CI⁺) 365 ((M-C₁₀H₁₀N+H)⁺).

### Methyl 4-Chloro-6-vinylanthranilate

Vinyltributyltin (1.6ml), lithium chloride (dried at 100°C under vacuum for 18h) (780g) and bis triphenylphosphine palladium (II) dichloride (265mg) were added to a stirred solution of methyl 4-chloro-6-iodoanthranilate (1.63g) in dry dimethyl formamide (20ml) at room temperature. The mixture was heated under nitrogen at 60°C for 40 mins. After cooling, the solution was diluted with ethyl acetate (125ml), washed with water (3 x 100ml) and brine, dried (Na₂SO₄) and evaporated in vacuo to give an oil. This was purified by flash chromatography eluting with ethylacetate/hexane to give the anthranilate as an off-white crystalline solid (1.02g); δ (d₆-DMSO) 6.83 (1H, dd, *J* = 17.3Hz and 11.0Hz, ArCH), 6.75-6.73 (2H, m, H-3 and H-5), 6.01 (2H, br s, NH₂), 5.65 (1H, dd, *J* = 17.3Hz and 1.1Hz, CH_{A}H_{B}, H_{A} cis to Ar), 5.26 (1H, dd, *J* = 11.0Hz and 1.1Hz, CH_{A}H_{B}, H_{B} trans to Ar), 3.80 (3H, s, CH₃).

### Methyl 4-Chloro-6-ethylanthranilate

5% platinum on sulphide carbon (300mg) was added to a solution of methyl 4-chloro-6-vinylanthranilate (1.01g) in ethyl acetate (80ml). The suspension was hydrogenated at 50 p.s.i. for 2h. The catalyst was removed by filtration and the solution evaporated in vacuo to give the anthranilate as a clear colourless oil (1.02g); δ (d₆-DMSO) 6.65 (1H, d, *J* = 2.5Hz, H-3 or H-5), 6.46 (1H, d, *J* = 2.5Hz, H-3 or H-5), 5.85 (2H, br s, NH₂), 3.81 (3H, s, OCH₃), 2.60 (2H, q, *J* = 7.5Hz, CH₂), 1.08 (3H, t, *J* = 7.5Hz, CH₂CH₃).

### EXAMPLE 53

### Methyl 7-Chloro-5-ethyl-2,4-dioxo-3-(1,2,3,4-tetrahydroquinoline)carboxylate

Methyl malonyl chloride (140µl) was added to a solution of methyl 4-chloro-6-ethaylanthranilate (250mg) in dry dichloromethane (10ml). The mixture was heated at reflux under nitrogen to redissolve the precipitate. After 2h, the solution was cooled and evaporated in vacuo. The residue was diluted with ethyl acetate, washed with 0.5N citric acid, saturated sodium bicarbonate solution and brine, dried (Na₂SO₄) and reduced in vacuo to give a clear colourless oil (340mg). 300mg of this oil was dissolved in dry methanol (10ml) then sodium methoxide (90mg) was added. After 1h the suspension was evaporated in vacuo and the residue partitioned between 1N sodium hydroxide solution and ether. The aqueous layer was acidified with concentrated hydrochloric acid and the resulting precipitate extracted into ethyl acetate (2 x 75ml). The combined organic extracts were washed with water and brine, dried (Na₂SO₄) and evaporated to give an off-white solid. This solid was recrystallised from dimethyl formamide and water keeping the temperature below 90°C, then washed with water, acetone and ether and dried to give the ester as fine white rhombic crystals. m.p. > 320°C change of crystalline form at 197-198°C; (Found: C, 55.22; H, 4.22; N, 4.78. C₁₃H₁₂ClNO₄ requires C, 55.43; H, 4.29; N, 4.97%). δ (d₆-DMSO) 14.5 (1H, v br s), 11.54 (1H, br s), 7.17 (1H, d, *J* = 2.0Hz, H-6 or H-8), 7.05 (1H, d, *J* = 2.0Hz, H-6 or H-8), 3.88 (3H, s, OCH₃, 3.12 (2H, q, *J* = 7.3Hz, CH₂), 1.20 (3H, t, *J* = 7.3Hz, CH₂CH₃).

### EXAMPLE 54

### 7-Chloro-3-(3-methyl-1,2,4-oxadiaxol-5-yl)-2,4-dioxo-1,2,3,4-tetrahydroquinoline

Ethyl-7-chloro-2,4-dioxo-3-(1,2,3,4-tetrahydroquinoline)carboxylate (0.40g) and acetamide oxime (1.1g) were heated at 130°C in dimethylformamide (10ml) for 4h. The mixture was cooled, water (25ml) and 1M hydrochloric acid (2ml) were added, and the solid collected, washed with water and ethanol, dried and recrystallised from dimethylsulphoxide to give the tetrahydroquinoline (107mg) as a tan solid, m.p. >320°C, (Found: C, 51.09; H, 3.06; N, 14.61. C₁₂H₈N₃O₃Cl + 0.3 H₂O requires C, 50.92; H, 3.06; N, 14.84%); δ (d₆-DMSO) 11.68 (1H, s), 7.99 (1H, d, *J* = 8.6Hz, H-5), 7.32 (1H, d, *J* = 1.8H-8), 7.28 (1H, dd, *J* = 1.8 ad 8.6Hz, H-6), 2.46 (3H, s, Me); m/z (EI⁺) 277 (M⁺).

### EXAMPLE 55

### (E) Ethyl 3-(7-Chloro-2,4-dioxo-(1,2,3,4-tetrahydroquinolin-3-yl)-2-propenoate

Sodium hydroxide (13ml of a 4N aqueous solution) was added to diethylglutaconate (10g) in ethanol (100ml). After 14h the mixture was diluted with water, washed with ether, acidified and extracted with ether (3 x 50ml). The combined organic layers were washed with water and brine, dried, and evaporated to give an oil (5.1g). Oxalyl chloride (1.34ml) was added to a solution of 1.21g of this oil in dry dichloromethane (18ml) and dimethylformamide (1 drop). After 2h the mixture was evaporated, dissolved in dichloromethane (7ml) and added to a solution of ethyl 4-chloroanthranilate (0.4g), triethylamine (1.4ml) and 4-dimethylaminopyridine (100mg) in dichloromethane (20ml) at 0°C. The mixture was stirred at 0°C for 1h, room temperature for 1h, diluted with ethyl acetate, washed with 1M citric acid, sodium hydroxide solution, and brine, dried, evaporated and purified by flash chromatography, eluting with hexanes: ethyl acetate, (4:1 v/v) to give the acylated anthranilate as a mixture of isomers (247mg). This was dissolved in ethanol (10ml) and sodium methoxide (0.05g) added. After 1h the mixture was poured into 0.2M HCl (25ml) the product collected, washed with water, dried, suspended in boiling ethanol (100ml), cooled, the solid collected, washed with ethanol, dried to give the ester (64mg) as white needles, m.p. 246-249°C; (Found: C, 57.11; H, 4.20; N, 4.71. C₁₄H₁₂NO₄Cl requires C, 57.25; H, 4.12; N, 4.77%); δ (d₆-DMSO) 11.62 (1H, s), 8.04 (1H, d, *J* = 8.7Hz, H-5), 7.98 (1H, d, *J* = 15.8Hz, CHCHCO), 7.51 (1H, d, *J* = 2.0Hz, H-8), 7.26 (1H, d, *J* = 15.8Hz, CHCHCO), 7.24 (1H, dd, *J* = 2.0 and 8.7Hz, H-6), 4.16 (2H, q, *J* = 7.1Hz, OCH₂), 1.25 (3H, t, *J* = 7.1Hz, CH₃); m/z (EI⁺) 293 (M⁺).

### EXAMPLE 56

### 7-Chloro-2,4-dioxo-3-(1,2,3,4-tetrahydroquinoline)carboxylic acid

Sodium hydride (0.8g of an 80% dispersion in oil) was added to a mixture of ethyl 7-chloro-2,4-dioxo-3-(1,2,3,4-tetrahydroquinoline)carboxylate (0.4g) and 4-methoxyphenol (.20g) in dry dimethylformamide (5ml) under nitrogen. After heating at 80°C for 45 min the mixture was cooled, then water and dilute hydrochloric acid added. The resulting acid (0.21g) was collected as a light tan solid, m.p. > 310°C (from acetone); (Found: C, 49.98; H, 2.41; H, 5.72. C₁₀H₆NO₄Cl requires C, 50.13; H, 2.52; N, 5.85); ν (KBr disc) 3300-2500 cm⁻¹; δ (d₆-DMSO) 8.05 (1H, d, *J* = 8.6Hz, H-5), 7.48 (1H, d, *J* = 1.7Hz, H-8), 7.45 (1H, dd, *J* = 1.7 and 8.6Hz, H-6); m/z (EI⁺) 239 (M⁺). This compound is unstable with respect to decarboxylation on heating.

The following compounds were made in the same general way (Method E): The desired ester and the corresponding amine were refluxed in dry pyridine for 2h. On cooling and addition of dilute hydrochloric acid the product precipitated and was collected, washed with hydrochloride acid and ester, then dried.

### EXAMPLE 57

### 7-Chloro-2,4-dioxo-3-(1,2,3,4-tetrahydroquinoline)hydroxamic acid

From methyl 7-chloro-2,4-dioxo-3-(1,2,3,4 tetrahydroquinoline)carboxylate (0.44g) and hydroxylamine hydrochloride (2g) as fine white needles (0.14g), m.p. 280°C (dec); (Found: C, 47.32; H, 2.59; N, 10.91. C₁₀H₇N₂O₄Cl requires C, 47.17; H, 2.77; N, 11.00%); δ (d₆-DMSO) 18 (1H, br s), 11.9 (1H, br s), 11.8 (1H, br s), 9.8 (1H, br s), 7.96 (1H, d, *J* = 8.6Hz, H-5), 7.38 (1H, d, *J* = 1.8Hz, H-8), 7.32 (1H, dd, *J* = 1.8 and 8.6Hz, H-6); m/z (CI⁺, NH₃) 255 (M⁺+H).

### EXAMPLE 58

### 7-Chloro-5-iodo-2,4-dioxo-3-(1,2,3,4-tetrahydroquinoline)hydroxamic acid:-

Ethyl 7-chloro-5-iodo-2,4-dioxo-3-(1,2,3,4-tetrahydroquinoline)carboxylate (0.2g) and hydroxylamine hydrochloride (1.1g) gave the acid (0.14g) as white needles, m.p. 295°C (dec); (Found C, 31.48; H, 1.45; N, 7.15. C₁₀H₆N₂O₄ClI requires C, 31.56; H, 1.59; N, 7.36); δ (d₆-DMSO) 18 (1H, br s), 12.0 (2H, br s), 9.9 (1H, br s), 7.90 (1H, d, *J* = 2Hz, H-8), 7.40 (1H, d, *J* = 2Hz, H-6); m/z (EI⁺) 380 (M⁺).

### EXAMPLE 59

### 7-Chloro-2,4-dioxo-3-(1,2,3,4-tetrahydroquinoline)carboxylic acid hydrazide

From methyl 7-chloro-2,4-dioxo-3-(1,2,3,4-tetrahydroquinoline (0.4g) and hydrazine hydrate (2g) as fine white needles (0.23g), m.p. 288°C (dec); (Found: C, 46.96; H, 3.11; N, 16.40. C₁₀H₈N₃O₃Cl + 0.05 H₂O requires C, 47.19; H, 3.21; N, 16.51%); δ (d₆-DMSO) 12 (4H, br s), 11 (1H, br s), 7.96 (1H, d, *J* = 8.6Hz, H-5), 7.40 (1H, d, *J* = 1.8Hz, H-8), 7.33 (1H, dd, *J* = 8.6 and 1.8Hz, H-6); m/z (EI⁺) 253 (M⁺).

### EXAMPLE 60

### 7-Chloro-2,4-dioxo-3-(3-thienyl)-1,2,3,4-tetrahydroquinoline

To a solution of thiophene-3-acetic acid (1.4g, 10mmol) in dichloromethane (40ml) was added oxalyl chloride (1.74ml, 20mmol) and dimethylformamide (catalytic, 3 drops) and the reaction stirred for 1h. The solvent was evaporated and the residue co-evaporated with toluene (3 x 20ml). The resulting acid chloride was dissolved in 1,2-dichloroethane (40ml), methyl 2-amino-4-chlorobenzoate (1.5g, 8mmol) added, and the reaction refluxed for 2h. The residue remaining on evaporation of the solvent was triturated with diethyl ether to afford methyl 4- chloro-2-(3-thienyl)acetamidobenzoate as a tan solid (1g), m.p. 96-97°C. To a solution of the foregoing amide (0.5g, 1.6mmol) in tetrahydrofuran (25ml) was added a solution of potassium hexamethyldisilazide in toluene (0.5M, 8ml, 4mmol) and the reaction stirred for 2h. Methanol (5ml) was added and the solvent evaporated. The residue was partitioned between aqueous sodium hydroxide (1M, 20ml) and diethyl ether (20ml), and the aqueous layer acidified with hydrochloric acid (5M). The resultant precipitate was collected and recrystallised from dimethylformamide/water to afford the title compound as a white solid; m.p. 341-344°C (from DMF/H₂O) (Found: C, 55.07; H, 2.86; Cl, 12.94; N, 5.16; S, 11.42; C₁₃H₈ClNO₂S, 0.5 H₂O requires C, 55.32; H, 3.03, Cl, 12.56; N, 4.96; S, 11.36%); δ (DMSO-d₆) 7.22 (1H, dd, *J* = 8.6 and 2Hz, 6-H), 7.31 (1H, s, 8-H), 7.43 (1H, d, *J* = 4.9Hz, 5'-H), 7.49-7.51 (1H, m, 4'-H), 7.76 (1H, s, 2'-H), 7.98 (1H, d, *J* = 8.6Hz, 5-H), 11.55 (1H, s, NH); m/z 277 (M⁺).
The following compounds were made by the same method (Method F), typified by Example 60 above.

### EXAMPLE 61

### 7-Chloro-2,4-dioxo-3-(2-thienyl)-1,2,3,4-tetrahydroquinoline

m.p. 305-307°C (from DMF/H₂O) (Found: C, 55.32; H, 2.96; N, 5.09; S, 11.17; C₁₃H₈ClNO₂S, 0.25 H₂O requires C, 55.32; H, 3.04; N, 4.96;, S, 11.36%); δ (DMSO-d₆) 7.11 (1H, dd, *J* = 5.1 and 3.8Hz, Th-H), 7.26 (1H, dd, *J* = 8.8 and 2.1Hz, 6-H), 7.35 (1H, d, *J* = 2Hz, 8-H), 7.50 (1H, dd, *J* = 5.1 and 1.1Hz, Th-H), 8.07 (2H, m, 5-H, Th-H), 11.73 (1H, s, NH); m/z 277 (M⁺).

### EXAMPLE 62

### 7-Chloro-2,4-dioxo-3-(2-pyridyl)-1,2,3,4-tetrahydroquinoline

To a solution of methyl 2-amino-4-chlorobenzoate (2g, 10.8mmol) in 1,2-dichloroethane (60ml) was added 2-pyridylacetic acid hydrochloride (1.9g, 10.8mmol), triethylamine (3.2ml, 22.7mmol) and bis (2-oxo-3-oxaxolidinyl)phosphinic chloride and the reaction refluxed for 8h. More 2-pyridylacetic acid (1.9g, 10.8mmol) and triethylamine (3.2ml, 22.7mmol) were added and refluxing continued for a further 16h. The solvent was evaporated and the residue partitioned between saturated aqueous sodium bicarbonate solution (50ml) and dichloromethane (50ml). The organic phase was dried (MgSO₄) and evaporated. The residue was purified by flash chromatography on silica (eluting with 50% ethyl acetate/60-90° petrol) to afford methyl 4-chloro-2-(3-pyridyl)acetamidobenzoate as a green solid (2.5g), m.p. 85-87°C. To a solution of the foregoing amide (1g, 3.3mmol) in tetrahydrofuran (40mml) was added a solution of potassium hexamethyldisilazide in toluene (0.5M, 16.6ml, 8.3mmol) and the reaction stirred for 2h. Methanol (15ml) was added and the solvent evaporated. The residue was partitioned between aqueous sodium hydroxide (1M, 40ml) and diethyl ether (40ml), and the aqueous layer acidified with hydrochloric acid (5M). The resultant precipitate was collected and recrystallised from dimethylformamide/water to afford the title compound as a yellow - green solid; m.p. > 330°C (from DMF/H₂O) (Found: C, 61.48; H, 3.20; N, 10.20; C₁₄H₉ClN₂O₂ requires C, 61.66; H, 3.33; N, 10.27%); δ_{H} (DMSO-d₆) 7.15 (1H, dd, *J* = 8.5Hz and 1.9Hz, 5-H), 7.26 (1H, s, 8-H), 7.42 (1H, t, *J* = 6.1Hz, 5'-H), 8.02 (1H, d, *J* = 8.5Hz, 5-H), 8.16 (1H, t, *J* = 7.3Hz, 4'-H), 8.78 (1H, d, *J* = 8.6Hz, 6'-H), 9.38 (1H, d, *J* = 8.8Hz, 3'-H), 11.20 (1H, s, NH); m/z 272 (M⁺).
The following compounds were made by the same general method (Method G), typified by Example 62 above.

### EXAMPLE 63

### 7-Chloro-2,4-dioxo-3-(3-pyridyl)-1,2,3,4

tetrahydroquinoline

m.p. > 340°C (from DMF/H₂O) (Found: C, 61.23; H, 3.33; N, 10.23; C₁₄H₉ClN₂O₂, 0.05 H₂O requires C, 61.46; H, 3.35, N, 10.24%); δ_{H} (DMSO-d₆) 7.21 (1H, dd, *J* = 8.5 and 1.9Hz, 6-H), 1H, s, 8-H), 7.42-7.46 (1H, m, 5'-H), 7.90-8.48 (1H, m, 5-H, 6'-H), 8.48 (1H, s, 4'-H), 8.86 (1H, s, 2'-H), 11.52 (1H, s, NH); m/z 272 (M⁺).

### EXAMPLE 64

### 7-Chloro-2,4-dioxo-3-(4-pyridyl)-1,2,3,4-tetrahydroquinoline

m.p. > 330°C (from DMF/H₂O); δ_{H} (DMSO-d₆) 7.08 (1H, dd, *J* = 8.5 and 1.9Hz, 6-H), 7.20 (1H, d, *J*, 8-H), 7.99 (1H, d, *J* = 8.5Hz, 5-H), 8.44 (2H, d, *J* = 6.9Hz, 2'-H, 6'-H), 8.95 (2H, d, *J* = 6.9Hz, 3'-H, 5'-H), 10.76 (1H, s, NH); m/z 272 (M⁺) (Found; m/z 272.0319; C₁₄H₉ClN₂O₂ requires 272.0352).

### EXAMPLE 65

### 7-Chloro-2,4-dioxo-3-(2-benzofuranyl)-1,2,3,4-tetrahydroquinoline

This compound was prepared in the same way as described in Example 62, except using benzofuranyl-2-acetic acid (Bisagni *et al*, Bull. Soc. Chim. Fr., 1962, 86-90), to give the title compound as a white crystalline solid, mpt 342°C; (Found: C, 65.67; H, 2.96; N, 4.39; C₁₇H₁₀ClNO₃ requires C, 65.50; H, 3.23; N, 4.49%); δ (d₆ DMSO) 11.75 (1H, s, NH), 11.00 (1H, bs, OH), 7.99 (1H, d, *J* = 8.7Hz, H-5), 7.67 (1H, d, *J* = 8.0Hz, benzofuran H-6), 7.61 (1H, d, *J* = 8.0Hz, benzofuran H-5), 7.29 (5H, m, H-6, H-8, benzofuran H-3, H-4, H-7); *m / z* (EI⁺) 311 (M⁺).

### EXAMPLE 66

### 7-Chloro-2,4-dioxo-3-(3-benzofuranyl)-1,2,3,4-tetrahydroquinoline

This compound was prepared in the same way as described in Example 62, except using benzofuranyl-3-acetic acid (Chatterjea *et al*, J. Indian Chem. Soc., 1980, 633-636), to give the title compound as a white crystalline solid, mpt 290°C slow decomp; (Found: C, 64.55; H, 3.30; N, 4.49. C₁₇H₁₀ClNO₃.0.5H₂O requires C, 64.55; H, 3.35; N, 4.43%); δ (d₆ DMSO) 11.6 (1H, s, NH), 10.58 (1H, bs, OH), 8.1 (1H, s, benzofuran H-2), 7.95 (1H, d, *J* = 8.7Hz, H-5), 7.62 (1H, d, *J* = 7.4Hz, benzofuran H-4), 7.34 (2H, m, H-8, benzofuran H-6), 7.23 (2H, m, H-6, benzofuran H-5); *m / z* (EI⁺) 311 (M⁺).

### EXAMPLE 67

### 7-Chloro-2,4-dioxo-3-([3-methyl]-2-furanyl)-1,2,3,4-tetrahydroquinoline

To a solution of methyl, 3-methyl furanoate (10g, 67.6mmol) in dry tetrahydrofuran (200ml) under a nitrogen atmosphere at 0°C was added lithium aluminium hydride (101ml of a 1M solution in THF, 101mmol) dropwise over 30 mins. The mixture was heated at reflux for 1 hour, and cooled to room temperature. To this solution was added methanol (70ml) dropwise, followed by water (3.83ml), 15% NaOH (3.83ml), and water (3.83ml). The mixture was filtered through celite and the solvent was removed under reduced pressure to afford the product (5.43g) as a pale yellow oil. To a solution of this compound (4.5g, 40mmol) in dry dichloromethane (200ml) under a nitrogen atmosphere was added manganese dioxide (13.98g, 160mmol). The mixture was heated to reflux for 1 hour and further manganese dioxide (13.,98g, 160mmol) added, and the mixture heated for a further 2 hours. The mixture was cooled to room temperature and filtered through celite. The mixture was washed with saturated sodium hydrogen carbonate (150ml), saturated sodium chloride (150ml), dried over MgSO₄, filtered and the solvent was removed under reduced pressure to give the product (3.35g) as a pale yellow oil. To a solution of sodium carbonate (12.9g, 122mmol), and potassium cyanide (3.97g, 61mmol), in water (150ml) was added in rapid succession glyoxal bisulphite addition compound (12.12g, 42.6mmol), the above product (3.45g, 30.5mmol), 1,4-dioxane (7ml), and water (50ml). The mixture was stirred at room temperature for 2 hours, then acidified to pH = 2 with c.H₂SO₄. The mixture was stirred for a further 1 hour then extracted into chloroform (3 x 100ml) and the combined organics were extracted with 1N NaOH (200ml). The aqueous NaOH was washed with chloroform (150ml), then acidified with c.HCl, and extracted with chloroform (3 x 100ml), dried over MgSO₄, filtered and the solvent was removed under reduced pressure to give 3-methyl-2-furanacetic acid (2.45g) as a brown solid.

The title compound was prepared in the same way as described in Example 62 except using 3-methyl-2-furanacetic acid to give the final product as a white crystalline solid, mpt 265°C slow decomp; (Found: C, 60.50; H, 3.46; N, 4.93. C₁₄H₁₀ClNO₃.0.2H₂O requires C, 60.60; H, 3.70; N, 5.04%); δ (d₆ DMSO) 11.50 (1H, s, NH), 10.67 (1H, bs, OH), 7.80 (1H, d, *J* = 8.6Hz, H-5), 7.61 (1H, d, *J* 1.8Hz, furan H-5), 7.31 (1H, d, *J* 2.0Hz, H-8), 7.21 (1H, dd, *J* = 8.6 and 2.0Hz, H-6), 6.40 (1H, d, *J* = 1.8Hz, furan H-4), 1.87 (3H, s, -CH₃); *m / z* (EI⁺ 275 (M⁺).

### EXAMPLE 68

### 7-Chloro-2,4-dioxo-3-(3-(2-methyl)furanyl)-1,2,3,4-tetrahydroquinoline

This compound was prepared in the same way as described in Example 62 except using 2-methylfuranylacetic acid (Heterocycles 23 3 549 1985) to give the title compound as a white crystalline solid; mp 259°C decomp (from DMF/H₂O); δH (DMSO-d₆) 2.12 (3H, s, CH₃), 6.41 (1H, d, *J* = 1.8Hz, 4-furanyl-H), 7.16 (1H, dd, *J* 8.5Hz and 1.9Hz, 6-H), 7.30 (1H, d, *J* = 1.9Hz, 8-H), 7.53 (1H, d, *J* = 1.8Hz, 5-furanyl-H), 7.89 (1H, d, *J* = 8.5Hz, 5-H), 10.15 (1H, bs, 3-H), 11.47 (1H, s, NH).

### EXAMPLE 69

### 7-Chloro-2,4-dioxo-3(2-(4-isopropyl)furanyl)-1,2,3,4-tetrahydroquinoline

4-Isopropyl-2-furaldehyde (Gilman *et al*, JACS, 1935, 57 906) was converted to 4-isopropyl furan-2-acetic acid under the conditions of Breen *et al*, (Aust. J. Chem., 1973, 26, 2221). Reaction of 4-isopropyl furan-2-acetic acid with methyl 2-amino-4-chlorobenzoate under the conditions described in Example 62 gave the title compound as colourless needles; mp 285°C dec; (Found: C, 62.71; H, 4.51; N, 4.64. C₁₆H₁₄ClNO₃.0.1H₂O requires C, 62.86; H, 4.68; N, 4.58%); δ (d₆-DMSO, 360MHz) 1.20 (6H, d, *J* = 6.9Hz), 2.81 (1H, m), 6.97 (1H, s), 7.23 (1H, dd, *J* = 8.7 and 2.0Hz), 7.31 (1H, d, *J* = 2.0Hz), 7.47 (1H, s), 7.93 (1H, d, *J* = 8.7Hz), 11.65 (1H, br s); *m / z* (EI) 303 (M⁺).

### EXAMPLE 70

### 7-Chloro-2,4-dioxo-3(2-(4-methyl)furanyl)-1,2,3,4-tetrahydroquinoline

3-Carboxy-4-methyl-furan-2-acetic acid (13g) was dissolved in 1,2-dichloromethane (150ml) with acetyl chloride (20ml) and heated under reflux for 14h. After cooling and evaporation, the residue was recrystallised from diethyl ether and collected by filtration to give a solid (9g), δ (CDCl₃, 360MHz) 2.24 (3H, s), 4.07 (2H, s), 7.28 (1H, s). This was dissolved in ethanol (100ml) and heated under reflux for 3h then concentrated in vacuo to give a white solid (9.4g); δ CDCl₃ 1.17 (3H, t, *J* = 7.1Hz), 2.07 (3H, s), 4.0 (2H, s), 4.08 (2H, q, *J* = 7.1Hz), 7.45 (1H, s), 12.64 (1H, br s). A 4g portion of this monoester was dissolved in quinoline (30ml) with cuprous oxide (0.5g) and heated at 200°C for 30 minutes. After cooling, the reaction mixture was filtered and diluted with dichloromethane (100ml), then washed with dilute hydrochloric acid (2 x 50ml). The organic layer was dried (Na₂SO₄), filtered and concentrated under vacuum to give a yellow oil (2.3g); δ 1.25 (3H, t, *J* = 7.1Hz), 1.99 (3H, s), 3.61 (2H, s), 4.16 (2H, q, *J* = 7.1Hz), 6.08 (1H, s), 7.11 (1H, s). A 2.1g portion of the yellow oil was dissolved in 50% aqueous methanol (50ml) with sodium hydroxide (1g) and stirred at room temperature for 2h. After removal of the solvents in vacuo the residue was dissolved in water (50ml) and washed with diethyl ether (2 x 30ml). The aqueous layer was acidified to pH 2 with dilute HCl and extracted into diethyl ether (2 x 50ml), dried (Na₂SO₄), filtered and concentrated in vacuo. The residue was recrystallised from 60-80 petrol to give 4-methyl-furan-2-acetic acid as a colourless solid (1.3g), δ CDCl₃ 1.99 (3H, s), 3.67 (2H, s), 6.11 (1H, s), 7.13 (1H, s). Reaction of 4-methyl-furan-2-acetic acid with methyl-2-amino-4-chlorobenzoate under the conditions described in Example 62 gave the title compound as colourless needles mp 276°C dec; δ (d₆-DMSO, 360MHz) 2.05 (3H, s), 6.68 (1H, s), 7.23 (1H, dd, *J* = 8.6 and 2.0Hz), 7.31 (1H, d, *J* = 2.0Hz), 7.48 (1H, s), 7.93 (1H, d, *J* = 8.6Hz), 11.64 (1H, br s).

### EXAMPLE 71

### 7-Chloro-2,4-dioxo-3(2-(5-phenyl)furanyl)-1,2,3,4-tetrahydroquinoline

5-Phenyl-2-furaldehyde (Kaltenbronn *et al*, J. Med. Chem., 1968, 902) was converted to 5-phenylfuran-2-acetic acid under the conditions of Breen *et al*, (Aust. J. Chem., 1973, 26, 2221). Reaction of 5-phenyl-furan-2-acetic acid with methyl 2-amino-4-chlorobenzoate under the conditions described in Example 62 gave the title compound as colourless needles mp > 300°C; (Found: C, 66.69; H, 3.56; N, 4.08. C₁₉H₁₂ClNO₃ requires C, 66.50; H, 3.70; N, 4.08%; δ (d₆-DMSO, 360MHz) 7.06 (2H, s), 7.24-7.46 (5H, m), 7.76 (2H, d, *J* = 7.4Hz), 8.00 (1H, d, *J* =8.7Hz), 10.80 (1H, br s), 11.66 (1H, br, s); *m / z* (EI) 337 (M⁺).

### EXAMPLE 72

### 7-Chloro-2,4-dioxo-3-(2-(5-methyl)thienyl)-1,2,3,4-tetrahydroquinoline

This compound was prepared in the same way as described in Example 60 except using 5-methyl-thienylacetic acid to give the title compound as a white crystalline solid; mp 285°C slow decomp. (MeOH, DMF, H₂O) (Found: C, 57.68; H, 3.71; N, 4.58; C₁₄H₁₀ClNO₂S requires C, 57.64; H, 3.46; N, 4.80%); δH (DMSO-d₆) 2.46 (3H, s, CH₃), 6.78 (1H, d, *J* = 3.7Hz, 4-thienyl-H), 7.24 (1H, dd, *J* = 8.5Hz and 1.9Hz, 6-H), 7.33 (1H, d, *J* = 1.9Hz, 8-H), 7.84 (1H, d, *J* = 3.7Hz, 3-thienyl-H), 8.04 (1H, d, *J* = 8.5Hz, 5-H), 11.15 (1H, bs, 3-H), 11.72 (1H, s, NH); *m / z* 292 (M+1).

### EXAMPLE 73

### 3-(2-(5-Benzyl)thienyl)-7-chloro-2,4-dioxo-1,2,3,4-tetrahydroquinoline

This compound was prepared in the same way as described in Example 60 except using 5-benzylthienylacetic acid to give the title compound as a white crystalline solid; mp 195°C slow decomp. (DMF, H₂O) (Found: C, 63.48; H, 3.56; N, 3.77; C₂₀H₁₄ClNO₂S.0.92H₂O requires C, 63.86; H, 4.00; N, 3.72%) δH (DMSO-d₆) 4.14 (2H, s, CH₂), 6.86 (1H, d, *J* = 3.8Hz, 3-thienyl-H), 7.19-7.34 (7H, m, 8-H, 5-H, Ar), 7.89 (1H, d, *J* = 3.8Hz, 4-thienyl-H), 8.04 (1H, d, *J* = 8.5Hz, 5-H), 11.24 (1H, bs, 3-H), 11.74 (1H, s, NH); *m / z* 368 (M+1).

### EXAMPLE 74

### 3-(3-Benzothienyl)-7-chloro-2,4-dioxo-1,2,3,4-tetrahydroquinoline

To a solution of methyl-4-chloroanthranilate (1g, 5.4mmol) and methyl benzothienyl-2-acetate (1.3g, 5.94mmol) in dry tetrahydrofuran (50ml) was added a solution of potassium hexamethyldisilazide in toluene (0.5M, 26ml, 12.5mmol) and the reaction stirred for 2h. Methanol (15ml) was added and the solvent evaporated. The residue was partitioned between aqueous sodium hydroxide (1M, 40ml) and diethyl ether (40ml), and the aqueous layer was acidified with hydrochloric acid (5M). The resultant precipitate was collected and recrystallised from dimethylformamide/water to afford the title compound as a white solid; mp 320°C slow decomp. (from DMF/H₂O) (Found: C, 61.65; H, 2.86; N, 4.08; C₁₇H₁₀ClNO₂S.0.1H₂O requires C, 61.95; H, 3.12; N, 4.25) δH (DMSO-d₆) 7.22 (1H, dd, *J* = 8.5Hz and 1.9Hz, 6-H), 7.34-7.41 (4H, m, 8-H, Ar), 7.87 (1H, s, 2-thienyl-H), 7.94 (1H, d, *J* = 8.5Hz, 7-thienyl-H), 8.01 (1H, d, *J* = 8.5Hz, 5-H), 10.4 (1H, bs, 3-H), 11.59 (1H, s, NH); m/z 328 (M+1).

### Tablet Preparation

Tablets containing 1.0, 2.0, 25.0, 26.0, 50.0 and 100.0mg, respectively of the following compounds are prepared as illustrated below:
3-(Cyclopropanecarbonyl)-7-chloro-2,4-dioxo-1,2,3,4-tetrahydroquinoline
3-(5'-[2'-Methylfuranyl])-7-chloro-2,4-dioxo-1,2,3,4-tetrahydroquinoline
Methyl 7-Chloro-2,4-dioxo-3-(1,2,3,4-tetrahydroquinoline)carboxylate
(E)-(3-Methoxyphenyl)prop-2-enyl 7-Chloro-2,4-dioxo-3-(1,2,3,4-tetrahydroquinoline)carboxylate
7-Chloro-2,4-dioxo-3-(3-pyridyl)-1,2,3,4-tetrahydroquinoline

| TABLE FOR DOSES CONTAINING FROM 1-25MG OF THE ACTIVE COMPOUND | | | |
|---|---|---|---|
| | Amount-mg | | |
| Active Compound | 1.0 | 2.0 | 25.0 |
| Microcrystalline cellulose | 49.25 | 48.75 | 37.25 |
| Modified food corn starch | 49.25 | 48.75 | 37.25 |
| Magnesium stearate | 0.50 | 0.50 | 0.50 |

| TABLE FOR DOSES CONTAINING FROM 26-100MG OF THE ACTIVE COMPOUND | | | |
|---|---|---|---|
| | Amount-mg | | |
| Active Compound | 26.0 | 50.0 | 100.0 |
| Microcrystalline cellulose | 52.0 | 100.0 | 200.0 |
| Modified food corn starch | 2.21 | 4.25 | 8.5 |
| Magnesium stearate | 0.39 | 0.75 | 1.5 |

All of the active compound, cellulose, and a portion of the corn starch are mixed and granulated to 10% corn starch paste. The resulting granulation is sieved, dried and blended with the remainder of the corn starch and the magnesium stearate. The resulting granulation is then compressed into tablets containing 1.0mg, 2.0mg, 25.0mg, 26.0mg, 50.0mg and 100mg of the active ingredient per tablet.

## Claims

1. The use of a compound of formula IA or a pharmaceutically acceptable salt thereof or a prodrug thereof: 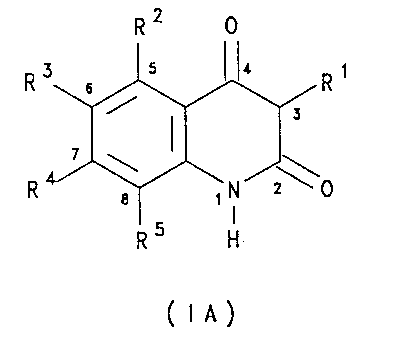 wherein
R¹ is a group of part formula (i) or (ii):
-(CH=CH)ₙ-T (i) 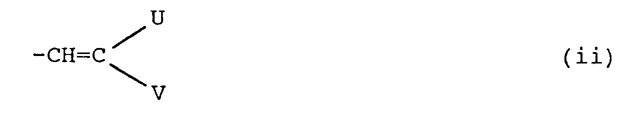
wherein
U and V independently represent cyano, carboxy, -COR⁶, -CO₂R⁶, -CO.SR⁶, -CONHOH or -CONHNH₂;
n is zero or 1;
T represents cyano, carboxy, -COR⁶, -CO₂R⁶, -CO.SR⁶, -CONHOH, -CONHNH₂ or a group of formula 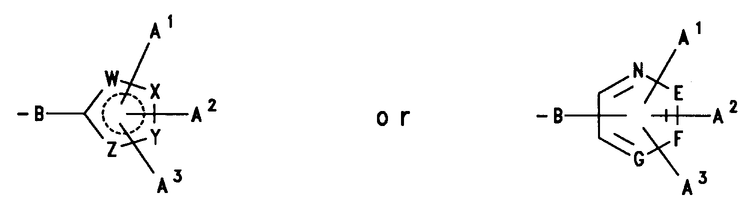 in which the broken circle represents two non-adjacent double bonds in any position in the five-membered ring;
B represents a bond or a carbonyl group (C=O);
W, X, Y and Z independently represent oxygen, sulphur, nitrogen or carbon, provided that no more than one of W, X, Y and Z represents oxygen or sulphur, at least one of W, X, Y and Z represents carbon and at least one of W, X, Y and Z is other than carbon;
one of E, F and G represents nitrogen or carbon and the remainder represent carbon;
A¹, A² and A³ represent one, two or three substituents not exceeding the maximum number permissible by the disposition of heteroatoms in the five- or six-membered ring, which substituents are independently selected from hydrogen, halogen, cyano, trifluoromethyl, nitro, -OR^{a}, -SR^{a}, -SOR^{a}, -SO₂R^{a}, -SO₂NR^{a}R^{a}, -NR^{a}R^{b}, -NR^{a}COR^{b}, -NR^{a}CO₂R^{b}, -COR^{a}, -CO₂^{a}, -CONR^{a}R^{b}, or a hydrocarbon group comprising a straight-chained, branched or cyclic group containing up to 18 carbon atoms; or A¹ and A² or A² and A³ together represent the residue of an aromatic or heteroaromatic ring;
R², R³, R⁴ and R⁵ independently represent hydrogen, halogen, cyano, trifluoromethyl, nitro, -OR^{a}, -SR^{a}, -SOR^{a}, -SO₂R^{a}, -SO₂NR^{a}R^{b}, -NR^{a}R^{b}, -NR^{a}COR^{b}, -NR^{a}CO₂R^{b}, -COR^{a}, -CO₂R^{a}, -CONR^{a}R^{b}, or a hydrocarbon group comprising a straight-chained, branched or cyclic group containing up to 18 carbon atoms; or R² and R³, R³ and R⁴ or R⁴ and R⁵ together represent the residue of an aromatic or heteroaromatic ring;
R⁶ represents a hydrocarbon group comprising a straight-chained, branched or cyclic group containing up to 18 carbon atoms; and
R^{a} and R^{b} independently represent hydrogen or a hydrocarbon group comprising a straight-chained, branched or cyclic group containing up to 18 carbon atoms; for the manufacture of a medicament for the treatment and/or prevention of conditions which require the administration of a selective non-competitive antagonist of NMDA receptors.

2. The use of a compound of formula IA as defined in claim 1 or a pharmaceutically acceptable salt thereof or a prodrug thereof for the manufacture of a medicament for the treatment and/or prevention of conditions which require the administration of an antagonist of AMPA receptors.

3. A compound of formula IB or a salt or prodrug thereof: 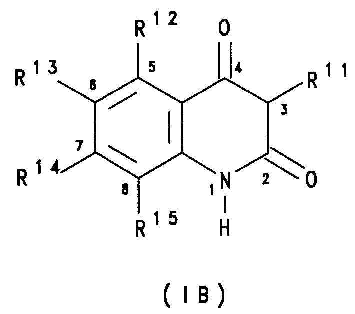 wherein
R¹¹ is a group of part formula (i) or (ii):
-(CH=CH)ₙ-T (i) 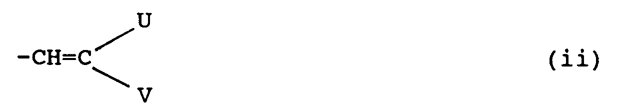
wherein
U and V independently represent cyano, carboxy, -COR⁶, -CO₂R⁶, -CO.SR⁶, -CONHOH or -CONHNH₂;
n is zero or 1;
T represents cyano, carboxy, -COR⁶, -CO₂R⁶, -CO.SR⁶, -CONHOH, -CONHNH₂ or a group of formula 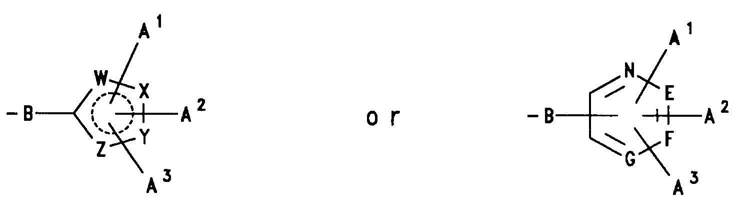 in which the broken circle represents two non-adjacent double bonds in any position in the five-membered ring;
B represents a bond or a carbonyl group (C=O);
W, X, Y and Z independently represent oxygen, sulphur, nitrogen or carbon, provided that no more than one of W, X, Y and Z represents oxygen or sulphur, at least one of W, X, Y and Z represents carbon and at least one of W, X, Y and Z is other than carbon;
one of E, F and G represents nitrogen or carbon and the remainder represent carbon;
A¹, A² and A³ represent one, two or three substituents not exceeding the maximum number permissible by the disposition of heteroatoms in the five- or six-membered ring, which substituents are independently selected from hydrogen, halogen, cyano, trifluoromethyl, nitro, -OR^{a}, -SR^{a}, -SOR^{a}, -SO₂R^{a}, -SO₂NR^{a}R^{b}, -NR^{a}R^{b}, -NR^{a}COR^{b}, -NR^{a}CO₂R^{b}, -COR^{a}, -CO₂R^{a}, -CONR^{a}R^{b}, or a hydrocarbon group comprising a straight-chained, branched or cyclic group containing up to 18 carbon atoms; or A¹ and A² or A² and A³ together represent the residue of an aromatic or heteroaromatic ring;
R¹², R¹³, R¹⁴ and R¹⁵ independently represent hydrogen, halogen, cyano, trifluoromethyl, nitro, -OR^{a}, -SR^{a}, -SOR^{a}, -SO₂R^{a}, -SO₂NR^{a}R^{b}, -NR^{a}R^{b}, -NR^{a}COR^{b}, -NR^{a}CO₂R^{b}, -COR^{a}, -CO₂R^{a}, -CONR^{a}R^{b} or a hydrocarbon group comprising a straight-chained, branched or cyclic group containing up to 18 carbon atoms; or R¹² and R¹³, R¹³ and R¹⁴ or R¹⁴ and R¹⁵ together represent the residue of an aromatic or heteroaromatic ring;
R⁶ represents a hydrocarbon group comprising a straight-chained, branched or cyclic group containing up to 18 carbon atoms; and
R^{a} and R^{b} independently represent hydrogen or a hydrocarbon group comprising a straight-chained, branched or cyclic group containing up to 18 carbon atoms;
provided that:
(a) when two of the substituents R¹² to R¹⁵ represent hydrogen and the other two substituents R¹² to R¹⁵ independently represent hydrogen, C₁₋₈ alkyl, halogen, C₁₋₅ alkoxy, nitro, cyano, C₁₋₃ haloalkyl, carboxy or C₂₋₄ alkoxycarbonyl, then R¹¹ does not represent a benzoyl group optionally mono-, di- or trisubstituted by C₁₋₈ alkyl, halogen, C₁₋₅ alkoxy, nitro, cyano, C₁₋₃ haloalkyl, carboxy or C₂₋₄ alkoxycarbonyl;
(b) when R¹² to R¹⁵ independently represent hydrogen, C₁₋₅ alkyl, C₁₋₅ alkoxy, benzyloxy, C₂₋₆ alkoxycarbonyl, nitro or halogen, then R¹¹ does not represent acetyl;
(c) when one of R¹² to R¹⁵ represents hydrogen or methoxy and the remainder represent hydrogen, then R¹¹ does not represent -CONHNH₂ or -COR⁶, in which R⁶ is C₂₋₉ alkyl;
(d) when two of the substituents R¹² to R¹⁵ represent hydrogen and the other two substituents R¹² to R¹⁵ independently represent hydrogen, halogen, C₁₋₄ alkyl, C₁₋₄ alkoxy, nitro or trifluoromethyl or together represent methylenedioxy, then R¹¹ does not represent cyano, carboxy or -CO₂R⁶, in which R⁶ is C₁₋₉ alkyl, C₃₋₆ alkenyl or C₃₋₆ alkynyl;
(e) when two of the substituents R¹² to R¹⁵ represent hydrogen, a third substituent R¹² to R¹⁵ represents an optionally substituted C₅₋₈ cycloalkyl or cycloalkenyl group or adamantyl, and the remaining substituent R¹² to R¹⁵ represents hydrogen, halogen, lower alkyl or lower alkoxy, then R¹¹ does not represent carboxy or -COR¹⁶, in which R¹⁶ is an etherified hydroxyl group;
(f) when R¹² to R¹⁵ each represents hydrogen and R¹¹ is a group of part formula (ii) as defined above, then U and V are not simultaneously carboxy, and U is not carboxy or ethoxycarbonyl when V is cyano; and
(g) when R¹² to R¹⁵ each represents hydrogen, then R¹¹ does not represent unsubstituted 2-pyridyl, 3-pyridyl, 2-furyl or 2-benzothiazolyl.

4. A compound as claimed in claim 3 represented by formula IIA, and salts and prodrugs thereof: 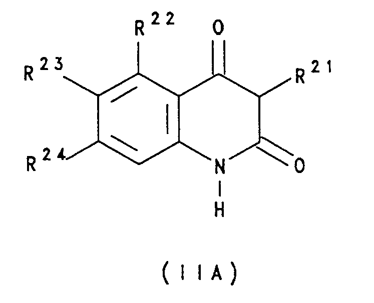 wherein
R²¹ represents -COR²⁶ or -CO₂R²⁶;
R²², R²³ and R²⁴ independently represent hydrogen, halogen, cyano, trifluoromethyl, nitro, hydroxy, amino, carboxy, C₁₋₆ alkyl, C₁₋₆ alkoxy, C₁₋₆ alkylthio or C₂₋₆ alkoxycarbonyl, provided that at least one of R²², R²³ and R²⁴ is other than hydrogen; and
R²⁶ represents C₃₋₇ cycloalkyl, aryl(C₁₋₆)-alkyl, aryl(C₂₋₆)alkenyl, aryl(C₂₋₆)alkynyl, heteroaryl(C₁₋₆)alkyl or heteroaryl(C₂₋₆)alkenyl, any of which groups may be optionally substituted.

5. A compound as claimed in claim 3 represented by formula IIB, and salts and prodrugs thereof: 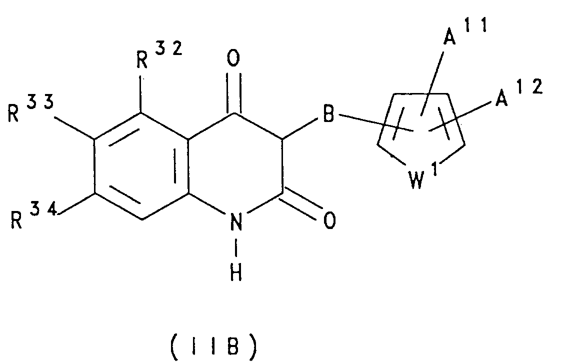 wherein
W¹ represents oxygen, sulphur or N-A¹³;
A¹¹ and A¹² independently represent hydrogen, halogen, cyano, trifluoromethyl, nitro, hydroxy, amino, carboxy, C₁₋₆ alkyl, C₂₋₆ alkenyl, C₂₋₆ alkynyl, C₃₋₇ cycloalkyl, C₃₋₇ cycloalkyl(C₁₋₆)alkyl, aryl, aryl(C₁₋₆)alkyl, C₁₋₆ alkoxy, C₂₋₆ alkenyloxy, C₁₋₆ alkylthio, C₂₋₆ alkenylthio, C₂₋₆ alkylcarbonyl, arylcarbonyl or C₂₋₆ alkoxycarbonyl; or A¹¹ and A¹² together represent the residue of an optionally substituted aromatic or heteroaromatic ring;
A¹³ represents hydrogen, C₁₋₆ alkyl or aryl(C₁₋₆)alkyl;
B represents a bond or a carbonyl group (C=O); and
R³², R³³ and R³⁴ independently represent hydrogen, halogen, cyano, trifluoromethyl, nitro, hydroxy, amino, carboxy, C₁₋₆ alkyl, C₁₋₆ alkoxy, C₁₋₆ alkylthio or C₂₋₆ alkoxycarbonyl, provided that at least one of R³², R³³ and R³⁴ is other than hydrogen.

6. A compound as claimed in claim 3 represented by formula IIC, and salts and prodrugs thereof: 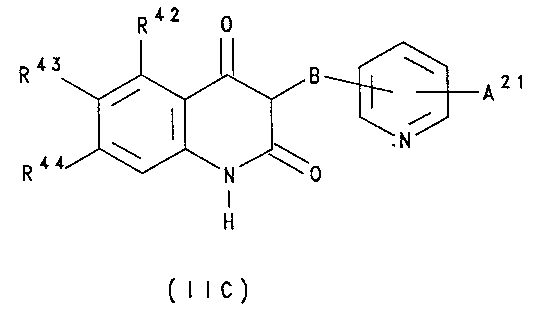 wherein
A²¹ represents hydrogen, halogen, cyano, trifluoromethyl, nitro, hydroxy, amino, carboxy, C₁₋₆ alkyl, C₁₋₆ alkoxy, C₁₋₆ alkylthio, C₂₋₆ alkylcarbonyl, arylcarbonyl or C₂₋₆ alkoxycarbonyl;
B represents a bond or a carbonyl group (C=O); and
R⁴², R⁴³ and R⁴⁴ independently represent hydrogen, halogen, cyano, trifluoromethyl, nitro, hydroxy, amino, carboxy, C₁₋₆ alkyl, C₁₋₆ alkoxy, C₁₋₆ alkylthio or C₂₋₆ alkoxycarbonyl, provided that at least one of R⁴², R⁴³ and R⁴⁴ is other than hydrogen.

7. A compound as claimed in claim 3 selected from:
3-benzyloxycarbonyl-7-chloro-2,4-dioxo-1,2,3,4-tetrahydroquinoline;
7-chloro-2,4-dioxo-3-(2-phenylethoxy)carbonyl-1,2,3,4-tetrahydroquinoline;
7-chloro-2,4-dioxo-3-(3-phenylpropoxy)carbonyl-1,2,3,4-tetrahydroquinoline;
7-chloro-2,4-dioxo-3-[2-(3-hydroxyphenyl)ethoxy]carbonyl-1,2,3,4-tetrahydroquinoline;
7-chloro-2,4-dioxo-3-[3-(4-hydroxyphenyl)propoxy]carbonyl-1,2,3,4-tetrahydroquinoline;
7-chloro-2,4-dioxo-3-[2-(2-hydroxyphenyl)ethoxy]carbonyl-1,2,3,4-tetrahydroquinoline;
7-chloro-3-cyclopropylmethoxycarbonyl-2,4-dioxo-1,2,3,4-tetrahydroquinoline;
7-chloro-2,4-dioxo-3-[2-(4-hydroxyphenyl)ethoxy]carbonyl-1,2,3,4-tetrahydroquinoline;
7-chloro-2,4-dioxo-3-(3-hydroxyphenylmethoxy)carbonyl-1,2,3,4-tetrahydroquinoline;
7-chloro-2,4-dioxo-3-(pyrid-2-ylmethoxy)carbonyl-1,2,3,4-tetrahydroquinoline;
2,4-dioxo-3-[3-(4-hydroxyphenyl)propoxy]carbonyl-7-nitro-1,2,3,4-tetrahydroquinoline;
7-chloro-2,4-dioxo-3-(2-hydroxyethoxy)carbonyl-1,2,3,4-tetrahydroquinoline;
7-chloro-2,4-dioxo-3-(3-thienyl)carbonyl-1,2,3,4-tetrahydroquinoline;
7-chloro-2,4-dioxo-3-(2-furyl)carbonyl-1,2,3,4-tetrahydroquinoline;
7-chloro-2,4-dioxo-3-(2-thienyl)carbonyl-1,2,3,4-tetrahydroquinoline;
7-chloro-3-(2,5-dimethyl-3-furyl)carbonyl-2,4-dioxo-1,2,3,4-tetrahydroquinoline;
7-chloro-2,4-dioxo-3-(5-methyl-2-furyl)carbonyl-1,2,3,4-tetrahydroquinoline;
7-chloro-2,4-dioxo-3-[2-(5-nitrobenzofuryl)carbonyl]-1,2,3,4-tetrahydroquinoline;
3-[2-(benzofuryl)carbonyl]-7-chloro-2,4-dioxo-1,2,3,4-tetrahydroquinoline;
3-[2-(benzo[b]thienyl)carbonyl]-7-chloro-2,4-dioxo-1,2,3,4-tetrahydroquinoline;
3-benzylcarbonyl-7-chloro-2,4-dioxo-1,2,3,4-tetrahydroquinoline;
7-chloro-2,4-dioxo-3-(5-methyl-2-thienyl)carbonyl-1,2,3,4-tetrahydroquinoline;
7-chloro-2,4-dioxo-3-(3-methyl-2-thienyl)carbonyl-1,2,3,4-tetrahydroquinoline;
7-chloro-3-(2,5-dimethyl-3-thienyl)carbonyl-2,4-dioxo-1,2,3,4-tetrahydroquinoline;
7-chloro-2,4-dioxo-3-[2-(3-thienyl)ethenyl]carbonyl-1,2,3,4-tetrahydroquinoline;
3-(5-bromo-2-thienyl)carbonyl-7-chloro-2,4-dioxo-1,2,3,4-tetrahydroquinoline;
7-chloro-2,4-dioxo-3-(1-methylpyrrol-2-yl)carbonyl-1,2,3,4-tetrahydroquinoline;
7-chloro-2,4-dioxo-3-(1-methylpyrrol-3-yl)carbonyl-1,2,3,4-tetrahydroquinoline;
7-chloro-2,4-dioxo-3-(1-methylindol-3-yl)carbonyl-1,2,3,4-tetrahydroquinoline;
7-chloro-3-cyclopropylcarbonyl-2,4-dioxo-1,2,3,4-tetrahydroquinoline;
7-chloro-2,4-dioxo-3-(5-methyl-2-furyl)-1,2,3,4-tetrahydroquinoline;
7-chloro-2,4-dioxo-3-(5-ethyl-2-furyl)-1,2,3,4-tetrahydroquinoline;
7-chloro-2,4-dioxo-3-(1-methylpyrrol-2-yl)-1,2,3,4-tetrahydroquinoline;
7-chloro-2,4-dioxo-3-(2-furyl)-1,2,3,4-tetrahydroquinoline;
3-(4-benzoyl-1-methylpyrrol-2-yl)-7-chloro-2,4-dioxo-1,2,3,4-tetrahydroquinoline;
3-(5-benzoyl-1-methylpyrrol-2-yl)-7-chloro-2,4-dioxo-1,2,3,4-tetrahydroquinoline;
7-chloro-2,4-dioxo-3-[2-(5-methoxyindol-3-yl)ethoxy]carbonyl-1,2,3,4-tetrahydroquinoline;
7-chloro-2,4-dioxo-3-[3-(3-methoxyphenyl)prop-2-ynyloxy]carbonyl-1,2,3,4-tetrahydroquinoline;
7-chloro-2,4-dioxo-3-(3-indol-3-ylpropoxy)carbonyl-1,2,3,4-tetrahydroquinoline;
3-[2-[3,4-bis(methoxymethoxy)phenyl]ethoxy]carbonyl-7-chloro-2,4-dioxo-1,2,3,4-tetrahydroquinoline;
7-chloro-2,4-dioxo-3-[4-(3-hydroxyphenyl)butoxy]carbonyl-1,2,3,4-tetrahydroquinoline;
7-chloro-2,4-dioxo-3-[3-(3-hydroxyphenyl)propoxy]carbonyl-1,2,3,4-tetrahydroquinoline;
7-chloro-2,4-dioxo-3-(2-indol-3-ylethoxy)carbonyl-1,2,3,4-tetrahydroquinoline;
7-chloro-2,4-dioxo-3-[3-(2-hydroxyphenyl)propoxy]carbonyl-1,2,3,4-tetrahydroquinoline;
3-[2-[4-(N-t-butoxycarbonylaminomethyl)phenyl]ethoxy]carbonyl-7-chloro-2,4-dioxo-1,2,3,4-tetrahydroquinoline;
7-chloro-2,4-dioxo-3-[2-(3-thienyl)ethoxy]carbonyl-1,2,3,4-tetrahydroquinoline;
7-chloro-2,4-dioxo-3-[2-(2-thienyl)ethoxy]carbonyl-1,2,3,4-tetrahydroquinoline;
7-chloro-2,4-dioxo-3-[3-(3-hydroxyphenyl)prop-2-ynyloxy]carbonyl-1,2,3,4-tetrahydroquinoline;
7-chloro-2,4-dioxo-3-[3-(3-methoxyphenyl)prop-2-enyloxy]carbonyl-1,2,3,4-tetrahydroquinoline;
7-chloro-2,4-dioxo-3-[2-(phenylthio)ethoxy]carbonyl-1,2,3,4-tetrahydroquinoline;
7-chloro-2,4-dioxo-3-[2-(phenyl)ethylthio]carbonyl-1,2,3,4-tetrahydroquinoline;
7-chloro-2,4-dioxo-3-(2-indol-3-ylethoxy)carbonyl-5-iodo-1,2,3,4-tetrahydroquinoline;
7-chloro-2,4-dioxo-3-(3-methyl-1,2,4-oxadiazol-5-yl)-1,2,3,4-tetrahydroquinoline;
7-chloro-2,4-dioxo-3-[2-(ethoxycarbonyl)ethenyl]-1,2,3,4-tetrahydroquinoline;
7-chloro-2,4-dioxo-1,2,3,4-tetrahydroquinoline-3-hydroxamic acid;
7-chloro-2,4-dioxo-5-iodo-1,2,3,4-tetrahydroquinoline-3-hydroxamic acid;
7-chloro-2,4-dioxo-1,2,3,4-tetrahydroquinoline-3-carboxylic acid hydrazide;
7-chloro-2,4-dioxo-3-(3-thienyl)-1,2,3,4-tetrahydroquinoline;
7-chloro-2,4-dioxo-3-(2-thienyl)-1,2,3,4-tetrahydroquinoline;
7-chloro-2,4-dioxo-3-(2-pyridyl)-1,2,3,4-tetrahydroquinoline;
7-chloro-2,4-dioxo-3-(3-pyridyl)-1,2,3,4-tetrahydroquinoline;
7-chloro-2,4-dioxo-3-(4-pyridyl)-1,2,3,4-tetrahydroquinoline;
3-(2-benzofuryl)-7-chloro-2,4-dioxo-1,2,3,4-tetrahydroquinoline;
3-(3-benzofuryl)-7-chloro-2,4-dioxo-1,2,3,4-tetrahydroquinoline;
7-chloro-2,4-dioxo-3-(3-methyl-2-furyl)-1,2,3,4-tetrahydroquinoline;
7-chloro-2,4-dioxo-3-(2-methyl-3-furyl)-1,2,3,4-tetrahydroquinoline;
7-chloro-2,4-dioxo-3-(4-isopropyl-2-furyl)-1,2,3,4-tetrahydroquinoline;
7-chloro-2,4-dioxo-3-(4-methyl-2-furyl)-1,2,3,4-tetrahydroquinoline;
7-chloro-2,4-dioxo-3-(5-phenyl-2-furyl)-1,2,3,4-tetrahydroquinoline;
7-chloro-2,4-dioxo-3-(5-methyl-2-thienyl)-1,2,3,4-tetrahydroquinoline;
3-(5-benzyl-2-thienyl)-7-chloro-2,4-dioxo-1,2,3,4-tetrahydroquinoline;
3-(3-benzo[b]thienyl)-7-chloro-2,4-dioxo-1,2,3,4-tetrahydroquinoline;
and salts and prodrugs thereof.

8. A compound selected from:
7-chloro-3-cyano-2,4-dioxo-1,2,3,4-tetrahydroquinoline; 2,4-dioxo-3-ethoxycarbonyl-6-nitro-1,2,3,4-tetrahydroquinoline;
7-chloro-2,4-dioxo-3-methoxycarbonyl-1,2,3,4-tetrahydroquinoline;
7-chloro-2,4-dioxo-3-n-propoxycarbonyl-1,2,3,4-tetrahydroquinoline;
6,7-dinitro-2,4-dioxo-3-ethoxycarbonyl-1,2,3,4-tetrahydroquinoline;
7-chloro-2,4-dioxo-3-ethoxycarbonyl-1,2,3,4-tetrahydroquinoline;
2,4-dioxo-3-ethoxycarbonyl-7-nitro-1,2,3,4-tetrahydroquinoline;
7-chloro-2,4-dioxo-3-n-pentyloxycarbonyl-1,2,3,4-tetrahydroquinoline;
7-chloro-2,4-dioxo-3-(2-propenyloxy)carbonyl-1,2,3,4-tetrahydroquinoline;
7-chloro-2,4-dioxo-3-ethoxycarbonyl-5-iodo-1,2,3,4-tetrahydroquinoline;
7-chloro-2,4-dioxo-5-ethyl-3-methoxycarbonyl-1,2,3,4-tetrahydroquinoline;
3-carboxy-7-chloro-2,4-dioxo-1,2,3,4-tetrahydroquinoline; and salts and prodrugs thereof.

9. A pharmaceutical composition comprising at least one compound as claimed in any one of claims 3 to 8 or a pharmaceutically acceptable salt thereof or a prodrug thereof in association with one or more pharmaceutically acceptable carriers and/or excipients.

10. A process for the preparation of a compound of formula IB as defined in claim 3, which process comprises:
(A) the reductive cyclisation of a compound of formula III: 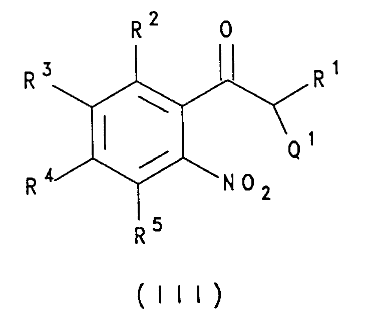 wherein R¹, R², R³, R⁴ and R⁵ are as defined in claim 1; and Q¹ represents a reactive carboxylate moiety; or
(B) cyclisation of a compound of formula V: 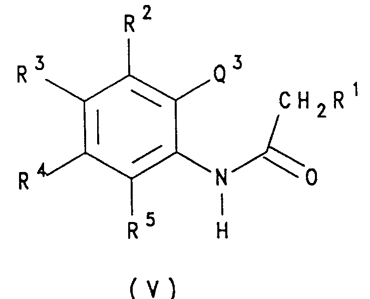 wherein R¹, R², R³, R⁴ and R⁵ are as defined in claim 1; and Q³ represents a reactive carboxylate moiety; or
(C) reacting a compound of formula Q¹-CH₂-R¹ with a compound of formula VII: 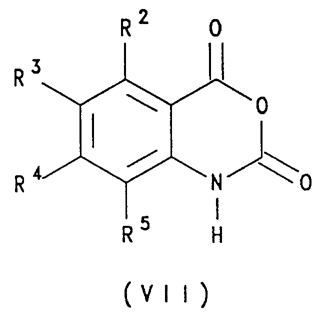 wherein R¹, R², R³, R⁴ and R⁵ are as defined in claim 1, and Q¹ is as defined above; and
(D) if desired, converting a compound of formula IB initially obtained into a further compound of formula IB using methods known per se.

## Patentansprüche

1. Die Verwendung einer Verbindung der Formel IA oder eines pharmazeutisch annehmbaren Salzes hiervon oder eines Prodrugs hiervon: 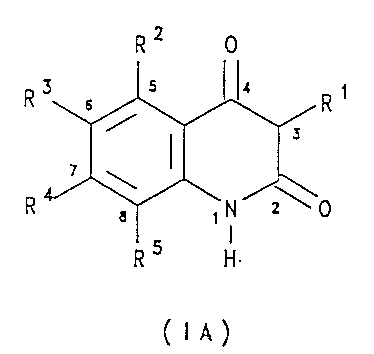 worin
R¹ eine Gruppe mit der Teilformel (i) oder (ii) ist:
-(CH=CH)ₙ-T (i) 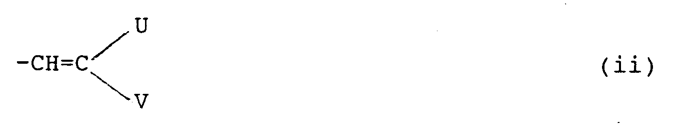
worin
U und V unabhängig voneinander Cyan, Carboxy, COR⁶, CO₂R⁶, CO.SR⁶, -CONHOH oder -CONHNH₂ bedeuten;
n Null oder 1 ist;
T bedeutet Cyan, Carboxy, COR⁶, CO₂R⁶, CO.SR⁶, -CONHOH, -CONHNH₂ oder eine Gruppe der Formel 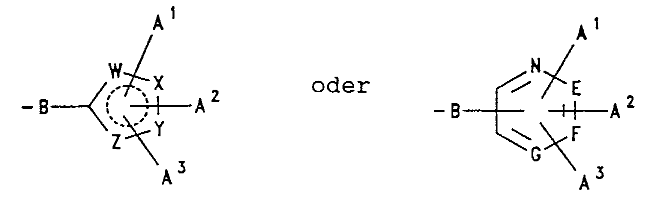 worin der gestrichelte Kreis zwei nicht benachbarte Doppelbindungen in jeder beliebigen Stellung des fünfgliedrigen Ringes bedeutet;
B bedeutet eine Bindung oder eine Carbonylgruppe (C=O);
W, X, Y und Z bedeuten unabhängig voneinander Sauerstoff, Schwefel, Stickstoff oder Kohlenstoff, unter der Bedingung, daß nicht mehr als einer der Reste W, X, Y und Z Sauerstoff oder Schwefel bedeutet, mindestens einer von W, X, Y und Z Kohlenstoff bedeutet und mindestens einer von W, X, Y und Z eine andere Bedeutung als Kohlenstoff besitzt;
einer der Reste E, F und G bedeutet Stickstoff oder Kohlenstoff und die restlichen bedeuten Kohlenstoff;
A¹, A² und A³ bedeuten einen, zwei oder drei Substituenten, welche die maximale Zahl, die durch die Anordnung der Heteroatome in dem fünf- oder sechsgliedrigen Ring zulässig ist, nicht überschreiten, wobei diese Substituenten unabhängig voneinander aus Wasserstoff, Halogen, Cyan, Trifluormethyl, Nitro, -OR^{a}, -SR^{a}, -SOR^{a}, -SO₂R^{a}, -SO₂NR^{a}R^{b}, -NR^{a}R^{b}, -NR^{a}COR^{b}, -NR^{a}CO₂R^{b}, -COR^{a}, -CO₂R^{a}, -CONR^{a}R^{b}, oder aus einer Kohlenwasserstoffgruppe, die eine geradkettige, verzweigte oder cyclische Gruppe, die bis zu 18 Kohlenstoffatome enthält, umfaßt, ausgewählt sind; oder A¹ und A² oder A² und A³ bedeuten zusammen den Rest eines aromatischen oder heteroaromatischen Ringes;
R², R³, R⁴ und R⁵ bedeuten unabhängig voneinander Wasserstoff, Halogen, Cyan, Trifluormethyl, Nitro, -OR^{a}, -SR^{a}, -SOR^{a}, -SO₂R^{a}, -SO₂NR^{a}R^{b}, -NR^{a}R^{b}, -NR^{a}COR^{b}, -NR^{a}CO₂R^{b}, -COR^{a}, - CO₂R^{a}, -CONR^{a}R^{b}, oder eine Kohlenwasserstoffgruppe, die eine geradkettige, verzweigte oder cyclische Gruppe, die bis zu 18 Kohlenstoffatome enthält, umfaßt, oder R² und R³, R³ und R⁴ oder R⁴ und R⁵ bedeuten zusammen den Rest eines aromatischen oder heteroaromatischen Ringes;
R⁶ bedeutet eine Kohlenwasserstoffgruppe, die eine geradkettige, verzweigte oder cyclische Gruppe, die bis zu 18 Kohlenstoffatome enthält, umfaßt; und
R^{a} und R^{b} bedeuten unabhängig voneinander Wasserstoff oder eine Kohlenwasserstoffgruppe, die eine geradkettige, verzweigte oder cyclische Gruppe, die bis zu 18 Kohlenstoffatome enthält, umfaßt;
für die Herstellung eines Arzneimittels zur Behandlung und/oder Vorbeugung von Zuständen, welche die Verabreichung eines selektiven, nicht kompetitiven Antagonisten von NMDA-Rezeptoren erfordern.

2. Die Verwendung einer Verbindung der Formel IA, wie sie im Anspruch 1 definiert ist, oder eines pharmazeutisch annehmbaren Salzes hiervon oder eines Prodrugs hiervon für die Herstellung eines Arzneimittels zur Behandlung und/oder Vorbeugung von Zuständen, welche die Verabreichung eines Antagonisten von AMPA-Rezeptoren erfordern.

3. Eine Verbindung der Formel IB oder ein Salz oder ein Prodrug hiervon: 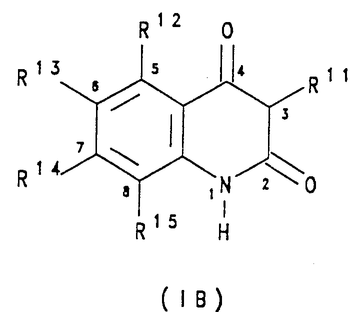 worin
R¹¹ eine Gruppe mit der Teilformel (i) oder (ii) ist:
-(CH=CH)ₙ-T (i) 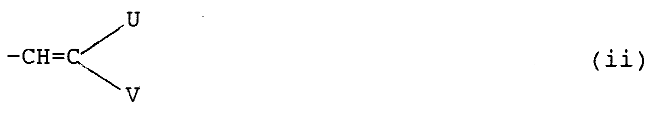
worin
U und V unabhängig voneinander Cyan, Carboxy, COR⁶, CO₂R⁶ , CO.SR⁶, -CONHOH oder -CONHNH₂ bedeuten;
n Null oder 1 ist;
T bedeutet Cyan, Carboxy, COR⁶, CO₂R⁶, CO.SR⁶, -CONHOH, -CONHNH₂ oder eine Gruppe der Formel 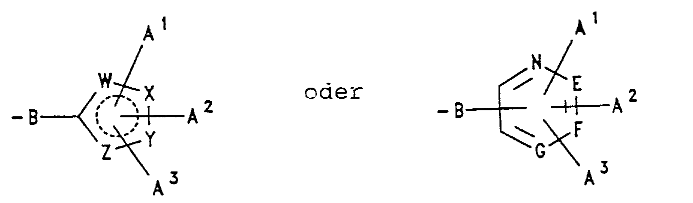 worin der gestrichelte Kreis zwei nicht benachbarte Doppelbindungen in jeder beliebigen Stellung des fünfgliedrigen Ringes bedeutet;
B bedeutet eine Bindung oder eine Carbonylgruppe (C=O);
W, X, Y und Z bedeuten unabhängig voneinander Sauerstoff, Schwefel, Stickstoff oder Kohlenstoff, unter der Bedingung, daß nicht mehr als einer der Reste W, X, Y und Z Sauerstoff oder Schwefel bedeutet, mindestens einer von W, X, Y und Z Kohlenstoff bedeutet und mindestens einer von W, X, Y und Z eine andere Bedeutung als Kohlenstoff besitzt;
einer der Reste E, F und G bedeutet Stickstoff oder Kohlenstoff und die restlichen bedeuten Kohlenstoff;
A¹, A² und A³ bedeuten einen, zwei oder drei Substituenten, welche die maximale Zahl, die durch die Anordnung der Heteroatome in dem fünf- oder sechsgliedrigen Ring zulässig ist, nicht überschreiten, wobei diese Substituenten unabhängig voneinander aus Wasserstoff, Halogen, Cyan, Trifluormethyl, Nitro, -OR^{a}, -SR^{a}, -SOR^{a}, -SO₂R^{a}, -SO₂NR^{a}R^{b}, -NR^{a}R^{b}, -NR^{a}COR^{b}, -NR^{a}CO₂R^{b}, -COR^{a}, -CO₂R^{a}, -CONR^{a}R^{b}, oder aus einer Kohlenwasserstoffgruppe, die eine geradkettige, verzweigte oder cyclische Gruppe, die bis zu 18 Kohlenstoffatome enthält, umfaßt, ausgewählt sind; oder A¹ und A² oder A² und A³ bedeuten zusammen den Rest eines aromatischen oder heteroaromatischen Ringes;
R¹², R¹³, R¹⁴ und R¹⁵ bedeuten unabhängig voneinander Wasserstoff, Halogen, Cyan, Trifluormethyl, Nitro, -OR^{a}, -SR^{a}, -SOR^{a}, -SO₂R^{a}, -SO₂NR^{a}R^{b}, -NR^{a}R^{b}, -NR^{a}COR^{b}, -NR^{a}CO₂R^{b}, -COR^{a}, -CO₂R^{a}, -CONR^{a}R^{b}, oder eine Kohlenwasserstoffgruppe, die eine geradkettige, verzweigte oder cyclische Gruppe, die bis zu 18 Kohlenstoffatome enthält, umfaßt, oder R¹² und R¹³, R¹³ und R¹⁴ oder R¹⁴ und R¹⁵ bedeuten zusammen den Rest eines aromatischen oder heteroaromatischen Ringes;
R⁶ bedeutet eine Kohlenwasserstoffgruppe, die eine geradkettige, verzweigte oder cyclische Gruppe, die bis zu 18 Kohlenstoffatome enthält, umfaßt; und
R^{a} und R^{b} bedeuten unabhängig voneinander Wasserstoff oder eine Kohlenwasserstoffgruppe, die eine geradkettige, verzweigte oder cyclische Gruppe, die bis zu 18 Kohlenstoffatome enthält, umfaßt;
unter der Bedingung, daß
(a) wenn zwei der Substituenten R¹² bis R¹⁵ Wasserstoff bedeuten und die anderen beiden Substituenten R¹² bis R¹⁵ unabhängig voneinander Wasserstoff, C₁₋₈ Alkyl, Halogen, C₁₋₅ Alkoxy, Nitro, Cyan, C₁₋₃ Halogenalkyl, Carboxy oder C₂₋₄ Alkoxycarbonyl bedeuten, dann bedeutet R¹¹ nicht eine Benzoylgruppe, die gegebenenfalls durch C₁₋₈ Alkyl, Halogen, C₁₋₅ Alkoxy, Nitro, Cyan, C₁₋₃ Halogenalkyl, Carboxy oder C₂₋₄ Alkoxycarbonyl mono-, di- oder trisubstituiert ist;
(b) wenn R¹² bis R¹⁵ unabhängig voneinander Wasserstoff, C₁₋₅ Alkyl, C₁₋₅ Alkoxy, Benzyloxy, C₂₋₆ Alkoxycarbonyl, Nitro oder Halogen bedeuten, dann bedeutet R¹¹ nicht Acetyl;
(c) wenn einer der Substituenten R¹² bis R¹⁵ Wasserstoff oder Methoxy und die restlichen Wasserstoff bedeuten, dann bedeutet R¹¹ nicht -CONHNH₂ oder -COR⁶, worin R⁶ C₂₋₉ Alkyl ist;
(d) wenn zwei der Substituenten R¹² bis R¹⁵ Wasserstoff bedeuten und die anderen beiden Substituenten R¹² bis R¹⁵ unabhängig voneinander Wasserstoff, Halogen, C₁₋₄ Alkyl, C₁₋₄ Alkoxy, Nitro oder Trifluormethyl oder zusammen Methylendioxy bedeuten, dann bedeutet R¹¹ nicht Cyan, Carboxy oder -CO₂R⁶, worin R⁶ C₁₋₉ Alkyl, C₃₋₆ Alkenyl oder C₃₋₆ Alkinyl ist;
(e) wenn zwei der Substituenten R¹² bis R¹⁵ Wasserstoff bedeuten, ein dritter Substituent R¹² bis R¹⁵ eine gegebenenfalls substituierte C₅₋₈ Cycloalkyl- oder Cycloalkenylgruppe oder Adamantyl bedeutet, und der restliche Substituent R¹² bis R¹⁵ Wasserstoff, Halogen, niederes Alkyl oder niederes Alkoxy bedeutet, dann bedeutet R¹¹ nicht Carboxy oder -COR¹⁶, worin R¹⁶ eine veretherte Hydroxylgruppe ist;
(f) wenn jeder der Substituenten R¹² bis R¹⁵ Wasserstoff bedeutet und R¹¹ eine Gruppe der Teilformel (ii), wie sie oben definiert, ist, dann sind U und V nicht gleichzeitig Carboxy, und U ist nicht Carboxy oder Ethoxycarbonyl, wenn V Cvan ist; und
(g) wenn jeder der Substituenten R¹² bis R¹⁵ Wasserstoff bedeutet, dann bedeutet R¹¹ nicht unsubstituiertes 2-Pyridyl, 3-Pyridyl, 2-Furyl oder 2-Benzothiazolyl.

4. Eine Verbindung, wie sie im Anspruch 3 beansprucht wird, dargestellt durch die Formel IIA, und Salze und Prodrugs hiervon: 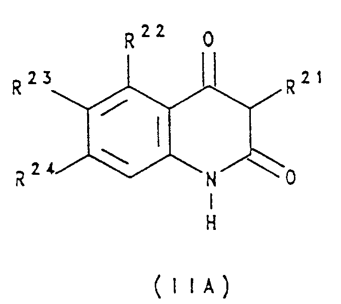 worin
R²¹ -COR²⁶ oder -CO₂R²⁶ bedeutet;
R²², R²³ und R²⁴ unabhängig voneinander Wasserstoff, Halogen, Cyan, Trifluormethyl, Nitro, Hydroxy, Amino, Carboxy, C₁₋₆ Alkyl, C₁₋₆ Alkoxy, C₁₋₆ Alkylthio oder C₂₋₆ Alkoxycarbonyl bedeuten, unter der Bedingung, daß mindestens einer der Reste R²², R²³ und R²⁴ eine andere Bedeutung als Wasserstoff aufweist; und
R²⁶ C₃₋₇ Cycloalkyl, Aryl-(C₁₋₆)alkyl, Aryl-(C₂₋₆)alkenyl, Aryl-(C₂₋₆)alkinyl, Heteroaryl-(C₁₋₆)alkyl oder Heteroaryl-(C₂₋₆)alkenyl bedeutet, wobei jede dieser Gruppen gegebenenfalls substituiert sein kann.

5. Eine Verbindung, wie sie im Anspruch 3 beansprucht wird, dargestellt durch die Formel IIB, und Salze und Prodrugs hiervon: 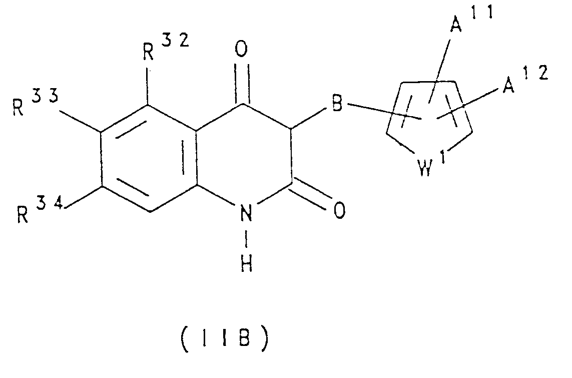 worin
W¹ Sauerstoff, Schwefel oder N-A¹³ bedeutet,
A¹¹ und A¹² bedeuten unabhängig voneinander Wasserstoff, Halogen, Cyan, Trifluormethyl, Nitro, Hydroxy, Amino, Carboxy, C₁₋₆ Alkyl, C₂₋₆ Alkenyl, C₂₋₆ Alkinyl, C₃₋₇ Cycloalkyl, C₃₋₇ Cycloalkyl-(C₁₋₆)alkyl, Aryl, Aryl-(C₁₋₆)-alkyl, C₁₋₆ Alkoxy, C₂₋₆ Alkenyloxy, C₁₋₆ Alkylthio, C₂₋₆ Alkenylthio, C₂₋₆ Alkylcarbonyl, Arylcarbonyl oder C₂₋₆ Alkoxycarbonyl; oder A¹¹ und A¹² bedeuten zusammen den Rest eines gegebenenfalls substituierten aromatischen oder heteroaromatischen Ringes;
A¹³ bedeutet Wasserstoff, C₁₋₆ Alkyl oder Aryl-(C₁₋₆)alkyl;
B bedeutet eine Bindung oder eine Carbonylgruppe (C=O); und
R³², R³³ und R³⁴ bedeuten unabhängig voneinander Wasserstoff, Halogen, Cyan, Trifluormethyl, Nitro, Hydroxy, Amino, Carboxy, C₁₋₆ Alkyl, C₁₋₆ Alkoxy, C₁₋₆ Alkylthio oder C₂₋₆ Alkoxycarbonyl, unter der Bedingung, daß mindestens einer der Reste R³², R³³ und R³⁴ eine andere Bedeutung als Wasserstoff aufweist.

6. Eine Verbindung, wie sie im Anspruch 3 beansprucht wird, dargestellt durch die Formel IIC, und Salze und Prodrugs hiervon: 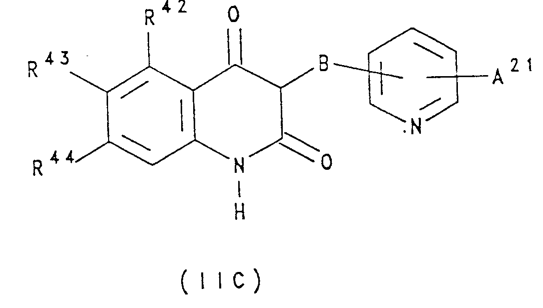 worin
A²¹ Wasserstoff, Halogen, Cyan, Trifluormethyl, Nitro, Hydroxy, Amino, Carboxy, C₁₋₆ Alkyl, C₁₋₆ Alkoxy, C₁₋₆ Alkylthio, C₂₋₆ Alkylcarbonyl, Arylcarbonyl oder C₂₋₆ Alkoxycarbonyl bedeutet;
B bedeutet eine Bindung oder eine Carbonylgruppe (C=O);
R⁴², R⁴³ und R⁴⁴ bedeuten unabhängig voneinander Wasserstoff, Halogen, Cyan, Trifluormethyl, Nitro, Hydroxy, Amino, Carboxy, C₁₋₆ Alkyl, C₁₋₆ Alkoxy, C₁₋₆ Alkylthio oder C₂₋₆ Alkoxycarbonyl, unter der Bedingung, daß mindestens einer der Reste R⁴², R⁴³ und R⁴⁴ eine andere Bedeutung als Wasserstoff aufweist.

7. Eine Verbindung, wie sie im Anspruch 3 beansprucht wird, ausgewählt aus:
3-Benzyloxycarbonyl-7-chlor-2,4-dioxo-1,2,3,4-tetrahydrochinolin;
7-Chlor-2,4-dioxo-3-(2-phenylethoxy)carbonyl-1,2,3,4-tetrahydrochinolin;
7-Chlor-2,4-dioxo-3-(3-phenylpropoxy)carbonyl-1,2,3,4-tetrahydrochinolin;
7-Chlor-2,4-dioxo-3-[2-(3-hydroxyphenyl)-ethoxy]carbonyl-1,2,3,4-tetrahydrochinolin;
7-Chlor-2,4-dioxo-3-[3-(4-hydroxyphenyl)-propoxy]carbonyl-1,2,3,4-tetrahydrochinolin;
7-Chlor-2,4-dioxo-3-[2-(2-hydroxyphenyl)-ethoxy]carbonyl-1,2,3,4-tetrahydrochinolin;
7-Chlor-3-cyclopropylmethoxycarbonyl-2,4,-dioxo-1,2,3,4-tetrahydrochinolin;
7-Chlor-2,4-dioxo-3-[2-(4-hydroxyphenyl)-ethoxy]carbonyl-1,2,3,4-tetrahydrochinolin;
7-Chlor-2,4-dioxo-3-(3-hydroxyphenylmethoxy)carbonyl-1,2,3,4-tetrahydrochinolin;
7-Chlor-2,4-dioxo-3-(3-pyrid-2-yl-methoxy)carbonyl-1,2,3,4-tetrahydrochinolin;
2,4-Dioxo-3-[3-(4-hydroxyphenyl)-propoxy]carbonyl-7-nitro-1,2,3,4-tetrahydrochinolin;
7-Chlor-2,4-dioxo-3-(2-hydroxyethoxy)carbonyl-1,2,3,4-tetrahydrochinolin;
7-Chlor-2,4-dioxo-3-(3-thienyl)carbonyl-1,2,3,4-tetrahydrochinolin;
7-Chlor-2,4-dioxo-3-(2-furyl)carbonyl-1,2,3,4-tetrahydrochinolin;
7-Chlor-2,4-dioxo-3-(2-thienyl)carbonyl-1,2,3,4-tetrahydrochinolin;
7-Chlor-3-(2,5-dimethyl-3-furyl)carbonyl-2,4-dioxo-1,2,3,4-tetrahydrochinolin;
7-Chlor-2,4-dioxo-3-(5-methyl-2-furyl)carbonyl-1,2,3,4-tetrahydrochinolin;
7-Chlor-2,4-dioxo-3-[2-(5-nitrobenzofuryl)carbonyl-1,2,3,4-tetrahydrochinolin;
3-[2-(Benzofuryl)carbonyl]-7-chlor-2,4-dioxo-1,2,3,4-tetrahydrochinolin;
3-[2-(Benzo[b]thienyl)carbonyl]-7-chlor-2,4-dioxo-1,2,3,4-tetrahydrochinolin;
3-Benzylcarbonyl-7-chlor-2,4-dioxo-1,2,3,4-tetrahydrochinolin;
7-Chlor-2,4-dioxo-3-(5-methyl-2-thienyl)carbonyl-1,2,3,4-tetrahydrochinolin;
7-Chlor-2,4-dioxo-3-(3-methyl-2-thienyl)carbonyl-1,2,3,4-tetrahydrochinolin;
7-Chlor-3-(2,5-dimethyl-3-thienyl)carbonyl-2,4-dioxo-1,2,3,4-tetrahydrochinolin;
7-Chlor-2,4-dioxo-3-[2-(3-thienyl)ethenyl]carbonyl-1,2,3,4-tetrahydrochinolin;
3-(5-Brom-2-thienyl)carbonyl-7-chlor-2,4-dioxo-1,2,3,4-tetrahydrochinolin;
7-Chlor-2,4-dioxo-3-(1-methylpyrrol-2-yl)carbonyl-1,2,3,4-tetrahydrochinolin;
7-Chlor-2,4-dioxo-3-(1-methylpyrrol-3-yl)carbonyl-1,2,3,4-tetrahydrochinolin;
7-Chlor-2,4-dioxo-3-(1-methylindol-3-yl)carbonyl-1,2,3,4-tetrahydrochinolin;
7-Chlor-3-cyclopropylcarbonyl-2,4-dioxo-1,2,3,4-tetrahydrochinolin;
7-Chlor-2,4-dioxo-3-(5-methyl-2-furyl)-1,2,3,4-tetrahydrochinolin;
7-Chlor-2,4-dioxo-3-(5-ethyl-2-furyl)-1,2,3,4-tetrahydrochinolin;
7-Chlor-2,4-dioxo-3-(1-methylpyrrol-2-yl)-1,2,3,4-tetrahydrochinolin;
7-Chlor-2,4-dioxo-3-(2-furyl)-1,2,3,4-tetrahydrochinolin;
3-(4-Benzoyl-1-methylpyrrol-2-yl)-7-chlor-2,4-dioxo-1,2,3,4-tetrahydrochinolin;
3-(5-Benzoyl-1-methylpyrrol-2-yl)-7-chlor-2,4-dioxo-1,2,3,4-tetrahydrochinolin;
7-Chlor-2,4-dioxo-3-[2-(5-methoxyindol-3-yl)-ethoxy]carbonyl-1,2,3,4-tetrahydrochinolin;
7-Chlor-2,4-dioxo-3-[3-(3-methoxyphenyl)-prop-2-inyloxy]carbonyl-1,2,3,4-tetrahydrochinolin;
7-Chlor-2,4-dioxo-3- (3-indol-3-yl-propoxy)carbonyl-1,2,3,4-tetrahydrochinolin;
3-[2-[3,4-Bis-(methoxymethoxy)phenyl]-ethoxy]carbonyl-7-chlor-2,4-dioxo-1,2,3,4-tetrahydrochinolin;
7-Chlor-2,4-dioxo-3-[4-(3-hydroxyphenyl)-butoxy]carbonyl-1,2,3,4-tetrahydrochinolin;
7-Chlor-2,4-dioxo-3-[3-(3-hydroxyphenyl)-propoxy]carbonyl-1,2,3,4-tetrahydrochinolin;
7-Chlor-2,4-dioxo-3-(2-indol-3-yl-ethoxy)carbonyl-1,2,3,4-tetrahydrochinolin;
7-Chlor-2,4-dioxo-3-[3-(2-hydroxyphenyl)-propoxy]carbonyl-1,2,3,4-tetrahydrochinolin;
3-[2-[4-(N-tert.Butoxycarbonylaminomethyl)phenyl]-ethoxy]carbonyl-7-chlor-2,4-dioxo-1,2,3,4-tetrahydrochinolin;
7-Chlor-2,4-dioxo-3-[2-(3-thienyl)-ethoxy]carbonyl-1,2,3,4-tetrahydrochinolin;
7-Chlor-2,4-dioxo-3-[2-(2-thienyl)-ethoxy]carbonyl-1,2,3,4-tetrahydrochinolin;
7-Chlor-2,4-dioxo-3-[3-(3-hydroxyphenyl)-prop-2-inyloxy]carbonyl-1,2,3,4-tetrahydrochinolin;
7-Chlor-2,4-dioxo-3-[3-(3-methoxyphenyl)-prop-2-enyloxy]carbonyl-1,2,3,4-tetrahydrochinolin;
7-Chlor-2,4-dioxo-3-[2-(phenylthio)-ethoxy]carbonyl-1,2,3,4-tetrahydrochinolin;
7-Chlor-2,4-dioxo-3-[2-(phenyl)-ethylthio]carbonyl-1,2,3,4-tetrahydrochinolin;
7-Chlor-2,4-dioxo-3-(2-indol-3-yl-ethoxy)carbonyl-5-iod-1,2,3,4-tetrahydrochinolin;
7-Chlor-2,4-dioxo-3-(3-methyl-1,2,4-oxadiazol-5-yl)-1,2,3,4-tetrahydrochinolin;
7-Chlor-2,4-dioxo-3-[2-(ethoxycarbonyl)-ethenyl]-1,2,3,4-tetrahydrochinolin;
7-Chlor-2,4-dioxo-1,2,3,4-tetrahydrochinolin-3-hydroxamsäure;
7-Chlor-2,4-dioxo-5-iod-1,2,3,4-tetrahydrochinolin-3-hydroxamsäure;
7-Chlor-2,4-dioxo-1,2,3,4-tetrahydrochinolin-3-carbonsäure-hydrazid;
7-Chlor-2,4-dioxo-3-(3-thienyl)-1,2,3,4-tetrahydrochinolin;
7-Chlor-2,4-dioxo-3-(2-thienyl)-1,2,3,4-tetrahydrochinolin;
7-Chlor-2,4-dioxo-3-(2-pyridyl)-1,2,3,4-tetrahydrochinolin;
7-Chlor-2,4-dioxo-3-(3-pyridyl)-1,2,3,4-tetrahydrochinolin;
7-Chlor-2,4-dioxo-3-(4-pyridyl)-1,2,3,4-tetrahydrochinolin;
3-(2-Benzofuryl)-7-chlor-2,4-dioxo-1,2,3,4-tetrahydrochinolin;
3-(3-Benzofuryl)-7-chlor-2,4-dioxo-1,2,3,4-tetrahydrochinolin;
7-Chlor-2,4-dioxo-3-(3-methyl-2-furyl)-1,2,3,4-tetrahydrochinolin;
7-Chlor-2,4-dioxo-3-(2-methyl-3-furyl)-1,2,3,4-tetrahydrochinolin;
7-Chlor-2,4-dioxo-3-(4-isopropyl-2-furyl)-1,2,3,4-tetrahydrochinolin;
7-Chlor-2,4-dioxo-3-(4-methyl-2-furyl)-1,2,3,4-tetrahydrochinolin;
7-Chlor-2,4-dioxo-3-(5-phenyl-2-furyl)-1,2,3,4-tetrahydrochinolin;
7-Chlor-2,4-dioxo-3-(5-methyl-2-thienyl)-1,2,3,4-tetrahydrochinolin;
3-(5-Benzyl-2-thienyl)-7-chlor-2,4-dioxo-1,2,3,4-tetrahydrochinolin;
3-(3-Benzo[b]thienyl)-7-chlor-2,4-dioxo-1,2,3,4-tetrahydrochinolin;
und aus Salzen und Prodrugs hiervon.

8. Eine Verbindung ausgewählt aus:
7-Chlor-3-cyan-2,4-dioxo-1,2,3,4-tetrahydrochinolin;
2,4-Dioxo-3-ethoxycarbonyl-6-nitro-1,2,3,4-tetrahydrochinolin;
7-Chlor-2,4-dioxo-3-methoxycarbonyl-1,2,3,4-tetrahydrochinolin;
7-Chlor-2,4-dioxo-3-n-propoxycarbonyl-1,2,3,4-tetrahydrochinolin;
6,7-Dinitro-2,4-dioxo-3-ethoxycarbonyl-1,2,3,4-tetrahydrochinolin;
7-Chlor-2,4-dioxo-3-ethoxycarbonyl-1,2,3,4-tetrahydro-chinolin;
2,4-Dioxo-3-ethoxycarbonyl-7-nitro-1,2,3,4-tetrahydrochinolin;
7-Chlor-2,4-dioxo-3-n-pentyloxycarbonyl-1,2,3,4-tetrahydrochinolin;
7-Chlor-2,4-dioxo-3-(2-propenyloxy)carbonyl-1,2,3,4-tetrahydrochinolin;
7-Chlor-2,4-dioxo-3-ethoxycarbonyl-5-iod-1,2,3,4-tetrahydrochinolin;
7-Chlor-2,4-dioxo-5-ethyl-3-methoxycarbonyl-1,2,3,4-tetrahydrochinolin;
3-Carboxy-7-chlor-2,4-dioxo-1,2,3,4-tetrahydrochinolin;
und aus Salzen und Prodrugs hiervon.

9. Eine pharmazeutische Zusammensetzung, umfassend mindestens eine Verbindung, wie sie in irgendeinem der Ansprüche 3 bis 8 beansprucht wird, oder ein pharmazeutisch annehmbares Salz hiervon oder ein Prodrug hiervon in Verbindung mit einem oder mehreren pharmazeutisch annehmbaren Trägern und/oder Vehikeln.

10. Ein Verfahren zur Herstellung einer Verbindung der Formel IB, wie sie im Anspruch 3 definiert ist, wobei dieses Verfahren:
(A) die reduktive Cyclisierung einer Verbindung der Formel III: 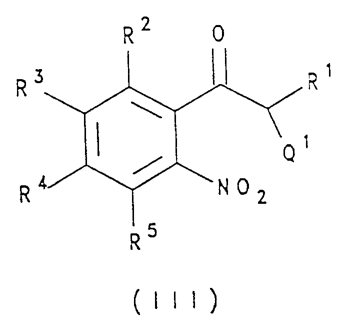 worin R¹, R², R³, R⁴ und R⁵ wie im Anspruch 1 definiert sind und Q¹ eine reaktionsfähige Carboxylatkomponente darstellt; oder
(B) Cyclisierung einer Verbindung der Formel V: 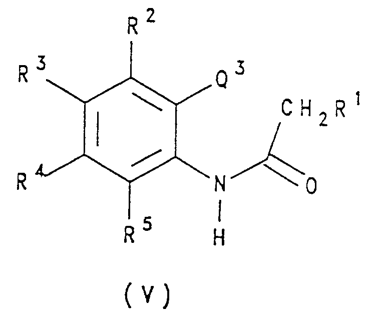 worin R¹, R², R³, R⁴ und R⁵ wie im Anspruch 1 definiert sind und Q³ eine reaktionsfähige Carboxylatkomponente darstellt; oder
(C) Reagieren einer Verbindung der Formel Q¹-CH₂-R¹ mit einer Verbindung der Formel VII: 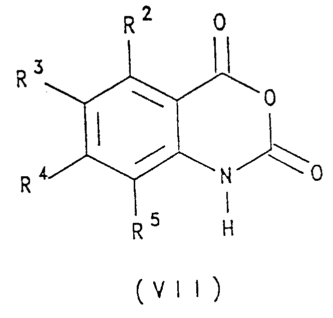 worin R¹, R², R³, R⁴ und R⁵ wie im Anspruch 1 definiert sind und Q¹ wie oben definiert ist; und
(D) gewünschtenfalls Überführung einer Verbindung der Foreml IB, die ursprünglich erhalten wurde, in eine weitere Verbindung der Formel IB unter Verwendung an sich bekannter Methoden umfaßt.

## Revendications

1. Utilisation d'un composé de formule IA ou d'un sel pharmaceutiquement acceptable de celui-ci ou d'un promédicament de celui-ci: 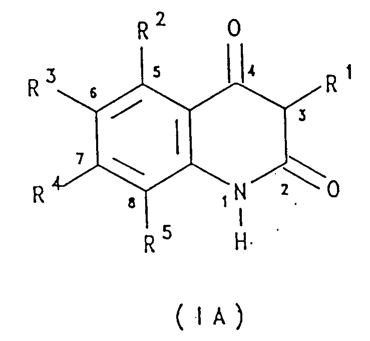 où
R¹ est un groupe de formule partielle (i) ou (ii):
-(CH=CH)ₙ-T (i) 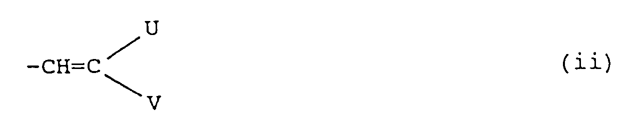
où
U et V représentent, indépendamment l'un de l'autre, un groupe cyano, carboxy, -COR⁶, -CO₂R⁶, -CO.SR⁶, -CONHOH ou -CONHNH₂;
n est zéro ou 1, de préférence zéro;
T représente un groupe cyano, carboxy, -COR⁶, -CO₂R⁶, -CO.SR⁶, -CONHOH, -CONHNH₂ ou un groupe de formule 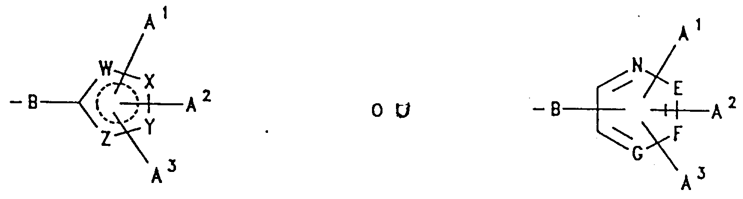 dans laquelle le cercle en pointillés représente deux doubles liaisons non-adjacentes en une position quelconque dans le cycle à cinq chaînons;
B désigne une liaison ou un groupe carbonyle (C=O);
W, X, Y et Z représentent, indépendamment l'un de l'autre, l'oxygène, le soufre, l'azote ou le carbone, à condition que par plus d'un des W, X, Y et Z représente l'oxygène ou le soufre, qu'au moins l'un des W, X, Y et Z représente le carbone et qu'au moins l'un des W, X, Y et Z soit autre que le carbone;
l'un des E, F et G représente l'azote ou le carbone et les autres représentent le carbone;
A¹, A² et A³ représentent un, deux ou trois substituants ne dépassant pas le nombre maximum permis par la disposition des hétéroatomes dans le cycle à cinq ou six chaînons, lesquels substituants sont choisis, indépendamment l'un de l'autre, parmi l'hydrogène, un halogène ou un groupe cyano, trifluorométhyle, nitro, -OR^{a}, -SR^{a}, -SOR^{a}, -SO₂R^{a}, -SO₂NR^{a}R^{b}, -NR^{a}R^{b}, -NR^{a}COR^{b}, -NR^{a}CO₂R^{b}, -COR^{a}, -CO₂R^{a}, -CONR^{a}R^{b}, ou un groupe hydrocarboné comprenant un groupe en chaîne droite, ramifié ou cyclique, contenant jusqu'à 18 atomes de carbone; ou A¹ et A² ou A² et A³ représentent ensemble le reste d'un cycle aromatique ou hétéroaromatique;
R², R³, R⁴ et R⁵ représentent, indépendamment l'un de l'autre, l'hydrogène, un halogène ou un groupe cyano, trifluorométhyle, nitro, -OR^{a}, -SR^{a}, -SOR^{a}, -SO₂R^{a}, -SO₂NR^{a}R^{b}_{,} -NR^{a}R^{b}, -NR^{a}COR^{b}, -NR^{a}CO₂R^{b}, -COR^{a}, -CO₂R^{a}, -CONR^{a}R^{b}_{,} ou un groupe hydrocarboné comprenant un groupe en chaîne droite, ramifié ou cyclique, contenant jusqu'à 18 atomes de carbone; ou R² et R³, R³ et R⁴ ou R⁴ et R⁵ représentent ensemble le reste d'un cycle aromatique ou hétéroaromatique;
R⁶ représente un groupe hydrocarboné comprenant un groupe en chaîne droite, ramifié ou cyclique, contenant jusqu'à 18 atomes de carbone; et
R^{a} et R^{b} représentent, indépendamment l'un de l'autre, l'hydrogène ou un groupe hydrocarboné comprenant un groupe en chaîne droite, ramifié ou cyclique contenant jusqu'à 18 atomes de carbone;
pour la production d'un médicament pour le traitement et/ou la prévention d'états, en particulier de troubles neurodégénératifs, qui requièrent l'administration d'un antagoniste sélectif, non-compétitif, des récepteurs de NMDA.

2. Utilisation d'un composé de formule IA tel que défini dans la revendication 1 ou d'un sel pharmaceutiquement acceptable de celui-ci ou d'un promédicament de celui-ci pour la préparation d'un médicament pour le traitement et/ou la prévention d'états qui requièrent l'administration d'un antagoniste des récepteurs d'AMPA.

3. Composé de formule IB ou sel ou promédicament de celui-ci: 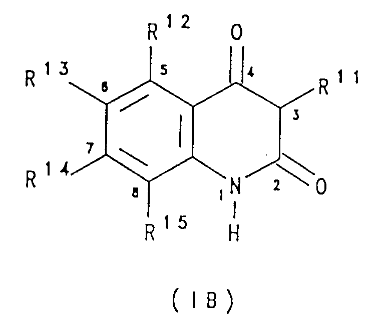 où
R¹¹ est un groupe de formule partielle (i) ou (ii):
-(CH=CH)ₙ-T (i) 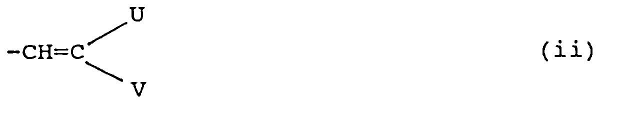
où
U et V représentent, indépendamment l'un de l'autre, un groupe cyano, carboxy, -COR⁶, -CO₂R⁶, -CO.SR⁶, -CONHOH ou -CONHNH₂;
n est zéro ou 1, de préférence zéro;
T représente un groupe cyano, carboxy, -COR⁶, -CO₂R⁶, -CO.SR⁶, -CONHOH, -CONHNH₂ ou un groupe de formule 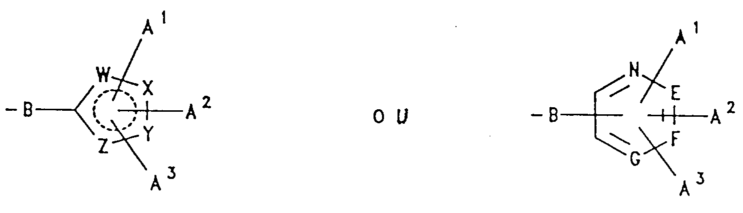 dans laquelle le cercle en pointillés représente deux doubles liaisons non-adjacentes en une position quelconque dans le cycle à cinq chaînons;
B désigne une liaison ou un groupe carbonyle (C=O);
W, X, Y et Z représentent, indépendamment l'un de l'autre, l'oxygène, le soufre, l'azote ou le carbone, a condition que par plus d'un des W, X, Y et Z représente l'oxygène ou le soufre, qu'au moins l'un des W, X, Y et Z représente le carbone et qu'au moins l'un des W, X, Y et Z soit autre que le carbone;
l'un des E, F et G représente l'azote ou le carbone et les autres représentent le carbone;
A¹, A² et A³ représentent un, deux ou trois substituants ne dépassant pas le nombre maximum permis par la disposition des hétéroatomes dans le cycle à cinq ou six chaînons, lesquels substituants sont choisis, indépendamment l'un de l'autre, parmi l'hydrogène, un halogène ou un groupe cyano, trifluorométhyle, nitro, -OR^{a}, -SR^{a}, -SOR^{a}, -SO₂R^{a}, -SO₂NR^{a}R^{b}, -NR^{a}R^{b}, -NR^{a}COR^{b}, -NR^{a}CO₂R^{b}, -COR^{a}, -CO₂R^{a}, -CONR^{a}R^{b}, ou un groupe hydrocarboné comprenant un groupe en chaîne droite, ramifié ou cyclique, contenant jusqu'à 18 atomes de carbone; ou A¹ et A² ou A² et A³ représentent ensemble le reste d'un cycle aromatique ou hétéroaromatique;
R¹², R¹³, R¹⁴ et R¹⁵ représentent, indépendamment l'un de l'autre, l'hydrogène, un halogène ou un groupe cyano, trifluorométhyle, nitro, -OR^{a}, -SR^{a}, -SOR^{a}, -SO₂R^{a}, -SO₂NR^{a}R^{b}, -NR^{a}R^{b}, -NR^{a}COR^{b}, -NR^{a}CO₂R^{b}, -COR^{a}, -CO₂R^{a}, -CONR^{a}R^{b}, ou un groupe hydrocarboné comprenant un groupe en chaîne droite, ramifié ou cyclique, contenant jusqu'à 18 atomes de carbone; ou R¹² et R¹³, R¹³ et R¹⁴ ou R¹⁴ et R¹⁵ représentent ensemble le reste d'un cycle aromatique ou hétéroaromatique;
R⁶ représente un groupe hydrocarboné comprenant un groupe en chaîne droite, ramifié ou cyclique, contenant jusqu'à 18 atomes de carbone; et
R^{a} et R^{b} représentent, indépendamment l'un de l'autre, l'hydrogène ou un groupe hydrocarboné comprenant un groupe en chaîne droite, ramifié ou cyclique contenant jusqu'à 18 atomes de carbone;
à condition que:
(a) lorsque deux des substituants R¹² à R¹⁵ représentent l'hydrogène et que les deux autres substituants R¹² à R¹⁵ représentent, indépendamment l'un de l'autre, l'hydrogène ou un groupe alkyle en C₁₋₈, halogéno, alcoxy en C₁₋₅, nitro, cyano, halogénoalkyle en C₁₋₃, carboxy ou alcoxy(C₂₋₄)carbonyle, alors R₁₁ ne représente pas un groupe benzoyle éventuellement mono-, di- ou trisubstitué par un groupe alkyle en C₁₋₈, halogéno, alcoxy en C₁₋₅, nitro, cyano, halogénoalkyle en C₁₋₃, carboxy ou alcoxy(C₂₋₄)carbonyle;
(b) lorsque R¹² à R¹⁵ représentent, indépendamment l'un de l'autre, l'hydrogène ou un groupe alkyle en C₁₋₅, alcoxy en C₁₋₅, benzyloxy, alcoxy(C₂₋₆)carbonyle, nitro ou halogéno, alors R¹¹ ne représente pas un groupe acétyle;
(c) lorsque l'un des R¹² à R¹⁵ représente l'hydrogène ou un groupe méthoxy et que le reste représente l'hydrogène, alors R¹¹ ne représente pas -CONHNH₂ ou -COR⁶, dans lequel R⁶ est un groupe alkyle en C₂₋₉;
(d) lorsque deux des substituants R¹² à R¹⁵ représentent l'hydrogène et que les deux autres substituants R¹² à R¹⁵ représentent, indépendamment l'un de l'autre, l'hydrogène, un halogène ou un groupe alkyle en C₁₋₄, alcoxy en C₁₋₄, nitro ou trifluorométhyle ou représentent ensemble un groupe méthylènedioxy, alors R¹¹ ne représente pas un groupe cyano, carboxy ou -CO₂R⁶, dans lequel R⁶ est un groupe alkyle en C₁₋₉, alcényle en C₃₋₆ ou alcynyle en C₃₋₆;
(e) lorsque deux des substituants R¹² à R¹⁵ représentent l'hydrogène, qu'un troisième substituant R¹² à R¹⁵ représente un groupe cycloalkyle ou cycloalcényle en C₅₋₈ éventuellement substitué ou un groupe adamantyle, et que le substituant R¹² à R¹⁵ restant représente l'hydrogène, un halogène ou un groupe alkyle inférieur ou alcoxy inférieur, alors R¹¹ ne représente pas un groupe carboxy ou -COR¹⁶, dans lequel R¹⁶ est un groupe hydroxyle éthérifié;
(f) lorsque R¹² à R¹⁵ représentent chacun l'hydrogène et que R¹¹ est un groupe de formule partielle (ii) telle que définie plus haut, alors U et V ne soient pas simultanément des gorupes carboxy et que U ne soit pas un groupe carboxy ou éthoxycarbonyle quand V est un groupe cyano; et
(g) lorsque R¹² à R¹⁵ représentent chacun l'hydrogène, alors R¹¹ ne représente pas un groupe 2-pyridyle, 3-pyridyle, 2-furyle ou 2-benzothiazolyle non-substitué.

4. Composé selon la revendication 3, représenté par la formule IIA, et sels et promédicaments de celui-ci: 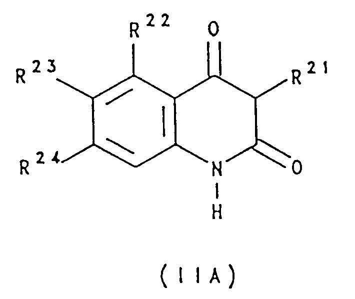 où
E²¹ représente -COR²⁶ ou -CO₂R²⁶;
R²², R²³ et R²⁴ représentent, indépendamment l'un de l'autre, l'hydrogène, un halogène ou un groupe cyano, trifluorométhyle, nitro, hydroxy, amino, carboxy, alkyle en C₁₋₆, alcoxy en C₁₋₆, alkylthio en C₁₋₆ ou alcoxy(C₂₋₆) carbonyle, à condition qu'au moins l'un des R²², R²³ et R²⁴ soit autre que l'hydrogène; et
R²⁶ représente un groupe cycloalkyle en C₃₋₇, arylalkyle(C₁₋₆), arylalcényle(C₂₋₆), arylalcynyle(C₂₋₆), hétéroarylalkyle(C₁₋₆) ou hétéroarylalcényle(C₂₋₆), l'un quelconque de ces groupes pouvant être éventuellement substitué.

5. Composé selon la revendication 3, représenté par la formule IIB, et sels et promédicaments de celui-ci: 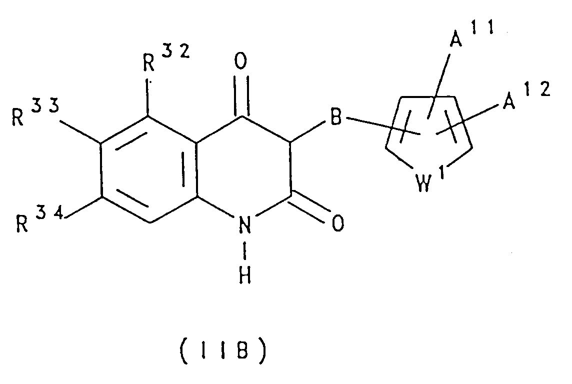 où
W¹ représente l'oxygène, le soufre ou N-A¹³;
A¹¹ et A¹² représentent, indépendamment l'un de l'autre, l'hydrogène, un halogène ou un groupe cyano, trifluorométhyle, nitro, hydroxy, amino, carboxy, alkyle en C₁₋₆, alcényle en C₂₋₆, alcynyle en C₂₋₆, cycloalkyle en C₃₋₇, cycloalkyl(C₃₋₇)alkyle(C₁₋₆), aryle, arylalkyle-(C₁₋₆), alcoxy en C₁₋₆, alcényloxy en C₂₋₆, alkylthio en C₁₋₆, alcénylthio en C₂₋₆, alkyl(C₂₋₆)carbonyle, arylcarbonyle ou alcoxy(C₂₋₆)carbonyle; ou A¹¹ et A¹² représentent ensemble le reste d'un cycle aromatique ou hétéroaromatique éventuellement substitué;
A¹³ représente l'hydrogène ou un groupe alkyle en C₁₋₆ ou arylalkyle(C₁₋₆);
B représente une double liaison ou un groupe carbonyle (C=O);
et
R³², R³³ et R³⁴ représentent, indépendamment l'un de l'autre, l'hydrogène, un halogène ou un groupe cyano, trifluorométhyle, nitro, hydroxy, amino, carboxy, alkyle en C₁₋₆, alcoxy en C₁₋₆, alkylthio en C₁₋₆ ou alcoxy(C₂₋₆) carbonyle, à condition qu'au moins l'un des R³², R³³ et R³⁴ soit autre que l'hydrogène.

6. Composé selon la revendication 3, représenté par la formula IIC, et sels et promédicaments de celui-ci: 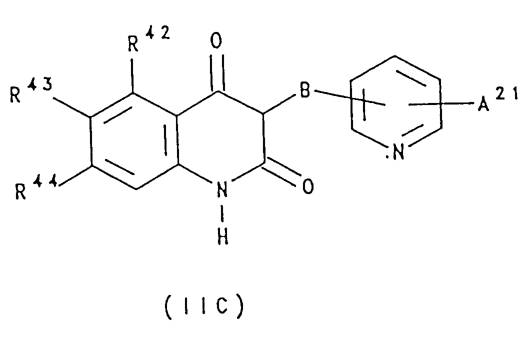 où
A²¹ représente l'hydrogène, un halogène ou un groupe cyano, trifluorométhyle, nitro, hydroxy, amino, carboxy, alkyle en C₁₋₆, alcoxy en C₁₋₆, alkylthio en C₁₋₆, alkyl(C₂₋₆)carbonyle, arylcarbonyle ou alcoxy(C₂₋₆) carbonyle;
B représente une liaison ou un groupe carbonyle (C=O);
et
R⁴², R⁴³ et R⁴⁴ représentent, indépendamment l'un de l'autre, l'hydrogène, un halogène ou un groupe cano, trifluorométhyle, nitro, hydroxy, amino, carboxy, alkyle en C₁₋₆, alcoxy en C₁₋₆, alkylthio en C₁₋₆ ou alcoxy(C₂₋₆)-carbonyle, à condition qu'au moins l'un des R⁴², R⁴³ et R⁴⁴ soit autre que l'hydrogène.

7. Composé selon la revendication 3, choisi parmi les suivants:
3-benzyloxycarbonyl-7-chloro-2,4-dioxo-1,2,3,4-tétrahydroquinoléine;
7-chloro-2,4-dioxo-3-(2-phényléthoxy)carbonyl-1,2,3,4-tétrahydroquinoléine;
7-chloro-2,4-dioxo-3-(3-phénylpropoxy)carbonyl-1,2,3,4-tétrahydroquinoléine;
7-chloro-2,4-dioxo-3-[2-(3-hydroxyphényl)éthoxy]carbonyl-1,2,3,4-tétrahydroquinoléine;
7-chloro-2,4-dioxo-3-[3-(4-hydroxyphényl)propoxy]carbonyl-1,2,3,4-tétrahydroquinoléine;
7-chloro-2,4-dioxo-3-[2-(2-hydroxyphényl)éthoxy]carbonyl-1,2,3,4-tétrahydroquinoléine;
7-chloro-3-cyclopropylméthoxycarbonyl-2,4-dioxo-1,2,3,4-tétrahydroquinoléine;
7-chloro-2,4-dioxo-3-[2-(4-hydroxyphényl)éthoxy]carbonyl-1,2,3,4-tétrahydroquinoléine;
7-chloro-2,4-dioxo-3-(3-hydroxyphénylméthoxy)carbonyl-1,2,3,4-tétrahydroquinoléine;
7-chloro-2,4-dioxo-3-(pyrid-2-ylméthoxy)carbonyl-1,2,3,4-tétrahydroquinoléine;
2,4-dioxo-3-[3-(4-hydroxyphényl)propoxy]carbonyl-7-nitro-1,2,3,4-tétrahydroquinoléine;
7-chloro-2,4-dioxo-3-(2-hydroxyéthoxy)carbonyl-1,2,3,4-tétrahydroquinoléine;
7-chloro-2,4-dioxo-3-(3-thiényl)carbonyl-1,2,3,4-tétrahydroquinoléine;
7-chloro-2,4-dioxo-3-(2-furyl)carbonyl-1,2,3,4-tétrahydroquinoléine;
7-chloro-2,4-dioxo-3-(2-thiényl)carbonyl-1,2,3,4-tétrahydroquinoléine;
7-chloro-3-(2,5-diméthyl-3-furyl)carbonyl-1,2,3,4-tétrahydroquinoléine;
7-chloro-2,4-dioxo-3-(5-méthyl-2-furyl)carbonyl-1,2,3,4-tétrahydroquinoléine;
7-chloro-2,4-dioxo-3-[2-(5-nitrobenzofuryl)carbonyl]-1,2,3,4-tétrahydroquinoléine;
3-[2-(benzofuryl)carbonyl]-7-chloro-2,4-dioxo-1,2,3,4-tétrahydroquinoléine;
3-[2-(benzo[b]thiényl)carbonyl]-7-chloro-2,4-dioxo-1,2,3,4-tétrahydroquinoléine;
3-benzylcarbonyl-7-chloro-2,4-dioxo-1,2,3,4-tétrahydroquinoléine;
7-chloro-2,4-dioxo-3-(5-méthyl-2-thiényl)carbonyl-1,2,3,4-tétrahydroquinoléine;
7-chloro-2,4-dioxo-3-(3-méthyl-2-thiényl)carbonyl-1,2,3,4-tétrahydroquinoléine;
7-chloro-3-(2,5-diméthyl-3-thiényl)carbonyl-2,4-dioxo-1,2,3,4-tétrahydroquinoléine;
7-chloro-2,4-dioxo-3-[2-(3-thiényl)éthényl)]carbonyl-1,2,3,4-tétrahydroquinoléine;
3-(5-bromo-2-thiényl)carbonyl-7-chloro-2,4-dioxo-1,2,3,4-tétrahydroquinoléine;
7-chloro-2,4-dioxo-3-(1-méthylpyrrole-2-yl)carbonyl-1,2,3,4-tétrahydroquinoléine;
7-chloro-2,4-dioxo-3-(1-méthylpyrrole-3-yl)carbonyl-1,2,3,4-tétrahydroquinoléine;
7-chloro-2,4-dioxo-3-(1-méthylindole-3-yl)carbonyl-1,2,3,4-tétrahydroquinoléine;
7-chloro-3-cyclopropylcarbonyl-2,4-dioxo-1,2,3,4-tétrahydroquinoléine;
7-chloro-2,4-dioxo-3-(5-méthyl-2-furyl)-1,2,3,4-tétrahydroquinoléine;
7-chloro-2,4-dioxo-3-(5-éthyl-2-furyl)-1,2,3,4-tétrahydroquinoléine;
7-chloro-2,4-dioxo-3-(1-méthylpyrole-2-yl)-1,2,3,4-tétrahydroquinoléine;
7-chloro-2,4-dioxo-3-(2-furyl)-1,2,3,4-tétrahydroquinoléine;
3-(4-benzoyl-1-méthylpyrrole-2-yl)-7-chloro-2,4-dioxo-1,2,3,4-tétrahydroquinoléine;
3-(5-benzoyl-1-méthylpyrrole-2-yl)-7-chloro-2,4-dioxo-1,2,3,4-tétrahydroquinoléine;
7-chloro-2,4-dioxo-3-[2-(5-méthoxylndole-3-yl)éthoxy]carbonyl-1,2,3,4-tétrahydroquinoléine;
7-chloro-2,4-dioxo-3-[3-(3-méthoxyphényl)prop-2-ynyloxy]carbonyl-1,2,3,4-tétrahydroquinoléine;
7-chloro-2,4-dioxo-3-(3-indole-3-ylpropoxy]carbonyl-1,2,3,4-tétrahydroquinoléine;
3-[2- [3,4-bis(méthoxyméthoxy)phényl]éthoxy]carbonyl-7-chloro-2,4-dioxo-1,2,3,4-tétrahydroquinoléine;
7-chloro-2,4-dioxo-3-[4-(3-hydroxyphényl)butoxy]carbonyl-1,2,3,4-tétrahydroquinoléine;
7-chloro-2,4-dioxo-3-[3-(3-hydroxyphényl)propoxy]carbonyl-1,2,3,4-tétrahydroquinoléine;
7-chloro-2,4-dioxo-3-(2-indole-3-yléthoxy)carbonyl-1,2,3,4-tétrahydroquinoléine;
7-chloro-2,4-dioxo-3-[3-(2-hydroxyphényl)propoxy]carbonyl-1,2,3,4-tétrahydroquinoléine;
3-[2- [4-(N-tert-butoxycarbonylaminoéthyl)phényl]éthoxy]carbonyl-7-chloro-2,4-dioxo-1,2,3,4-tétrahydroquinoléine;
7-chloro-2,4-dioxo-3-[2-(3-thiényl)éthoxy]carbonyl-1,2,3,4-tétrahydroquinoléine;
7-chloro-2,4-dioxo-3-[2-(2-thiényl)éthoxy]carbonyl-1,2,3,4-tétrahydroquinoléine;
7-chloro-2,4-dioxo-3-[3-(3-hydroxyphényl)prop-2-ynyloxy]carbonyl-1,2,3,4-tétrahydroquinoléine;
7-chloro-2,4-dioxo-3-[3-(3-méthoxyphényl)prop-2-ényloxy]carbonyl-1,2,3,4-tétrahydroquinoléine;
7-chloro-2,4-dioxo-3-[2-(phénylthio)éthoxy]carbonyl-1,2,3,4-tétrahydroquinoléine;
7-chloro-2,4-dioxo-3-[2-(phényl)éthylthio]carbonyl-1,2,3,4-tétrahydroquinoléine;
7-chloro-2,4-dioxo-3-(2-indole-3-yléthoxy)carbonyl-5-iodo-1,2,3,4-tétrahydroquinoléine;
7-chloro-2,4-dioxo-3-(3-méthyl-1,2,4-oxadiazole-5-yl)-1,2,3,4-tétrahydroquinoléine;
7-chloro-2,4-dioxo-3-[2-(éthoxycarbonyl)éthényl]-1,2,3,4-tétrahydroquinoléine;
acide 7-chloro-2,4-dioxo-1,2,3,4-tétrahydroquinoléine-3-hydroxamique;
acide 7-chloro-2,4-dioxo-5-iodo-1,2,3,4-tétrahydroquinoléine-3-hydroxamique;
hydrazide d'acide 7-chloro-2,4-dioxo-1,2,3,4-tétrahydroquinoléine-3-carboxylique;
7-chloro-2,4-dioxo-3-(3-thiényl)-1,2,3,4-tétrahydroquinoléine;
7-chloro-2,4-dioxo-3-(2-thiényl)-1,2,3,4-tétrahydroquinoléine;
7-chloro-2,4-dioxo-3-(2-pyridyl)-1,2,3,4-tétrahydroquinoléine;
7-chloro-2,4-dioxo-3-(3-pyridyl)-1,2,3,4-tétrahydroquinoléine;
7-chloro-2,4-dioxo-3-(4-pyridyl)-1,2,3,4-tétrahydroquinoléine;
3-(2-benzofuryl)-7-chloro-2,4-dioxo-1,2,3,4-tétrahydroquinoléine;
3-(3-benzofuryl)-7-chloro-2,4-dioxo-1,2,3,4-tétrahydroquinoléine;
7-chloro-2,4-dioxo-3-(3-méthyl-2-furyl)-1,2,3,4-tétrahydroquinoléine;
7-chloro-2,4-dioxo-3-(2-méthyl-3-furyl)-1,2,3,4-tétrahydroquinoléine;
7-chloro-2,4-dioxo-3-(4-isopropyl-2-furyl)-1,2,3,4-tétrahydroquinoléine;
7-chloro-2,4-dioxo-3-(4-méthyl-2-furyl)-1,2,3,4-tétrahydroquinoléine;
7-chloro-2,4-dioxo-3-(5-phényl-2-furyl)-1,2,3,4-tétrahydroquinoléine;
7-chloro-2,4-dioxo-3-(5-méthyl-2-thiényl)-1,2,3,4-tétrahydroquinoléine;
3-(5-benzyl-2-thiényl)-7-chloro-2,4-dioxo-1,2,3,4-tétrahydroquinoléine;
3-(3-benzo[b]thiényl)-7-chloro-2,4-dioxo-1,2,3,4-tétrahydroquinoléine;
et leurs sels et promédicaments.

8. Composé choisi parmi les suivants:
7-chloro-3-cyano-2,4-dioxo-1,2,3,4-tétrahydroquinoléine;
2,4-dioxo-3-éthoxycarbonyl-6-nitro-1,2,3,4-tétrahydroquinoléine;
7-chloro-2,4-dioxo-3-méthoxycarbonyl-1,2,3,4-tétrahydroquinoléine;
7-chloro-2,4-dioxo-3-n-propoxycarbonyl-1,2,3,4-tétrahydroquinoléine;
6,7-dinitro-2,4-dioxo-3-éthoxycarbonyl-1,2,3,4-tétrahydroquinoléine;
7-chloro-2,4-dioxo-3-éthoxycarbonyl-1,2,3,4-tétrahydroquinoléine;
2,4-dioxo-3-éthoxycarbonyl-7-nitro-1,2,3,4-tétrahydroquinoléine;
7-chloro-2,4-dioxo-3-n-pentyloxycarbonyl-1,2,3,4-tétrahydroquinoléine;
7-chloro-2,4-dioxo-3-(2-propényloxy)carbonyl-1,2,3,4-tétrahydroquinoléine;
7-chloro-2,4-dioxo-3-éthoxycarbonyl-5-iodo-1,2,3,4-tétrahydroquinoléine;
7-chloro-2,4-dioxo-5-éthyl-3-méthoxycarbonyl-1,2,3,4-tétrahydroquinoléine;
3-carboxy-7-chloro-2,4-dioxo-1,2,3,4-tétrahydroquinoléine;
et leurs sels et promédicaments.

9. Composition pharmaceutique comprenant au moins un composé selon l'une quelconque des revendications 3 à 8 ou un sel pharmaceutiquement acceptable de celui-ci ou un promédicament de celui-ci, en association avec un ou plusieurs vecteurs et/ou excipients pharmaceutiquement acceptables.

10. Procédé pour la préparation d'un composé de formule IB selon la revendication 3, lequel procédé comprend:
(A) la cyclisation par réduction d'un composé de formule III: 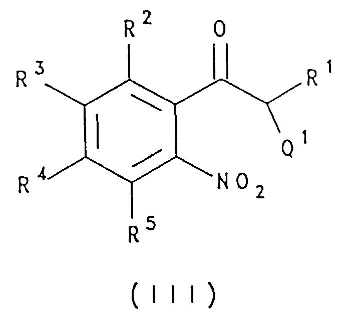 où R¹, R², R³, R⁴ et R⁵ sont tels que définis dans la revendication 1; et Q¹ représente une portion carboxylate réactive; ou
(B) la cyclisation d'un composé de formule V: 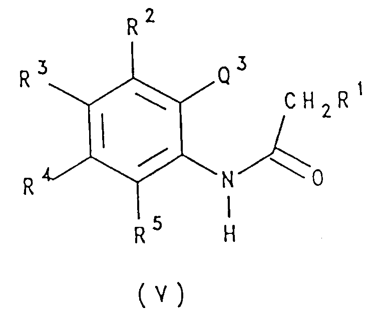 où R¹, R², R³, R⁴ et R⁵ sont tels que définis dans la revendication 1; et Q³ représente une portion carboxylate réactive; ou
(C) la réaction d'un composé de formule Q1-CH₂-R¹ avec un composé de formule VII: 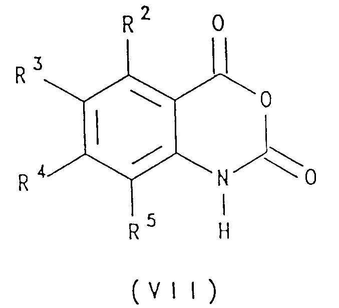 où R¹, R², R³, R⁴ et R⁵ sont tels que définis dans la revendication 1; et Q¹ représente une portion carboxylate réactive; et
(D) si on le souhaite, la conversion d'un composé de formule IB initialement obtenu en un autre composé de formule IB au moyen de procédés connus en eux-mêmes.
